# EUROPEAN PATENT APPLICATION

(11) **EP 4 722 252 A1**
(43) Date of publication of application: **08.04.2026**
(21) Application number: 23940480.9
(22) Date of filing: 06.12.2023
(51) Int. Cl.: C07K 16/38, A61K 39/395, C12N 15/13, A61P 7/04

(54) **ANTIBODY BINDING TISSUE FACTOR PATHWAY INHIBITOR AND USE OF ANTIBODY**

(30) Priority: 05.06.2023 WO PCT/CN2023/098242
(71) Applicant: Ampsource Biopharma Shanghai Inc., Shanghai 201318 (CN)
(72) Inventor: LI, Qiang, Shanghai 201318 (CN); DIAO, Jiasheng, Shanghai 201318 (CN); SUN, Jianyu, Shanghai 201318 (CN); ZHOU, Chi, Shanghai 201318 (CN); WU, Cui, Shanghai 201318 (CN); GAO, Yongjuan, Shanghai 201318 (CN); LI, Yuanli, Shanghai 201318 (CN); CHEN, Si, Shanghai 201318 (CN)
(74) Representative: J A Kemp LLP
(86) International application number: PCT/CN2023/136796
(87) International publication number: WO 2024/250605

(57) **Abstract**

The present invention provides an antibody or an antigen-binding fragment thereof that specifically binds to Tissue Factor Pathway Inhibitor (TFPI) in a pH-dependent manner. Also provided are nucleic acid molecules encoding the antibody, expression vectors and host cells for expressing the antibody, as well as methods for producing the antibody. Furthermore, the invention provides a pharmaceutical composition comprising the antibody or antigen-binding fragment thereof, and the use of the pharmaceutical composition in the manufacture of a medicament for the prevention and/or treatment of diseases including hereditary or acquired coagulation factor deficiency, Bernard-Soulier Syndrome, and Glanzmann Thrombasthenia.

## Description

### Technical Field

The present invention belongs to the field of therapeutic monoclonal antibodies. More specifically, the present invention relates to an antibody or antigen-binding fragment thereof targeting Tissue Factor Pathway Inhibitor (TFPI); it also relates to the use of the antibody in the preparation of a medicament for preventing or treating hereditary or acquired coagulation disorders or events.

### Background

Hemophilia A and B are hereditary coagulation disorder diseases characterized by defective generation of active prothrombinase, prolonged clotting time, and a lifelong tendency to bleed after minor trauma, with spontaneous bleeding occurring even in the absence of apparent injury in severe cases. Hemophilia A is characterized by the absence or deficiency of coagulation factor VIII (FVIII), accounting for more than 80% of all hemophilia cases, with a prevalence of 1 in 5,000 in the male population. Hemophilia B is characterized by the absence or deficiency of coagulation factor IX (FIX), with an incidence of 1 in 30,000 newborns (Chowdary, P. Int J Hematol, 2020, 111: 42-50). Recurrent, spontaneous, and trauma-related bleeding are hallmarks of severe hemophilia, with typical bleeding sites being joints and muscles. Under minimal therapy, severe patients experience up to 30-40 bleeding episodes per year, leading to irreversible joint damage and secondary disabilities. The most commonly used treatment at present is factor replacement therapy, which involves supplementing the deficient FVIII or FIX in hemophilia patients. Based on current treatment regimens, prophylactic therapy requires frequent infusions of FVIII or FIX, typically 2 to 4 times per week, to reduce spontaneous bleeding. While such treatment effectively prevents most spontaneous bleeding, it does not prevent traumatic bleeding. Moreover, the efficacy of the treatment is influenced by the frequency and dose of infusions as well as the number of missed or delayed doses. The dose and infusion frequency are, in turn, affected by the elimination half-life of the factor, the cost-effectiveness of the regimen, and patient acceptability. To overcome the limitations of factor replacement therapy, improve patient compliance, and reduce patient burden, it is necessary to explore new treatment approaches.

Platelets are a major component of the blood system. When blood vessels are damaged, platelets play a role in hemostasis and coagulation. A decrease in platelet count or functional impairment can lead to hemostatic dysfunction and poor thrombus formation in the body, thereby triggering related platelet disorders. Bernard-Soulier Syndrome (BSS), also known as giant platelet syndrome, is a rare hereditary bleeding disorder with an incidence of less than 1 in 1,000,000. It is primarily caused by defective synthesis and expression of the glycoprotein complex GPIb-IX-V on the platelet membrane or genetic defects in any of its subunits GPIb, GPIX, or GPV. The defect in GPIb-IX-V prevents platelets from adhering normally to the vascular endothelium, leading to cutaneous, mucosal, or visceral bleeding of varying severity. Large platelets are observed in the peripheral blood of patients, accompanied by mild to moderate thrombocytopenia. Glanzmann Thrombasthenia (GT) was first reported by Glanzmann in 1918 and is also referred to as "Glanzmann's disease." It is a hereditary bleeding disorder characterized by a diminished or absent response of platelets to various physiological aggregating agents, caused by qualitative or quantitative abnormalities of platelet membrane glycoproteins IIb (GPIIb) and/or IIIa (GPIIIa). In 1990, the Standardization Committee of the International Society on Thrombosis and Haemostasis defined GT as a congenital hereditary condition characterized by absent or severely reduced platelet aggregation in response to multiple agonists (such as adenosine diphosphate, thrombin, collagen, etc.), resulting from defects in the GPIIb or GPIIIa genes.

TFPI is a naturally occurring anticoagulant protein in the body that controls the initiation phase of coagulation. It specifically inhibits the tissue factor-initiated coagulation pathway by binding to the TF/FVIIa complex, thereby preventing the activation of factor X and subsequently inhibiting coagulation function. When TFPI activity is neutralized by anti-TFPI antibodies, the coagulation process is restored or amplified. TFPI is a glycoprotein consisting of 276 amino acid residues, containing three Kunitz domains (K1, K2, and K3), along with two linker regions, an acidic amino acid-rich N-terminus, and a basic amino acid-rich C-terminus. Domains K1 and K2 are critical for the anticoagulant activity of TFPI, while K3 is not essential for this function; however, its presence enhances TFPI's ability to inhibit coagulation. Recent studies have shown that K3 and the C-terminus can bind to heparin, membrane surface glycoproteins, and polysaccharides; in addition, these regions are also involved in the anti-inflammatory effects of TFPI. The inhibitory effect of TFPI in the coagulation pathway is achieved in two steps: first, TFPI binds to activated FX via K2 and competitively inhibits its activity, which is a Ca²⁺-independent reversible process; second, TFPI within the FXa/TFPI complex binds via K1 to the active site of FVIIa in the FVIIa/TF complex, thereby inhibiting the FVIIa/TF complex. Anti-TFPI antibodies target the K2 region of TFPI, competitively blocking the binding site for FXa on TFPI, thereby increasing FXa levels. They also disrupt the inhibitory effect of TFPI on TF-FVIIa, which occurs in the presence of FXa and Ca²⁺, leading to elevated levels of FXa and thrombin generation, thereby enhancing coagulation capacity through a dual mechanism.

Anti-TFPI antibodies can be applied in the treatment of hemophilia patients with or without inhibitors to FVIII and FIX, giant platelet syndrome (Bernard-Soulier Syndrome), and Glanzmann Thrombasthenia. Furthermore, such antibodies inherently possess a long half-life, which can reduce dosing frequency. Currently, there are no marketed antibody drugs targeting TFPI. There remains a need in the art to explore therapeutic antibodies with improved performance in terms of in vivo circulating half-life, in vivo stability, human safety, and manufacturability.

### Summary

The present invention provides an antibody capable of specifically binding human TFPI, wherein the antibody exhibits pH-dependent antigen-binding characteristics; a nucleic acid encoding the antibody; a vector comprising the nucleic acid; a host cell comprising the vector; a method for producing the antibody; and a pharmaceutical composition for preventing or treating coagulation disorders in patients with hereditary or acquired coagulation factor deficiencies, the pharmaceutical composition comprising the antibody as an active ingredient and being capable of inhibiting TFPI to thereby activate the coagulation pathway. The anti-human TFPI antibody disclosed in the present invention can be used for treating or preventing hemophilia.

In one aspect of the present invention, provided is an antibody or antigen-binding fragment thereof capable of pH-dependently binding to TFPI, characterized in that specific amino acids in the variable region of the TFPI antibody or antigen-binding fragment can interact with amino acid residues at or near the binding interface of the TFPI protein having the amino acid sequence of SEQ ID NO: 1, wherein the amino acid residues comprise basic amino acid residues of the tissue factor pathway inhibitor.

Preferably, the antibody or antigen-binding fragment specifically binds to at least one or several basic amino acids in the TFPI protein, wherein the basic amino acids are selected from R124, R112, K93, K120, K126, and K144, and more preferably are R124 and K126.

Preferably, the pKa of binding between specific amino acids in the variable region of the TFPI antibody or antigen-binding fragment and the TFPI protein is greater than 5, and the TFPI antibody or antigen-binding fragment exhibits pH-dependent antigen-binding capability, wherein the ratio of its K_{D} and/or koff for binding to TFPI at pH ≤ 6.0 relative to that at pH 7.4 is 2 or greater.

Preferably, the antibody or antigen-binding fragment binds to basic amino acids of the TFPI protein through specific amino acids in its variable region, wherein the specific amino acids in the variable region are selected from Glu or Asp. Preferably, the pKa of binding between the specific amino acids in the variable region and the TFPI protein is greater than 6; more preferably, the binding pKa is greater than 7.

Preferably, binding affinity of the antibody or antigen-binding fragment to TFPI is pH-dependent over a pH range of 4.5 to 7.4.

The pH-dependent TFPI antibody of the present invention exhibits a longer half-life in vivo. For example, the half-life of 100 mg of the antibody or antigen-binding fragment in the human body exceeds 100 hours, preferably exceeds 120 hours, and more preferably exceeds 140 hours.

Preferably, the amino acid residues further comprise T139 and/or T119, and the amino acid residues do not include R107. Preferably, the amino acid residues further comprise Y109. More preferably, the amino acid residues further comprise at least one amino acid residue selected from the group consisting of I110, T111, F125, and E138, and/or the amino acid residues further comprise at least one amino acid residue selected from the group consisting of E101, Y113, F114, N116, Q118, Q121, C122, E123, and L140.

Preferably, the shortest distance between the CA atom of an amino acid residue in the antibody or antigen-binding fragment and the CA atom of the R107 residue in TFPI is greater than 9.0 Å.

Preferably, the antibody or antigen-binding fragment comprises VH and VL, wherein the heavy chain variable region as defined according to the Kabat numbering system comprises at least one, two, or three CDRs selected from the group consisting of:
(1) HCDR1 having the amino acid sequence SYWMH;
(2) HCDR2 having the amino acid sequence EIKPDX₁GRIX₂YNEKFKT, wherein X₁ is selected from Q, H, or K, and X₂ is selected from N, D, or H; and
(3) HCDR3 having the amino acid sequence SSX₃X₄DX₅AX₆DY, wherein X₃ is selected from V or H, X₄ is selected from V, I, F, Y, or L, X₅ is selected from V, D, T, or E, and X₆ is selected from M, L, I, E, V, or D;
and/or, the light chain variable region thereof comprises at least one, two, or three CDRs selected from the group consisting of:
(4) LCDR1 having the amino acid sequence SASSSVSSSYLY;
(5) LCDR2 having the amino acid sequence GTSILAS; and
(6) LCDR3 having the amino acid sequence HQWX₇X₈YPFT, wherein X₇ is selected from S or T, and X₈ is selected from H, Y, or F.

Preferably, the antibody or antigen-binding fragment comprises VH and VL, wherein the heavy chain variable region as defined according to the IMGT numbering system comprises at least one, two, or three CDRs selected from the group consisting of:
(1) HCDR1 having the amino acid sequence GYTFTSYW;
(2) HCDR2 having the amino acid sequence IKPDX₁GRI, wherein X₁ is selected from Q, H, or K; and
(3) HCDR3 having the amino acid sequence ARSSX₃X₄DX₅AX₆DY, wherein X₃ is selected from V or H, X₄ is selected from V, I, F, Y, or L, X₅ is selected from V, D, T, or E, and X₆ is selected from M, L, I, E, V, or D;
and/or, the light chain variable region thereof comprises at least one, two, or three CDRs selected from the group consisting of:
(4) LCDR1 having the amino acid sequence SSVSSSY;
(5) LCDR2 having the amino acid sequence GTS; and
(6) LCDR3 having the amino acid sequence HQWX₇X₈YPFT, wherein X₇ is selected from S or T, and X₈ is selected from H, Y, or F.

In some preferred embodiments, the HCDR1, HCDR2, and HCDR3 contained in the heavy chain variable region, and/or the LCDR1, LCDR2, and LCDR3 contained in the light chain variable region are defined by the Kabat or IMGT numbering system. Table 2 exemplarily presents the amino acid sequences of the CDRs of preferred antibodies as defined by the Kabat or IMGT numbering system.

In certain preferred embodiments, the antibody or antigen-binding fragment comprises 3 VH CDRs and 3 VL CDRs as defined by the Kabat numbering system, selected from the group consisting of:
(1) Its HCDR1, HCDR2, HCDR3, LCDR1, LCDR2, and LCDR3 respectively having sequences as shown in SEQ ID NO: 46, 47, 48, 49, 50, or 51, or sequences having one or several amino acid substitutions, deletions, or additions (e.g., 1, 2, or 3 substitutions, deletions, or additions) compared to any of the aforementioned sequences;
(2) Its HCDR1, HCDR2, HCDR3, LCDR1, LCDR2, and LCDR3 respectively having sequences as shown in SEQ ID NO: 46, 47, 56, 49, 50, or 51, or sequences having one or several amino acid substitutions, deletions, or additions (e.g., 1, 2, or 3 substitutions, deletions, or additions) compared to any of the aforementioned sequences;
(3) Its HCDR1, HCDR2, HCDR3, LCDR1, LCDR2, and LCDR3 respectively having sequences as shown in SEQ ID NO: 46, 47, 58, 49, 50, or 51, or sequences having one or several amino acid substitutions, deletions, or additions (e.g., 1, 2, or 3 substitutions, deletions, or additions) compared to any of the aforementioned sequences;
(4) Its HCDR1, HCDR2, HCDR3, LCDR1, LCDR2, and LCDR3 respectively having sequences as shown in SEQ ID NO: 46, 60, 48, 49, 50, or 51, or sequences having one or several amino acid substitutions, deletions, or additions (e.g., 1, 2, or 3 substitutions, deletions, or additions) compared to any of the aforementioned sequences;
(5) Its HCDR1, HCDR2, HCDR3, LCDR1, LCDR2, and LCDR3 respectively having sequences as shown in SEQ ID NO: 46, 61, 48, 49, 50, or 51, or sequences having one or several amino acid substitutions, deletions, or additions (e.g., 1, 2, or 3 substitutions, deletions, or additions) compared to any of the aforementioned sequences;
(6) Its HCDR1, HCDR2, HCDR3, LCDR1, LCDR2, and LCDR3 respectively having sequences as shown in SEQ ID NO: 46, 63, 48, 49, 50, or 51, or sequences having one or several amino acid substitutions, deletions, or additions (e.g., 1, 2, or 3 substitutions, deletions, or additions) compared to any of the aforementioned sequences;
(7) Its HCDR1, HCDR2, HCDR3, LCDR1, LCDR2, and LCDR3 respectively having sequences as shown in SEQ ID NO: 46, 65, 48, 49, 50, or 51, or sequences having one or several amino acid substitutions, deletions, or additions (e.g., 1, 2, or 3 substitutions, deletions, or additions) compared to any of the aforementioned sequences;
(8) Its HCDR1, HCDR2, HCDR3, LCDR1, LCDR2, and LCDR3 respectively having sequences as shown in SEQ ID NO: 46, 47, 48, 49, 50, or 66, or sequences having one or several amino acid substitutions, deletions, or additions (e.g., 1, 2, or 3 substitutions, deletions, or additions) compared to any of the aforementioned sequences;
(9) Its HCDR1, HCDR2, HCDR3, LCDR1, LCDR2, and LCDR3 respectively having sequences as shown in SEQ ID NO: 46, 47, 48, 49, 50, or 67, or sequences having one or several amino acid substitutions, deletions, or additions (e.g., 1, 2, or 3 substitutions, deletions, or additions) compared to any of the aforementioned sequences;
(10) Its HCDR1, HCDR2, HCDR3, LCDR1, LCDR2, and LCDR3 respectively having sequences as shown in SEQ ID NO: 46, 47, 48, 49, 50, or 68, or sequences having one or several amino acid substitutions, deletions, or additions (e.g., 1, 2, or 3 substitutions, deletions, or additions) compared to any of the aforementioned sequences;
(11) Its HCDR1, HCDR2, HCDR3, LCDR1, LCDR2, and LCDR3 respectively having sequences as shown in SEQ ID NO: 46, 63, 58, 49, 50, or 51, or sequences having one or several amino acid substitutions, deletions, or additions (e.g., 1, 2, or 3 substitutions, deletions, or additions) compared to any of the aforementioned sequences;
(12) Its HCDR1, HCDR2, HCDR3, LCDR1, LCDR2, and LCDR3 respectively having sequences as shown in SEQ ID NO: 46, 47, 69, 49, 50, or 51, or sequences having one or several amino acid substitutions, deletions, or additions (e.g., 1, 2, or 3 substitutions, deletions, or additions) compared to any of the aforementioned sequences;
(13) Its HCDR1, HCDR2, HCDR3, LCDR1, LCDR2, and LCDR3 respectively having sequences as shown in SEQ ID NO: 46, 47, 71, 49, 50, or 51, or sequences having one or several amino acid substitutions, deletions, or additions (e.g., 1, 2, or 3 substitutions, deletions, or additions) compared to any of the aforementioned sequences;
(14) Its HCDR1, HCDR2, HCDR3, LCDR1, LCDR2, and LCDR3 respectively having sequences as shown in SEQ ID NO: 46, 47, 73, 49, 50, or 51, or sequences having one or several amino acid substitutions, deletions, or additions (e.g., 1, 2, or 3 substitutions, deletions, or additions) compared to any of the aforementioned sequences;
(15) Its HCDR1, HCDR2, HCDR3, LCDR1, LCDR2, and LCDR3 respectively having sequences as shown in SEQ ID NO: 46, 47, 75, 49, 50, or 51, or sequences having one or several amino acid substitutions, deletions, or additions (e.g., 1, 2, or 3 substitutions, deletions, or additions) compared to any of the aforementioned sequences;
(16) Its HCDR1, HCDR2, HCDR3, LCDR1, LCDR2, and LCDR3 respectively having sequences as shown in SEQ ID NO: 46, 47, 77, 49, 50, or 51, or sequences having one or several amino acid substitutions, deletions, or additions (e.g., 1, 2, or 3 substitutions, deletions, or additions) compared to any of the aforementioned sequences;
(17) Its HCDR1, HCDR2, HCDR3, LCDR1, LCDR2, and LCDR3 respectively having sequences as shown in SEQ ID NO: 46, 47, 79, 49, 50, or 51, or sequences having one or several amino acid substitutions, deletions, or additions (e.g., 1, 2, or 3 substitutions, deletions, or additions) compared to any of the aforementioned sequences;
(18) Its HCDR1, HCDR2, HCDR3, LCDR1, LCDR2, and LCDR3 respectively having sequences as shown in SEQ ID NO: 46, 47, 81, 49, 50, or 51, or sequences having one or several amino acid substitutions, deletions, or additions (e.g., 1, 2, or 3 substitutions, deletions, or additions) compared to any of the aforementioned sequences;
(19) Its HCDR1, HCDR2, HCDR3, LCDR1, LCDR2, and LCDR3 respectively having sequences as shown in SEQ ID NO: 46, 47, 83, 49, 50, or 51, or sequences having one or several amino acid substitutions, deletions, or additions (e.g., 1, 2, or 3 substitutions, deletions, or additions) compared to any of the aforementioned sequences;
(20) Its HCDR1, HCDR2, HCDR3, LCDR1, LCDR2, and LCDR3 respectively having sequences as shown in SEQ ID NO: 46, 47, 85, 49, 50, or 51, or sequences having one or several amino acid substitutions, deletions, or additions (e.g., 1, 2, or 3 substitutions, deletions, or additions) compared to any of the aforementioned sequences;
(21) Its HCDR1, HCDR2, HCDR3, LCDR1, LCDR2, and LCDR3 respectively having sequences as shown in SEQ ID NO: 46, 47, 87, 49, 50, or 51, or sequences having one or several amino acid substitutions, deletions, or additions (e.g., 1, 2, or 3 substitutions, deletions, or additions) compared to any of the aforementioned sequences;
(22) Its HCDR1, HCDR2, HCDR3, LCDR1, LCDR2, and LCDR3 respectively having sequences as shown in SEQ ID NO: 46, 47, 89, 49, 50, or 51, or sequences having one or several amino acid substitutions, deletions, or additions (e.g., 1, 2, or 3 substitutions, deletions, or additions) compared to any of the aforementioned sequences.

In certain preferred embodiments, the substitutions in any of the above items are conservative substitutions.

In certain preferred embodiments, the antibody or antigen-binding fragment comprises three VH variable region CDRs and three VL variable region CDRs as defined by the IMGT numbering system, selected from the group consisting of:
(1) Its HCDR1, HCDR2, HCDR3, LCDR1, LCDR2, and LCDR3 respectively having sequences as shown in SEQ ID NO: 52, 53, 54, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(2) Its HCDR1, HCDR2, HCDR3, LCDR1, LCDR2, and LCDR3 respectively having sequences as shown in SEQ ID NO: 52, 53, 57, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(3) Its HCDR1, HCDR2, HCDR3, LCDR1, LCDR2, and LCDR3 respectively having sequences as shown in SEQ ID NO: 52, 53, 59, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(4) Its HCDR1, HCDR2, HCDR3, LCDR1, LCDR2, and LCDR3 respectively having sequences as shown in SEQ ID NO: 52, 62, 54, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(5) Its HCDR1, HCDR2, HCDR3, LCDR1, LCDR2, and LCDR3 respectively having sequences as shown in SEQ ID NO: 52, 65, 54, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(6) Its HCDR1, HCDR2, HCDR3, LCDR1, LCDR2, and LCDR3 respectively having sequences as shown in SEQ ID NO: 52, 53, 54, 55, Gly-Thr-Ser, or SEQ ID NO: 66, or sequences having one or several amino acid substitutions, deletions, or additions (e.g., 1, 2, or 3 substitutions, deletions, or additions) compared to any of the aforementioned sequences;
(7) Its HCDR1, HCDR2, HCDR3, LCDR1, LCDR2, and LCDR3 respectively having sequences as shown in SEQ ID NO: 52, 53, 54, 55, Gly-Thr-Ser, or SEQ ID NO: 67, or sequences having one or several amino acid substitutions, deletions, or additions (e.g., 1, 2, or 3 substitutions, deletions, or additions) compared to any of the aforementioned sequences;
(8) Its HCDR1, HCDR2, HCDR3, LCDR1, LCDR2, and LCDR3 respectively having sequences as shown in SEQ ID NO: 52, 53, 54, 55, Gly-Thr-Ser, or SEQ ID NO: 68, or sequences having one or several amino acid substitutions, deletions, or additions (e.g., 1, 2, or 3 substitutions, deletions, or additions) compared to any of the aforementioned sequences;
(9) Its HCDR1, HCDR2, HCDR3, LCDR1, LCDR2, and LCDR3 respectively having sequences as shown in SEQ ID NO: 52, 64, 59, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(10) Its HCDR1, HCDR2, HCDR3, LCDR1, LCDR2, and LCDR3 respectively having sequences as shown in SEQ ID NO: 52, 53, 70, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(11) Its HCDR1, HCDR2, HCDR3, LCDR1, LCDR2, and LCDR3 respectively having sequences as shown in SEQ ID NO: 52, 53, 72, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(12) Its HCDR1, HCDR2, HCDR3, LCDR1, LCDR2, and LCDR3 respectively having sequences as shown in SEQ ID NO: 52, 53, 74, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(13) Its HCDR1, HCDR2, HCDR3, LCDR1, LCDR2, and LCDR3 respectively having sequences as shown in SEQ ID NO: 52, 53, 76, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(14) Its HCDR1, HCDR2, HCDR3, LCDR1, LCDR2, and LCDR3 respectively having sequences as shown in SEQ ID NO: 52, 53, 78, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(15) Its HCDR1, HCDR2, HCDR3, LCDR1, LCDR2, and LCDR3 respectively having sequences as shown in SEQ ID NO: 52, 53, 80, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(16) Its HCDR1, HCDR2, HCDR3, LCDR1, LCDR2, and LCDR3 respectively having sequences as shown in SEQ ID NO: 52, 53, 82, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(17) Its HCDR1, HCDR2, HCDR3, LCDR1, LCDR2, and LCDR3 respectively having sequences as shown in SEQ ID NO: 52, 53, 84, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(18) Its HCDR1, HCDR2, HCDR3, LCDR1, LCDR2, and LCDR3 respectively having sequences as shown in SEQ ID NO: 52, 53, 86, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(19) Its HCDR1, HCDR2, HCDR3, LCDR1, LCDR2, and LCDR3 respectively having sequences as shown in SEQ ID NO: 52, 53, 88, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(20) Its HCDR1, HCDR2, HCDR3, LCDR1, LCDR2, and LCDR3 respectively having sequences as shown in SEQ ID NO: 52, 53, 90, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences.

In certain preferred embodiments, the substitutions in any of the above items are conservative substitutions.

In certain embodiments, the antibody or antigen-binding fragment is humanized. Example 3 provides a basic workflow of the humanization strategy, and Table 3 lists the amino acid sequence numbers of the variable regions of preferred humanized antibodies.

In certain preferred embodiments, the humanized antibody or antigen-binding fragment comprises the following VH and VL sequences: A VH domain comprising the amino acid sequence as shown in SEQ ID NO: 2, or a sequence substantially identical thereto (e.g., having at least 80%, 85%, 90%, 92%, 95%, 97%, 98%, 99%, or higher identity, or having one or more amino acid substitutions, such as conservative substitutions); and a VL domain comprising the amino acid sequence as shown in SEQ ID NO: 3, or a sequence substantially identical thereto (e.g., having at least 80%, 85%, 90%, 92%, 95%, 97%, 98%, 99%, or higher identity, or having one or more amino acid substitutions, such as conservative substitutions).

In certain preferred embodiments, the humanized antibody or antigen-binding fragment comprises the following VH and VL sequences: A VH domain comprising the amino acid sequence as shown in SEQ ID NO: 4, or a sequence substantially identical thereto (e.g., having at least 80%, 85%, 90%, 92%, 95%, 97%, 98%, 99%, or higher identity, or having one or more amino acid substitutions, such as conservative substitutions); and a VL domain comprising the amino acid sequence as shown in SEQ ID NO: 5, or a sequence substantially identical thereto (e.g., having at least 80%, 85%, 90%, 92%, 95%, 97%, 98%, 99%, or higher identity, or having one or more amino acid substitutions, such as conservative substitutions).

In certain preferred embodiments, the humanized antibody or antigen-binding fragment comprises the following VH and VL sequences: A VH domain comprising the amino acid sequence as shown in SEQ ID NO: 6, or a sequence substantially identical thereto (e.g., having at least 80%, 85%, 90%, 92%, 95%, 97%, 98%, 99%, or higher identity, or having one or more amino acid substitutions, such as conservative substitutions); and a VL domain comprising the amino acid sequence as shown in SEQ ID NO: 7, or a sequence substantially identical thereto (e.g., having at least 80%, 85%, 90%, 92%, 95%, 97%, 98%, 99%, or higher identity, or having one or more amino acid substitutions, such as conservative substitutions).

In certain preferred embodiments, the humanized antibody or antigen-binding fragment comprises the following VH and VL sequences: A VH domain comprising the amino acid sequence as shown in SEQ ID NO: 8, or a sequence substantially identical thereto (e.g., having at least 80%, 85%, 90%, 92%, 95%, 97%, 98%, 99%, or higher identity, or having one or more amino acid substitutions, such as conservative substitutions); and a VL domain comprising the amino acid sequence as shown in SEQ ID NO: 9, or a sequence substantially identical thereto (e.g., having at least 80%, 85%, 90%, 92%, 95%, 97%, 98%, 99%, or higher identity, or having one or more amino acid substitutions, such as conservative substitutions).

In certain preferred embodiments, the humanized antibody or antigen-binding fragment comprises the following VH and VL sequences: A VH domain comprising the amino acid sequence as shown in SEQ ID NO: 10, or a sequence substantially identical thereto (e.g., having at least 80%, 85%, 90%, 92%, 95%, 97%, 98%, 99%, or higher identity, or having one or more amino acid substitutions, such as conservative substitutions); and a VL domain comprising the amino acid sequence as shown in SEQ ID NO: 11, or a sequence substantially identical thereto (e.g., having at least 80%, 85%, 90%, 92%, 95%, 97%, 98%, 99%, or higher identity, or having one or more amino acid substitutions, such as conservative substitutions).

In certain preferred embodiments, the humanized antibody or antigen-binding fragment comprises the following VH and VL sequences: A VH domain comprising the amino acid sequence as shown in SEQ ID NO: 12, or a sequence substantially identical thereto (e.g., having at least 80%, 85%, 90%, 92%, 95%, 97%, 98%, 99%, or higher identity, or having one or more amino acid substitutions, such as conservative substitutions); and a VL domain comprising the amino acid sequence as shown in SEQ ID NO: 13, or a sequence substantially identical thereto (e.g., having at least 80%, 85%, 90%, 92%, 95%, 97%, 98%, 99%, or higher identity, or having one or more amino acid substitutions, such as conservative substitutions).

In certain preferred embodiments, the humanized antibody or antigen-binding fragment comprises the following VH and VL sequences: A VH domain comprising the amino acid sequence as shown in SEQ ID NO: 14, or a sequence substantially identical thereto (e.g., having at least 80%, 85%, 90%, 92%, 95%, 97%, 98%, 99%, or higher identity, or having one or more amino acid substitutions, such as conservative substitutions); and a VL domain comprising the amino acid sequence as shown in SEQ ID NO: 15, or a sequence substantially identical thereto (e.g., having at least 80%, 85%, 90%, 92%, 95%, 97%, 98%, 99%, or higher identity, or having one or more amino acid substitutions, such as conservative substitutions).

In certain preferred embodiments, the humanized antibody or antigen-binding fragment comprises the following VH and VL sequences: A VH domain comprising the amino acid sequence as shown in SEQ ID NO: 16, or a sequence substantially identical thereto (e.g., having at least 80%, 85%, 90%, 92%, 95%, 97%, 98%, 99%, or higher identity, or having one or more amino acid substitutions, such as conservative substitutions); and a VL domain comprising the amino acid sequence as shown in SEQ ID NO: 17, or a sequence substantially identical thereto (e.g., having at least 80%, 85%, 90%, 92%, 95%, 97%, 98%, 99%, or higher identity, or having one or more amino acid substitutions, such as conservative substitutions).

In certain preferred embodiments, the humanized antibody or antigen-binding fragment comprises the following VH and VL sequences: A VH domain comprising the amino acid sequence as shown in SEQ ID NO: 18, or a sequence substantially identical thereto (e.g., having at least 80%, 85%, 90%, 92%, 95%, 97%, 98%, 99%, or higher identity, or having one or more amino acid substitutions, such as conservative substitutions); and a VL domain comprising the amino acid sequence as shown in SEQ ID NO: 19, or a sequence substantially identical thereto (e.g., having at least 80%, 85%, 90%, 92%, 95%, 97%, 98%, 99%, or higher identity, or having one or more amino acid substitutions, such as conservative substitutions).

In certain preferred embodiments, the humanized antibody or antigen-binding fragment comprises the following VH and VL sequences: A VH domain comprising the amino acid sequence as shown in SEQ ID NO: 20, or a sequence substantially identical thereto (e.g., having at least 80%, 85%, 90%, 92%, 95%, 97%, 98%, 99%, or higher identity, or having one or more amino acid substitutions, such as conservative substitutions); and a VL domain comprising the amino acid sequence as shown in SEQ ID NO: 21, or a sequence substantially identical thereto (e.g., having at least 80%, 85%, 90%, 92%, 95%, 97%, 98%, 99%, or higher identity, or having one or more amino acid substitutions, such as conservative substitutions).

In certain preferred embodiments, the humanized antibody or antigen-binding fragment comprises the following VH and VL sequences: A VH domain comprising the amino acid sequence as shown in SEQ ID NO: 22, or a sequence substantially identical thereto (e.g., having at least 80%, 85%, 90%, 92%, 95%, 97%, 98%, 99%, or higher identity, or having one or more amino acid substitutions, such as conservative substitutions); and a VL domain comprising the amino acid sequence as shown in SEQ ID NO: 23, or a sequence substantially identical thereto (e.g., having at least 80%, 85%, 90%, 92%, 95%, 97%, 98%, 99%, or higher identity, or having one or more amino acid substitutions, such as conservative substitutions).

In certain preferred embodiments, the humanized antibody or antigen-binding fragment comprises the following VH and VL sequences: A VH domain comprising the amino acid sequence as shown in SEQ ID NO: 24, or a sequence substantially identical thereto (e.g., having at least 80%, 85%, 90%, 92%, 95%, 97%, 98%, 99%, or higher identity, or having one or more amino acid substitutions, such as conservative substitutions); and a VL domain comprising the amino acid sequence as shown in SEQ ID NO: 25, or a sequence substantially identical thereto (e.g., having at least 80%, 85%, 90%, 92%, 95%, 97%, 98%, 99%, or higher identity, or having one or more amino acid substitutions, such as conservative substitutions).

In certain preferred embodiments, the humanized antibody or antigen-binding fragment comprises the following VH and VL sequences: A VH domain comprising the amino acid sequence as shown in SEQ ID NO: 26, or a sequence substantially identical thereto (e.g., having at least 80%, 85%, 90%, 92%, 95%, 97%, 98%, 99%, or higher identity, or having one or more amino acid substitutions, such as conservative substitutions); and a VL domain comprising the amino acid sequence as shown in SEQ ID NO: 27, or a sequence substantially identical thereto (e.g., having at least 80%, 85%, 90%, 92%, 95%, 97%, 98%, 99%, or higher identity, or having one or more amino acid substitutions, such as conservative substitutions).

In certain preferred embodiments, the humanized antibody or antigen-binding fragment comprises the following VH and VL sequences: A VH domain comprising the amino acid sequence as shown in SEQ ID NO: 28, or a sequence substantially identical thereto (e.g., having at least 80%, 85%, 90%, 92%, 95%, 97%, 98%, 99%, or higher identity, or having one or more amino acid substitutions, such as conservative substitutions); and a VL domain comprising the amino acid sequence as shown in SEQ ID NO: 29, or a sequence substantially identical thereto (e.g., having at least 80%, 85%, 90%, 92%, 95%, 97%, 98%, 99%, or higher identity, or having one or more amino acid substitutions, such as conservative substitutions).

In certain preferred embodiments, the humanized antibody or antigen-binding fragment comprises the following VH and VL sequences: A VH domain comprising the amino acid sequence as shown in SEQ ID NO: 30, or a sequence substantially identical thereto (e.g., having at least 80%, 85%, 90%, 92%, 95%, 97%, 98%, 99%, or higher identity, or having one or more amino acid substitutions, such as conservative substitutions); and a VL domain comprising the amino acid sequence as shown in SEQ ID NO: 31, or a sequence substantially identical thereto (e.g., having at least 80%, 85%, 90%, 92%, 95%, 97%, 98%, 99%, or higher identity, or having one or more amino acid substitutions, such as conservative substitutions).

In certain preferred embodiments, the humanized antibody or antigen-binding fragment comprises the following VH and VL sequences: A VH domain comprising the amino acid sequence as shown in SEQ ID NO: 32, or a sequence substantially identical thereto (e.g., having at least 80%, 85%, 90%, 92%, 95%, 97%, 98%, 99%, or higher identity, or having one or more amino acid substitutions, such as conservative substitutions); and a VL domain comprising the amino acid sequence as shown in SEQ ID NO: 33, or a sequence substantially identical thereto (e.g., having at least 80%, 85%, 90%, 92%, 95%, 97%, 98%, 99%, or higher identity, or having one or more amino acid substitutions, such as conservative substitutions).

In certain preferred embodiments, the humanized antibody or antigen-binding fragment comprises the following VH and VL sequences: A VH domain comprising the amino acid sequence as shown in SEQ ID NO: 34, or a sequence substantially identical thereto (e.g., having at least 80%, 85%, 90%, 92%, 95%, 97%, 98%, 99%, or higher identity, or having one or more amino acid substitutions, such as conservative substitutions); and a VL domain comprising the amino acid sequence as shown in SEQ ID NO: 35, or a sequence substantially identical thereto (e.g., having at least 80%, 85%, 90%, 92%, 95%, 97%, 98%, 99%, or higher identity, or having one or more amino acid substitutions, such as conservative substitutions).

In certain preferred embodiments, the humanized antibody or antigen-binding fragment comprises the following VH and VL sequences: A VH domain comprising the amino acid sequence as shown in SEQ ID NO: 36, or a sequence substantially identical thereto (e.g., having at least 80%, 85%, 90%, 92%, 95%, 97%, 98%, 99%, or higher identity, or having one or more amino acid substitutions, such as conservative substitutions); and a VL domain comprising the amino acid sequence as shown in SEQ ID NO: 37, or a sequence substantially identical thereto (e.g., having at least 80%, 85%, 90%, 92%, 95%, 97%, 98%, 99%, or higher identity, or having one or more amino acid substitutions, such as conservative substitutions).

In certain preferred embodiments, the humanized antibody or antigen-binding fragment comprises the following VH and VL sequences: A VH domain comprising the amino acid sequence as shown in SEQ ID NO: 38, or a sequence substantially identical thereto (e.g., having at least 80%, 85%, 90%, 92%, 95%, 97%, 98%, 99%, or higher identity, or having one or more amino acid substitutions, such as conservative substitutions); and a VL domain comprising the amino acid sequence as shown in SEQ ID NO: 39, or a sequence substantially identical thereto (e.g., having at least 80%, 85%, 90%, 92%, 95%, 97%, 98%, 99%, or higher identity, or having one or more amino acid substitutions, such as conservative substitutions).

In certain preferred embodiments, the humanized antibody or antigen-binding fragment comprises the following VH and VL sequences: A VH domain comprising the amino acid sequence as shown in SEQ ID NO: 40, or a sequence substantially identical thereto (e.g., having at least 80%, 85%, 90%, 92%, 95%, 97%, 98%, 99%, or higher identity, or having one or more amino acid substitutions, such as conservative substitutions); and a VL domain comprising the amino acid sequence as shown in SEQ ID NO: 41, or a sequence substantially identical thereto (e.g., having at least 80%, 85%, 90%, 92%, 95%, 97%, 98%, 99%, or higher identity, or having one or more amino acid substitutions, such as conservative substitutions).

In certain preferred embodiments, the humanized antibody or antigen-binding fragment comprises the following VH and VL sequences: A VH domain comprising the amino acid sequence as shown in SEQ ID NO: 42, or a sequence substantially identical thereto (e.g., having at least 80%, 85%, 90%, 92%, 95%, 97%, 98%, 99%, or higher identity, or having one or more amino acid substitutions, such as conservative substitutions); and a VL domain comprising the amino acid sequence as shown in SEQ ID NO: 43, or a sequence substantially identical thereto (e.g., having at least 80%, 85%, 90%, 92%, 95%, 97%, 98%, 99%, or higher identity, or having one or more amino acid substitutions, such as conservative substitutions).

In certain preferred embodiments, the humanized antibody or antigen-binding fragment comprises the following VH and VL sequences: A VH domain comprising the amino acid sequence as shown in SEQ ID NO: 44, or a sequence substantially identical thereto (e.g., having at least 80%, 85%, 90%, 92%, 95%, 97%, 98%, 99%, or higher identity, or having one or more amino acid substitutions, such as conservative substitutions); and a VL domain comprising the amino acid sequence as shown in SEQ ID NO: 45, or a sequence substantially identical thereto (e.g., having at least 80%, 85%, 90%, 92%, 95%, 97%, 98%, 99%, or higher identity, or having one or more amino acid substitutions, such as conservative substitutions).

In certain embodiments, the antibody or antigen-binding fragment is a full-length antibody, Fab, Fab', (Fab')2, Fd, Fv, dAb, scFv, or scFv-scFv.

In certain embodiments, the antibody comprises a heavy chain constant region and a light chain constant region derived from a human immunoglobulin.

Preferably, the antibody comprises an amino acid sequence of a human κ light chain constant region.

Preferably, the antibody comprises a heavy chain constant region selected from the group consisting of human IgG1, IgG2, IgG3, IgG4, IgM, IgA1, IgA2, IgD, and IgE; more preferably, it comprises a heavy chain constant region selected from the group consisting of human IgG1, IgG2, and IgG4; and the heavy chain constant region has a native sequence or a sequence having one or more amino acid substitutions, deletions, or additions compared to the native sequence from which it is derived.

Preferably, the antibody comprises a heavy chain constant region of human IgG4, and the heavy chain constant region contains an amino acid mutation S228P.

In any of the above embodiments, the antibody or antigen-binding fragment thereof of the present invention is capable of binding to TFPI with a K_{D} of 10 nM or lower, preferably with a K_{D} of 1 nM or lower; more preferably with a K_{D} of 100 pM or lower; and even more preferably with a K_{D} of 10 pM or lower.

In one aspect, the present invention provides a bispecific antibody or antigen-binding portion thereof, comprising the antibody or antigen-binding portion thereof described above linked to a second antibody or antigen-binding portion thereof. The bispecific antibody or antigen-binding portion is characterized in that the second antibody or antigen-binding portion thereof binds to tissue factor pathway inhibitor.

In one aspect, the present invention provides a DNA molecule encoding the antibody or antigen-binding fragment thereof described above.

In one aspect, the present invention provides a vector comprising the DNA molecule described above.

In one aspect, the invention provides a host cell comprising the vector described above; the host cell comprises prokaryotic cells, yeast, or mammalian cells, such as CHO cells, NS0 cells, or other mammalian cells, preferably CHO cells.

In one aspect, the present invention provides a pharmaceutical composition comprising the antibody or antigen-binding fragment thereof described above, and a pharmaceutically acceptable excipient, carrier, or diluent.

In certain embodiments, the pharmaceutical composition may be administered to a patient via subcutaneous injection. For example, an anti-TFPI antibody at a dose of 10 to 500 mg may be administered to the patient via subcutaneous injection at a frequency of once daily, once every 2 days, once every 3 days, once every 4 days, once every 5 days, once every 6 days, twice weekly, once weekly, once every two weeks, or once monthly.

In one aspect, the present invention provides the use of the antibody or antigen-binding fragment thereof in the manufacture of a medicament for preventing or treating a disease or event associated with hereditary or acquired coagulation factor deficiency. For example, the antibody or antigen-binding fragment thereof provided by the present invention can be used to block the interaction between TFPI and FXa or to prevent TFPI-dependent inhibition of TF/FVIIa activity. Furthermore, the human monoclonal antibody can also be used to restore TF/FVIIa-driven FXa generation, thereby bypassing the insufficient amplification of FXa dependent on FVIII or FIX. Further, the diseases include hemophilia A and hemophilia B, and also encompass spontaneous bleeding events in hemophilia patients, traumatic bleeding events, or bleeding events occurring during surgical prophylaxis, perioperative management, or surgical treatment.

In other aspects, the present invention provides a method for preventing or treating a disease or event associated with hereditary or acquired coagulation factor deficiency, the method comprising administering an effective amount of the antibody or antigen-binding fragment thereof of the present invention, or the pharmaceutical composition of the present invention.

In one aspect, the present invention provides the use of the antibody or antigen-binding fragment thereof in the manufacture of a medicament for preventing or treating a hereditary coagulation disorder. Preferably, the hereditary coagulation disorder is Glanzmann thrombasthenia or Bernard-Soulier syndrome.

The technical solution provided by the present invention has achieved beneficial effects, summarized as follows:
(1) The TFPI antibody provided by the present invention exhibits good binding affinity, can specifically recognize human TFPI, and is effective for treating and preventing hemophilia, as validated in clinical studies.
(2) The TFPI antibody provided by the present invention is a pH-dependent antigen-binding recycling antibody. Binding affinity between the antibody and antigen varies with pH, and the ratio of its KD and/or koff for binding to TFPI at pH ≤ 6.0 relative to that at pH 7.4 is 2 or greater. Human plasma is neutral (approximately pH 7.4), whereas intracellular space is acidic. This pH-dependent antigen-binding recycling antibody can bind antigens in plasma. After the antigen-antibody complex is internalized into cells, under acidic endosomal conditions (pH ≤ 6.0), the pH-dependent antigen-binding recycling antibody dissociates from the antigen. The dissociated antibody is captured by FcRn and recycled to the extracellular space. In the neutral extracellular space, FcRn releases the antibody, allowing it to return to the plasma and bind to other antigens, thereby enabling antibody recycling and resulting in an extended half-life. Pharmacokinetic studies have also demonstrated longer half-life of the antibody of the present invention. At a dose of 50 mg/kg, the TFPI antibody provided by the present invention achieved a half-life of 136 hours in rhesus monkeys. At a dose of 100 mg per human, the TFPI antibody provided by the present invention achieved a half-life of 146 hours in healthy subjects, which will significantly reduce its clinical dosing frequency.
(3) The TFPI antibody provided by the present invention has a stable downstream production process, simple and efficient purification steps, and a homogeneous expression product. Its physicochemical and in vivo stability are significantly improved, and it exhibits good safety, demonstrating substantial clinical value.

### Abbreviations and Definition of Terms

The following abbreviations are used herein:
CDR: Complementarity-Determining Region; the regions within the variable domain of an immunoglobulin that are hypervariable, defined by the Kabat, IMGT, Chothia, or AbM numbering systems (see the term "hypervariable region" or "CDR region" or "complementarity determining region").
EC₅₀: Concentration producing 50% of the maximal effect or binding.
ELISA: Enzyme-Linked Immunosorbent Assay.
FR: Framework Region of an antibody; the immunoglobulin variable region excluding the CDR regions.
HRP: Horseradish Peroxidase.
IC50: Concentration producing 50% inhibition.
IgG: Immunoglobulin G.
Kabat: The immunoglobulin amino acid sequence alignment and numbering system pioneered by Elvin A. Kabat.
PCR: Polymerase Chain Reaction.
V region: The segment of an IgG chain with variable sequence among different antibodies, extending to Kabat residue 109 in the light chain and residue 113 in the heavy chain.
K_{D}: Equilibrium dissociation constant.
ka: Association rate constant.
kd: Dissociation rate constant.

In the present invention, unless otherwise specified, scientific and technical terms used herein have meanings commonly understood by those skilled in the art. Furthermore, laboratory procedures such as cell culture, biochemistry, nucleic acid chemistry, and immunology used herein are conventional steps widely adopted in the respective fields. To facilitate a better understanding of the invention, definitions and explanations of related terms are provided below.

The term "tissue factor pathway inhibitor" or "TFPI" refers to any variant, isoform, or species homolog of human TFPI naturally expressed by cells.

The term "EU numbering system" refers to the numbering scheme based on the amino acid sequence of the first human IgG1 immunoglobulin, designated "Eu," which was isolated and purified by Gerald M. Edelman et al. in the late 1960s (1968-1969), and for which the amino acid sequence was determined and numbered (Edelman GM et al, 1969, Proc Natl Acad USA, 63:78-85). The amino acid sequences of the heavy chain constant regions of other immunoglobulins are aligned with that of Eu, and the corresponding amino acid positions are assigned according to this numbering. The EU numbering system primarily applies to the heavy chain constant regions of immunoglobulins, including CH1, CH2, CH3, and the hinge region.

The term "Kabat numbering system" refers to the standardized numbering scheme for human immunoglobulin variable regions first proposed by Kabat et al. in 1979 (Kabat EA, Wu TT, Bilofsky H, Sequences of Immunoglobulin Chains: Tabulation and Analysis of Amino Acid Sequences of Precursors, V-regions, C-regions, J-Chain and β2-Microglobulins. 1979. Department of Health, Education, and Welfare, Public Health Service, National Institutes of Health). In the publication "Sequences of Proteins of Immunological Interest" (Kabat EA, Wu TT, Perry HM, Gottesman KS, Foeller C. 1991. Sequences of Proteins of Immunological Interest, 5th edition. Bethesda, MD: US Department of Health and Human Services, National Institutes for Health), Kabat et al. aligned and numbered the amino acid sequences of antibody light and heavy chains. They observed that these analyzed sequences exhibited variable lengths, and that deletions or insertions of amino acids or amino acid fragments could only occur at specific positions. Interestingly, insertion sites are often located within the CDRs but may also appear at certain positions in the framework regions. In the Kabat numbering scheme, the light chain variable region is numbered up to position 109, and the heavy chain variable region up to position 113. Inserted amino acids in the light and heavy chains are identified and annotated with letters (e.g., 27a, 27b, ...). All lambda light chains lack a residue at position 10, and lambda and kappa light chains are encoded by different genes located on different chromosomes. Lambda and kappa light chains can be distinguished by differences in their constant region amino acid sequences. Unlike the EU numbering system, which applies only to heavy chain constant regions, the Kabat numbering system covers the full-length immunoglobulin sequence, including the variable and constant regions of both immunoglobulin light and heavy chains.

The term "antibody" generally refers to a protein-binding molecule having the function of an immunoglobulin class. Typical examples of antibodies are immunoglobulins, as well as derivatives or functional fragments thereof, provided they exhibit the desired binding specificity.

Techniques for producing antibodies are well known in the art. "Antibody" encompasses different classes of natural immunoglobulins (e.g., IgA, IgG, IgM, IgD, and IgE) and subclasses thereof (e.g., IgG1, IgG2, IgA1, IgA2, etc.). "Antibody" also includes non-natural immunoglobulins, including, for example, single-chain antibodies, chimeric antibodies (e.g., humanized murine antibodies), and heteroconjugated antibodies (e.g., bispecific antibodies), as well as antigen-binding fragments thereof (e.g., Fab', F(ab')2, Fab, Fv, and rIgG). See also, for example, Pierce Catalog and Handbook, 1994-1995 (Pierce Chemical Co, Rockford, Ill); Kuby J, Immunology, 3rd Ed, WH Freeman & Co, New York, 1997. An antibody may bind to one antigen, referred to as "monospecific"; or to two different antigens, referred to as "bispecific"; or to more than two different antigens, referred to as "multispecific." An antibody may be monovalent, bivalent, or multivalent, i.e., the antibody may bind to one, two, or more antigen molecules at a time. An antibody binds "monovalent" to a given protein when one molecule of the antibody binds to only one molecule of that protein, but the antibody may also bind to different proteins. When an antibody binds to only one molecule of each of two different proteins, it binds "monovalent" to each protein, and the antibody is "bispecific" and binds "monovalent" to each of the two different proteins. An antibody may be "monomeric," i.e., it consists of a single polypeptide chain. An antibody may comprise multiple polypeptide chains ("multimeric") or may comprise two ("dimeric"), three ("trimeric"), or four ("tetrameric") polypeptide chains. If the antibody is multimeric, it may be a homomultimer, i.e., the antibody comprises more than one molecule of only one type of polypeptide chain, including homodimers, homotrimers, or homotetramers. Alternatively, a multimeric antibody may be a heteromultimer, i.e., the antibody comprises more than one type of different polypeptide chain, including heterodimers, heterotrimers, or heterotetramers.

The term "monoclonal antibody (mAb)" refers to an antibody obtained from a substantially homogeneous population of antibodies, e.g., the individual antibodies in the population are identical except for possibly minor naturally occurring mutations. Thus, the modifier "monoclonal" indicates that the antibody is characterized as not being a mixture of discrete antibodies. Monoclonal antibodies are produced by methods known to those skilled in the art, such as by fusion of myeloma cells with immune spleen cells to prepare hybrid antibody-producing cells. They are synthesized through hybridoma culture and are not contaminated by other immunoglobulins. Monoclonal antibodies can also be obtained using techniques such as recombinant technology, phage display technology, synthetic technology, or other existing techniques.

The term "antigenic epitope" refers to any determinant capable of being bound by an antigen-binding protein, such as an antibody or T-cell receptor. An epitope is the region of an antigen that is bound by an antigen-binding protein targeting that antigen, and when the antigen is a protein, the epitope includes the specific amino acids that directly contact the antigen-binding protein. Most commonly, epitopes are located on proteins, but in some cases may be located on other types of molecules, such as nucleic acids. Epitope determinants may include chemically active surface groups of the molecule, such as amino acids, sugar side chains, phosphoryl or sulfonyl groups, and may have specific three-dimensional structural features and/or specific charge characteristics. Typically, an antibody specific for a particular target antigen will preferentially recognize an epitope on the target antigen within a complex mixture of proteins and/or macromolecules. An antigenic epitope may be composed of a continuous sequence (primary structure of the protein) or formed by the three-dimensional structure of discontinuous parts of the protein. Antigenic epitopes are mostly located on the surface of the antigenic substance. A natural antigenic substance may have multiple and various epitopes. The larger the antigen molecule, the greater the number of epitopes.

The term "binding" refers microscopically to the interaction and/or occlusion between an antibody or its antigen-binding fragment and its corresponding target site, typically considered to occur when the corresponding amino acid residues interact at a distance of 5 Å or closer. The forces involved in such interaction and/or occlusion may include hydrogen bonds, van der Waals forces, electrostatic forces, cation-π interactions, and/or hydrophobic forces.

An antibody that "preferentially binds" or "specifically binds" (used interchangeably in the present invention) to an epitope is a term well known in the art, and methods for determining such specificity or preferential binding are also well known. A molecule is the to exhibit "specific binding" or "preferential binding" if it reacts or binds more frequently, more rapidly, with greater duration, and/or with greater affinity to a particular cell or substance compared to its reaction or binding with alternative cells or substances. An antibody "specifically binds" or "preferentially binds" to a target if it binds with greater affinity, avidity, more readily, and/or for a longer duration to the target compared to its binding to other substances. Furthermore, an antibody "specifically binds" or "preferentially binds" to a target in a sample if it binds with greater affinity, avidity, more readily, and/or for a longer duration to that target compared to its binding to other substances present in the sample. For example, an antibody that specifically or preferentially binds to a TFPI epitope is one that binds to that epitope with greater affinity, avidity, more readily, and/or for a longer duration compared to its binding to other TFPI epitopes or non-TFPI epitopes. As understood from this definition, an antibody (or portion or epitope thereof) that "specifically binds" or "preferentially binds" to a first target may or may not specifically or preferentially bind to a second target. Thus, "specific binding" or "preferential binding" does not necessarily require (though it may include) exclusive binding. Generally, but not necessarily, reference to binding implies preferential binding. "Specific binding" or "preferential binding" includes compounds, such as proteins, nucleic acids, antibodies, etc., that recognize and bind to a specific molecule in a sample but do not substantially recognize or bind to other molecules. For example, an antibody or peptide receptor that recognizes and binds to its cognate ligand or binding partner (e.g., an anti-TFPI antibody binding to TFPI) in a sample, but does not substantially recognize or bind to other molecules in the sample, specifically binds to that cognate ligand or binding partner. Therefore, under specified assay conditions, a specific binding moiety (e.g., an antibody or its antigen-binding portion, or a receptor or its ligand-binding portion) preferentially binds to a particular target molecule and does not bind in significant amounts to other components present in the test sample.

The term "pKa" refers to the negative logarithm of the acid dissociation constant of an acidic substance. The pKa value is obtained by taking the negative base-10 logarithm of the acid dissociation constant Ka, i.e., pKa = -logio(Ka). It is an indicator used to measure the strength of an acid. A lower pKa value indicates a stronger acid, while a higher pKa value indicates a weaker acid. The acid dissociation constant (Ka) refers to the equilibrium constant for the reaction in which an acid dissociates in water to produce hydrogen ions (H⁺) and the corresponding anions (A⁻). A larger Ka value indicates a stronger acid, meaning a higher degree of dissociation. For the dissociation equilibrium: HA 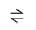 H⁺ + A⁻, Ka = [H⁺] [A⁻]/[HA], where [HA], [H⁺], and [A⁻] are the concentrations of the acid HA, hydrogen ions H⁺, and the conjugate base A⁻, respectively.

The term "antibody fragment" or "antigen-binding fragment" refers to antigen-binding fragments and antibody analogs that retain the ability to specifically bind an antigen, typically comprising at least a portion of the antigen-binding region or variable region of the parental antibody. The antibody fragment retains at least some of the binding specificity of the parental antibody. Generally, when activity is expressed in molar units (KD), the antibody fragment retains at least 10% of the binding activity of the parental antibody. Preferably, the antibody fragment retains at least 20%, 50%, 70%, 80%, 90%, 95%, or 100% of binding affinity of the parental antibody for the target. Antibody fragments include, but are not limited to: Fab fragments, Fab' fragments, F(ab')₂ fragments, Fv fragments, Fd fragments, complementarity-determining region (CDR) fragments, disulfide-stabilized proteins (dsFv), etc.; linear antibodies, single-chain antibodies (e.g., scFv antibodies), unibodies (technology from Genmab), bivalent single-chain antibodies, single-chain phage antibodies, single-domain antibodies (e.g., VH domain antibodies), domain antibodies (technology from Domantis), nanobodies (technology from Ablynx); multispecific antibodies formed from antibody fragments (e.g., triabodies, tetrabodies, etc.); and engineered antibodies such as chimeric antibodies (e.g., humanized murine antibodies), heteroconjugate antibodies, etc. These antibody fragments are obtained using conventional techniques known to those skilled in the art and screened for utility in the same manner as intact antibodies.

The term "single-chain Fv antibody" (or "scFv antibody") refers to an antibody fragment comprising the VH and VL domains of an antibody, which is a recombinant protein consisting of VH and VL connected by a linker that enables these two domains to associate and form an antigen-binding site. The linker sequence typically consists of flexible peptides, for example but not limited to G₂(GGGGS)₃. The size of an scFv is generally about one-sixth that of a full-length antibody. A single-chain antibody is preferably a single amino acid chain encoded by a single nucleotide chain. For a review on scFv, see Pluckthun A, 1994. Antibodies from Escherichia coli, in The Pharmacology of Monoclonal Antibodies, Vol 113, Rosenberg M and Moore GP (EDs.), Springer-Verlag, New York, pp 269-315. See also International Patent Application Publication No. WO 88/01649 and U.S. Patent Nos. 4,946,778 and 5,260,203.

The term "VL domain" refers to the amino-terminal variable region domain of an immunoglobulin light chain.

The term "VH domain" refers to the amino-terminal variable region domain of an immunoglobulin heavy chain.

The term "hinge region" encompasses the portion of the heavy chain molecule that links the CH1 domain to the CH2 domain. This hinge region comprises approximately 25 residues and is flexible, allowing independent movement of the two N-terminal antigen-binding regions. The hinge region can be divided into three distinct domains: upper, middle, and lower hinge domains (Roux KH et al., 1998, J Immunol, 161:4083-4090).

The term "Fv region" comprises the variable regions from both the heavy and light chains but lacks constant regions. It is the smallest fragment containing a complete antigen recognition and binding site.

The term "heavy chain constant region" encompasses amino acid sequences derived from an immunoglobulin heavy chain. A polypeptide comprising a heavy chain constant region comprises at least one of the following: a CH1 domain, a hinge domain (e.g., upper hinge region, middle hinge region, and/or lower hinge region), a CH2 domain, a CH3 domain, or a variant or fragment thereof. For example, an antigen-binding polypeptide used in the present application may comprise a polypeptide chain with a CH1 domain; a polypeptide with a CH1 domain, at least part of a hinge domain, and a CH2 domain; a polypeptide chain with a CH1 domain and a CH3 domain; a polypeptide chain with a CH1 domain, at least part of a hinge domain, and a CH3 domain; or a polypeptide chain with a CH1 domain, at least part of a hinge region, a CH2 domain, and a CH3 domain. In another embodiment, a polypeptide of the present application comprises a polypeptide chain with a CH3 domain. Furthermore, an antibody used in the present application may lack at least a portion of the CH2 domain (e.g., all or part of the CH2 domain). As described above, those skilled in the art will understand that the heavy chain constant region may be modified such that its amino acid sequence differs from that of a naturally occurring immunoglobulin molecule.

The term "light chain constant region" encompasses amino acid sequences derived from an antibody light chain. Preferably, the light chain constant region comprises at least one of a constant kappa domain and a constant lambda domain.

The term "Fc region" or "Fc fragment" refers to the C-terminal region of an immunoglobulin heavy chain, which comprises at least a portion of the hinge region, the CH2 domain, and the CH3 domain. This region mediates the binding of immunoglobulins to host tissues or factors, including binding to Fc receptors on various cells of the immune system (e.g., effector cells) or binding to the first component (C1q) of the classical complement system. The Fc region includes both native sequence Fc regions and variant Fc regions.

Typically, the Fc region of a human IgG heavy chain is the segment extending from the amino acid residue at its Cys 226 or Pro 230 to the carboxy terminus, although the boundaries may vary. The C-terminal lysine of the Fc region (residue 447 according to the EU numbering system) may be present or absent. Fc can also refer to this region when it exists independently or in the context of a protein polypeptide comprising the Fc region, such as a "binding protein comprising an Fc region," also termed an "Fc fusion protein" (e.g., an antibody or immunoadhesin). The native sequence Fc region in the antibodies of the present invention is derived from human IgG1, IgG2 (IgG2A, IgG2B), IgG3, and IgG4. In certain embodiments, relative to the amino acid sequence of a mammalian Fc polypeptide, the amino acid sequences of the two Fc polypeptide chains comprise approximately 10 single amino acid substitutions, insertions, and/or deletions per 100 amino acids. In some embodiments, the amino acid differences in the Fc region described above may be Fc alterations that extend half-life, enhance FcRn binding, enhance Fcγ receptor (FcγR) binding, and/or enhance ADCC, ADCP, and/or CDC.

The term "immune response" refers to the actions of immune cells (e.g., lymphocytes, antigen-presenting cells, phagocytes, or granulocytes) and soluble macromolecules produced by immune cells or the liver (including antibodies, cytokines, and complement), which result in selective damage, destruction, or elimination of invasive pathogens, cells or tissues infected by pathogens, cancer cells, or, in cases of autoimmunity or pathological inflammation, normal human cells or tissues from the body. In the present invention, the term "antigen-specific T cell response" refers to an immune response generated by T cells upon stimulation by an antigen specific to those T cells. Non-limiting examples of responses produced by T cells upon antigen-specific stimulation include T cell proliferation and the production of cytokines (e.g., IL-2).

The term "antibody-dependent cell-mediated cytotoxicity (ADCC)" refers to a form of cytotoxicity in which immunoglobulins (Ig) bind to Fc receptors (FcR) present on cytotoxic cells (e.g., natural killer (NK) cells, neutrophils, or macrophages), enabling these cytotoxic effector cells to specifically bind to target cells coated with antigen, subsequently killing the target cells through the secretion of cytotoxins. Methods for detecting the ADCC activity of antibodies are known in the art, for example, by assessing the binding activity between the test antibody and an Fc receptor (e.g., CD16a).

As used herein, the term "complement-dependent cytotoxicity (CDC)" refers to a form of cytotoxicity involving activation of the complement cascade via binding of the complement component C1q to the antibody Fc. Methods for detecting the CDC activity of antibodies are known in the art, for example, by assessing the binding activity between the test antibody and an Fc receptor (e.g., C1q).

In the IgG, IgA, and IgD antibody isotypes, the Fc region comprises the CH2 and CH3 constant domains from each of the two heavy chains of the antibody; the IgM and IgE Fc regions comprise three heavy chain constant domains (CH2-4 domains) in each polypeptide chain.

The term "humanized antibody" refers to a genetically engineered non-human antibody whose amino acid sequence has been modified to increase homology with the sequences of human antibodies. Most or all of the amino acids outside the CDR domains of the non-human antibody, such as a murine antibody, are replaced with corresponding amino acids from a human immunoglobulin, while most or all of the amino acids within one or more CDR regions remain unchanged. Additions, deletions, insertions, substitutions, or modifications of amino acids are permissible as long as they do not eliminate the antibody's ability to bind a specific antigen. A "humanized" antibody retains antigen specificity similar to that of the original antibody. The source of the CDRs is not particularly limited and may be derived from any animal. For example, CDR regions from murine, rat, rabbit, or non-human primate (e.g., cynomolgus monkey) antibodies may be utilized. Framework regions can be obtained by searching the IMGT antibody germline database (http://www.imgt.org/3Dstructure-DB/cgi/DomainGapAlign.cgi) for human antibody germline sequences. Generally, human germline antibody sequences with high homology to the non-human antibody being engineered are selected as the framework regions for the humanized antibody.

The term "hypervariable region" or "CDR region" or "complementarity determining region" refers to the amino acid residues of an antibody responsible for antigen binding, which are non-contiguous amino acid sequences. The CDR region sequences can be defined by the Kabat, Chothia, or IMGT methods (Lefranc et al., 2003, Dev Comparat Immunol, 27:55-77) or by any method for determining CDR region sequences known in the art to identify the amino acid residues within the variable region. For example, the hypervariable region comprises amino acid residues from the "complementarity determining regions" or "CDRs" as defined by sequence alignment (Kabat numbering system), such as residues at positions 24-34 (LCDR1), 50-56 (LCDR2), and 89-97 (LCDR3) of the light chain variable domain and residues at positions 31-35 (HCDR1), 50-65 (HCDR2), and 95-102 (HCDR3) of the heavy chain variable domain, as described in Kabat et al., 1991, Sequences of Proteins of Immunological Interest, 5th Edition, Public Health Service, National Institutes of Health, Bethesda, Md.; and/or residues from "hypervariable loops" (HVLs) defined based on structure (Chothia numbering system), such as residues at positions 26-32 (LCDR1), 50-52 (LCDR2), and 91-96 (LCDR3) of the light chain variable domain and residues at positions 26-32 (HCDR1), 53-55 (HCDR2), and 96-101 (HCDR3) of the heavy chain variable domain, as described in Chothia C and Lesk AM, 1987, J Mol Biol, 196:901-917; Chothia C et al., 1989, Nature, 342:878-883. "Framework" residues or "FR" residues are the variable domain residues other than the hypervariable region residues defined herein. In certain embodiments, the CDRs contained in the antibody or antigen-binding fragment of the present invention are preferably determined using the Kabat or IMGT numbering system. A person skilled in the art can readily assign each numbering system to any variable domain sequence without relying on any experimental data beyond the sequence itself. For example, the Kabat residue numbering for a given antibody can be determined by aligning the antibody sequence with the homologous regions of each "standard" numbering sequence. Determining the numbering for any variable region sequence in the sequence listing based on the numbering scheme provided herein is entirely within the routine skill of those in the art.

The term "isolated," as used herein with reference to nucleic acids (such as DNA or RNA), refers to molecules separated from other DNA or RNA molecules that exist as macromolecules in a natural source. The term "isolated," as used herein, also refers to nucleic acids or polypeptides that are substantially free of cellular material, viral material, or culture medium when produced by recombinant DNA technology, or substantially free of chemical precursors or other chemicals when prepared by chemical synthesis. Furthermore, an "isolated nucleic acid" refers to a nucleic acid fragment that is not naturally occurring and is not found in its natural state. The term "isolated" as used herein also refers to cells or polypeptides separated from other cellular proteins or tissues. An isolated polypeptide includes purified and recombinant polypeptides.

The term "cross-reactivity" refers to the ability of the antibody described herein to bind antigens from different species. Cross-reactivity can be measured by detecting specific reactivity with purified antigens in binding assays (e.g., SPR, ELISA), binding to cells physiologically expressing the antigen, or functional interactions with cells physiologically expressing the antigen in other ways. Examples of assays known in the art for measuring binding affinity include surface plasmon resonance (e.g., Biacore) or similar techniques (e.g., Kinexa or Octet).

The terms "immunobinding" and "immunobinding properties" refer to non-covalent interactions that occur between an immunoglobulin molecule and an antigen for which the immunoglobulin is specific. The strength or affinity of the immunobinding interaction can be expressed as the equilibrium dissociation constant (KD) of the interaction, where a lower KD value indicates higher affinity. The immunobinding properties of a selected polypeptide can be determined using methods well known in the art. One such method involves measuring the rates of formation and dissociation of antigen/antibody complexes. Both the "association rate constant" (ka or kon) and the "dissociation rate constant" (kd or koff) can be calculated from concentrations and the actual rates of association and dissociation (see Malmqvist M, 1993, Nature, 361:186-187). The ratio ka/kd equals the equilibrium dissociation constant KD (see Davies DR et al., 1990, Annual Rev Biochem, 59:439-473). The values of KD, ka, and kd can be measured using any effective method.

The term "host cell" refers to a cell in which a vector can be propagated and its DNA expressed, wherein the cell may be a prokaryotic or eukaryotic cell. This term also includes any progeny of the host cell in question. It is understood that not all progeny are identical to the parental cell, as mutations may occur during replication; such progeny are included. Host cells include prokaryotic cells, yeast, or mammalian cells, such as CHO cells, NS0 cells, or other mammalian cells.

The term "identity" is used to refer to the match between sequences of two polypeptides or two nucleic acids. When a position in both compared sequences is occupied by the same base or amino acid monomer subunit (e.g., if a position in each of two DNA molecules is occupied by adenine, or if a position in each of two polypeptides is occupied by lysine), then the molecules are identical at that position. The "percent identity" between two sequences is a function of the number of matching positions shared by the two sequences divided by the number of positions compared × 100. For example, if two sequences have matches at six out of ten positions, the two sequences have 60% identity. For instance, the DNA sequences CTGACT and CAGGTT share 50% identity (three out of six total positions match). Typically, the comparison is made when the two sequences are aligned to produce maximum identity. Such alignment can be conveniently performed using computer programs, such as the Align program (DNAstar, Inc.), implemented using the method of Needleman and Wunsch (Needleman SB and Wunsch CD, 1970, J Mol Biol, 48:443-453).

The term "mutated," "mutant," and "mutation" refer to the substitution, deletion, or insertion of one or more nucleotides or amino acids, respectively, compared to the native nucleic acid or polypeptide (i.e., a reference sequence that can be used to define the wild-type).

The term "conservative modification" is intended to mean amino acid modifications that do not significantly affect or alter the binding characteristics of an antibody containing the amino acid sequence. Such conservative modifications include amino acid substitutions, additions, and deletions. Modifications can be introduced into the antibodies of the present invention using standard techniques known in the art, such as site-directed mutagenesis and PCR-mediated mutagenesis. Conservative amino acid substitution refers to the replacement of an amino acid residue with another amino acid residue having a similar side chain. Families of amino acid residues with similar side chains are well-defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), β-branched side chains (e.g., threonine, valine, isoleucine), and aromatic side chains (e.g., tyrosine, phenylalanine, tryptophan, histidine). Therefore, one or more amino acid residues within the CDR regions of the antibody of the present invention may be replaced with other amino acid residues from the same side chain family.

The antibody of the present invention, or the nucleic acid or polynucleotide encoding the antibody of the present application, can be used in the preparation of pharmaceutical compositions or sterile formulations. For example, the antibody may be mixed with a pharmaceutically acceptable carrier, excipient, or stabilizer. The pharmaceutical composition may include one or a combination (e.g., two or more different) of the antibodies of the present invention. For instance, a pharmaceutical composition of the present invention may comprise a combination of antibodies or antibody fragments (or immunoconjugates) with complementary activities binding to different epitopes on the target antigen. Formulations of therapeutic and diagnostic agents can be prepared by mixing with pharmaceutically acceptable carriers, excipients, or stabilizers in forms such as lyophilized powders, slurries, aqueous solutions, or suspensions. The term "pharmaceutically acceptable" means that when the molecule itself, fragment, or composition is appropriately administered to an animal or human, it does not produce adverse, allergic, or other undesirable reactions. Specific examples of substances that can serve as pharmaceutically acceptable carriers or components thereof include carbohydrates (e.g., lactose), starches, cellulose and its derivatives, vegetable oils, gelatin, polyols (e.g., propylene glycol), alginates, and the like. The antibody of the present invention, or the nucleic acid or polynucleotide encoding the antibody of the present application, may be used alone or in combination with one or more other therapeutic agents, such as vaccines.

The term "pharmaceutically acceptable carrier and/or excipient and/or stabilizer" refers to a carrier, excipient, and/or stabilizer that is pharmacologically and/or physiologically compatible with the subject and the active ingredient, and that is non-toxic to cells or mammals exposed to it at the dosages and concentrations employed. This includes, but is not limited to: pH adjusting agents, surfactants, adjuvants, ionic strength enhancers, diluents, tonicity agents, absorption-delaying agents, and preservatives. For example, pH adjusting agents include, but are not limited to, phosphate buffers. Surfactants include, but are not limited to, cationic, anionic, or non-ionic surfactants, such as Tween-80. Ionic strength enhancers include, but are not limited to, sodium chloride. Preservatives include, but are not limited to, various antibacterial and antifungal agents, such as parabens, chlorobutanol, phenol, sorbic acid, etc. Tonicity agents include, but are not limited to, sugars, NaCl, and analogues thereof. Absorption-delaying agents include, but are not limited to, monostearate and gelatin. Diluents include, but are not limited to, water, aqueous buffers (e.g., buffered saline), alcohols, and polyols (e.g., glycerol), etc. Preservatives include, but are not limited to, various antibacterial and antifungal agents, such as thimerosal, 2-phenoxyethanol, parabens, chlorobutanol, phenol, sorbic acid, etc. Stabilizers have the meaning commonly understood by those skilled in the art and are capable of maintaining the desired activity of the active ingredient in the drug. They include, but are not limited to, sodium glutamate, gelatin, SPGA, sugars (such as sorbitol, mannitol, starch, sucrose, lactose, dextran, or glucose), amino acids (e.g., glutamic acid, glycine), proteins (e.g., dried whey, albumin, or casein) or their degradation products (e.g., lactalbumin hydrolysate), etc.

As used herein, the term "effective amount" refers to an amount sufficient to achieve or at least partially achieve a desired effect. For example, a prophylactically effective amount is an amount sufficient to prevent, hinder, or delay the onset of a disease; a therapeutically effective amount is an amount sufficient to cure or at least partially arrest a disease and its complications in a patient already suffering from the disease. Determining such effective amounts is entirely within the capability of those skilled in the art. For instance, an amount effective for therapeutic use will depend on the severity of the disease to be treated, the general state of the patient's own immune system, the patient's general condition such as age, weight, and gender, the mode of administration of the drug, and concurrent administration of other therapies, etc.

The embodiments of the present invention are further illustrated by the following examples. However, those skilled in the art should understand that the following drawings and examples are only intended to illustrate the present invention and not to further limit the same.

### Brief Description of the Drawings

Figure 1. ELISA binding of antibody AB8D, Novo Nordisk antibody Concizumab, and Pfizer antibody Marstacimab to human TFPI.
Figure 2-1. Competitive binding of antibody AB8D, Novo Nordisk antibody Concizumab, and Pfizer antibody Marstacimab with AB8D-HRP for human TFPI.
Figure 2-2. Competitive binding of antibody AB8D, Novo Nordisk antibody Concizumab, and Pfizer antibody Marstacimab with Concizumab-HRP for human TFPI.
Figure 3. Schematic diagram of antibodies AB8D, Novo Nordisk antibody Concizumab, and Pfizer antibody Marstacimab binding to the TFPI K2 domain. The structures of the AB8D/TFPI K2 and Marstacimab/TFPI K2 complexes were predicted by AlphaFold modeling. The Concizumab/TFPI K2 structure was obtained from the Protein Data Bank (PDB 4DTG).
Figure 4. Schematic diagram of the superimposed structures of antibodies AB8D, Novo Nordisk antibody Concizumab, and Pfizer antibody Marstacimab binding to the TFPI K2 domain. The three structures were superimposed using the common TFPI K2 domain.
Figure 5. Schematic diagram of the superimposed structures of antibody AB8D and coagulation factor FXa binding to the TFPI K2 domain. The structures of the AB8D/TFPI K2 and FXa/TFPI K2 complexes were superimposed using the common TFPI K2 domain. TFPI K2 domain residues R107, Y109, and R124 are displayed in a space-filling model.
Figure 6. Schematic diagram of the superimposed structures of antibody Concizumab and coagulation factor FXa binding to the TFPI K2 domain. The structures of the Concizumab/TFPI K2 and FXa/TFPI K2 complexes were superimposed using the common TFPI K2 domain. TFPI K2 domain residues R107 and Y109 are displayed in a space-filling model.
Figure 7. Schematic diagram of the superimposed structures of antibody Marstacimab and coagulation factor FXa binding to the TFPI K2 domain. The structures of the Marstacimab/TFPI K2 and FXa/TFPI K2 complexes were superimposed using the common TFPI K2 domain. TFPI K2 domain residues R107, Y109, and L131 are displayed in a space-filling model.
Figure 8. Schematic diagram of antibody AB8D binding to the TFPI K2 domain. Antibody heavy chain residues E50 and H35, as well as TFPI K2 domain residue R124, are displayed in a space-filling model.

### Detailed Description

The present invention is described with reference to the following examples, which are intended to illustrate, but not limit, the invention.

Unless otherwise specified, the molecular biology experimental methods and immunoassays used in the present invention are performed essentially as described in J. Sambrook et al., Molecular Cloning: A Laboratory Manual, 2nd Edition, Cold Spring Harbor Laboratory Press, 1989, and F. M. Ausubel et al., Current Protocols in Molecular Biology, 3rd Edition, John Wiley & Sons, Inc., 1995. The use of restriction enzymes follows the conditions recommended by the product manufacturers. Those skilled in the art understand that the examples describe the invention by way of illustration and are not intended to limit the scope claimed by the invention.

### Example 1: Preparation of Anti-Human TFPI Murine Monoclonal Antibodies

Human TFPI antigen (protein sequence: NCBI Sequence No. NP_006278.1) at 50 µg per mouse was emulsified with complete Freund's adjuvant and used to immunize male Balb/C mice via multiple-point injection, with immunization cycles every three weeks. Ten days after the third immunization, blood was collected from the tail vein, and the anti-human TFPI antibody titer in plasma was tested by ELISA to monitor the immune response in the mice. Three days before fusion, the mouse with the highest anti-human TFPI antibody titer was given a booster immunization. Three days later, the mouse was euthanized, and its spleen was removed and fused with the mouse myeloma Sp2/0 cell line. A mixture of 2×10^8 Sp2/0 cells and 2×10^8 spleen cells was fused in a solution containing 50% polyethylene glycol (molecular weight 1450) and 5% dimethyl sulfoxide (DMSO). The spleen cell number was adjusted to 5×10^5/mL using Iscove's medium (containing 10% fetal bovine serum, 100 U/mL penicillin, 100 µg/mL streptomycin, 0.1 mM hypoxanthine, 0.4 µM aminopterin, and 16 µg thymidine), and 0.3 mL was added to each well of 96-well culture plates, which were then placed in a 37°C, 5% CO₂ incubator. After 10 days of culture, high-throughput ELISA was used to detect clones producing antibodies with high-affinity binding to TFPI in the supernatant. The fusion cells from the wells containing the above monoclonal antibodies were then subcloned, and further screening yielded hybridoma cell line #3-71.

Clones producing specific antibodies were cultured in RPMI 1640 medium supplemented with 10% FCS. When cell density reached approximately 5×10^5 cells/mL, the medium was replaced with serum-free medium. After 2 to 4 days, the conditioned medium was centrifuged to collect the culture supernatant. A Protein G column was used to purify the antibody. The monoclonal antibody eluate was dialyzed against 150 mM NaCl. The dialyzed solution was filter-sterilized through a 0.22 µm filter to obtain purified anti-human TFPI murine monoclonal antibody mAb3-71 for testing.

### Example 2: Affinity Determination and Kinetic Study of Murine TFPI Antibodies

Bio-Layer Interferometry (BLI) was used to determine the binding affinity constants of the purified anti-human TFPI murine monoclonal antibody mAb3-71 to human TFPI, using the ForteBio Octet RED&QK system from PALL Corporation. For parallel quantitative analysis across multiple channels, a concentration gradient was set at: 3.125, 6.25, 12.5, 25, 50, and 100 nM. His-tagged human TFPI at 10 µg/mL was immobilized on Ni-NTA sensors. The affinity determination results are shown in Table 1. The results show that the murine monoclonal antibody has extremely high binding affinity for human TFPI, with K_{D} values reaching the 10⁻¹² M range.

**Table 1. Affinity Determination Results for the Murine Monoclonal Antibody**

| **Antibody** | **K_{D} (M)** | **kon (1/Ms)** | **koff (1/s)** |
|---|---|---|---|
| mAb3-71 | 4.539E-12 | 2.893E+05 | 1.313E-06 |

### Example 3: Humanization of Anti-Human TFPI Murine Antibodies

The murine antibodies were humanized using the CDR grafting method. The basic principle of CDR grafting is to transplant the CDR regions of the murine antibody onto a human antibody template while also introducing several or some key murine FR residues that are important for stabilizing the CDR conformation and for antigen-antibody binding onto the human antibody template (backmutations). This aims to reduce the immunogenicity of the murine antibody while maintaining its affinity. In addition to the CDR grafting operation, we further calculated four aspects for the humanized antibodies post-grafting: isoelectric point (pI), hydrophobic aggregation, post-translational modifications (PTM, such as glycosylation, cleavage, isomerization sites, etc.), and immunogenicity. Amino acids causing issues in these four aspects were mutated to enable the humanized antibodies to exert their full therapeutic efficacy in clinical use.

The specific workflow for antibody humanization is as follows. A human antibody germline database was searched to obtain human antibody templates with high similarity to the murine antibody. The CDR regions of the murine antibody and the human antibody template were annotated, defining the CDR regions according to the Kabat or IMGT scheme. The six CDR regions of the murine antibody were used to replace the six CDR regions of the human antibody template, respectively. Each individual CDR region transplanted could be the amino acid region defined by Kabat or by IMGT. Following CDR grafting, backmutations from the murine antibody to the FR regions of the humanized template were performed. Key murine FR amino acids important for stabilizing the antibody CDR conformation and for antigen-antibody binding include four categories of amino acid residues: 1) Amino acids buried beneath the antibody surface within 6 Å of the CDR regions; 2) Amino acids exposed on the antibody surface within 6 Å of the CDR regions; 3) Interface amino acids between the light and heavy chain domains of the antibody; and 4) Vernier zone residues stabilizing the antibody CDR conformation (Foote J and Winter G, 1992, J Mol Biol, 224:487-499). These four categories of key murine FR residues were identified by constructing a three-dimensional structural model of the murine antibody. For amino acids in these four categories where the human template sequence differed from the murine antibody, structural analysis was used to select residues important for maintaining the CDR conformation and antigen-antibody binding for transplantation or substitution from the murine antibody to the human template. Subsequently, the humanized antibody resulting from the transplantation of these four categories of amino acids was further analyzed by calculating its pI, hydrophobic aggregation propensity, PTM sites, and immunogenicity. Problematic amino acids were mutated to obtain the final humanized antibody sequence.

Based on the above method, multiple humanized antibodies were constructed based on the CDRs of the murine antibody mAb3-71. The amino acid sequences of the CDR regions contained in the variable regions of the relevant humanized antibodies are shown in Table 2, and the amino acid sequences of the heavy and light chain variable regions are shown in Table 3.

**Table 2. CDR Region Sequences of Exemplary Anti-TFPI Humanized Antibodies**

| **Antibody Number** | **CDR** | **Kabat** | **IMGT** |
|---|---|---|---|
| AB8D | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDVAMDY (SEQ ID NO: 48) | ARSSVVDVAMDY (SEQ ID NO: 54) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 1-1 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSHVDVAMDY (SEQ ID NO: 56) | ARSSHVDVAMDY (SEQ ID NO: 57) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 1-2 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVIDVAMDY (SEQ ID NO: 58) | ARSSVIDVAMDY (SEQ ID NO: 59) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 1-3 | HCDR1 | SYWMH (SEQ ID NO: 46) | GYTFTSYW (SEQ ID NO: 52) |
| | HCDR2 | EIKPDQGRIHYNEKFKT (SEQ ID NO: 60) | IKPDQGRI (SEQ ID NO: 53) |
| | HCDR3 | SSVVDVAMDY (SEQ ID NO: 48) | ARSSVVDVAMDY (SEQ ID NO: 54) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 1-4 | HCDR1 | SYWMH (SEQ ID NO: 46) | GYTFTSYW (SEQ ID NO: 52) |
| | HCDR2 | EIKPDHGRINYNEKFKT (SEQ ID NO: 61) | IKPDHGRI (SEQ ID NO: 62) |
| | HCDR3 | SSVVDVAMDY (SEQ ID NO: 48) | ARSSVVDVAMDY (SEQ ID NO: 54) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 1-5 | HCDR1 | SYWMH (SEQ ID NO: 46) | GYTFTSYW (SEQ ID NO: 52) |
| | HCDR2 | EIKPDKGRINYNEKFKT (SEQ ID NO: 63) | IKPDKGRI (SEQ ID NO: 64) |
| | HCDR3 | SSVVDVAMDY (SEQ ID NO: 48) | ARSSVVDVAMDY (SEQ ID NO: 54) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 1-6 | HCDR1 | SYWMH (SEQ ID NO: 46) | GYTFTSYW (SEQ ID NO: 52) |
| | HCDR2 | EIKPDQGRIDYNEKFKT (SEQ ID NO: 65) | IKPDQGRI (SEQ ID NO: 53) |
| | HCDR3 | SSVVDVAMDY (SEQ ID NO: 48) | ARSSVVDVAMDY (SEQ ID NO: 54) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 1-7 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDVAMDY (SEQ ID NO: 48) | ARSSVVDVAMDY (SEQ ID NO: 54) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSYYPFT (SEQ ID NO: 66) | HQWSYYPFT (SEQ ID NO: 66) |
| Antibody 1-8 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDVAMDY (SEQ ID NO: 48) | ARSSVVDVAMDY (SEQ ID NO: 54) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSFYPFT (SEQ ID NO: 67) | HQWSFYPFT (SEQ ID NO: 67) |
| Antibody 1-9 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDVAMDY (SEQ ID NO: 48) | ARSSVVDVAMDY (SEQ ID NO: 54) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWTHYPFT (SEQ ID NO: 68) | HQWTHYPFT (SEQ ID NO: 68) |
| Antibody 1-10 | HCDR1 | SYWMH (SEQ ID NO: 46) | GYTFTSYW (SEQ ID NO: 52) |
| | HCDR2 | EIKPDKGRINYNEKFKT (SEQ ID NO: 63) | IKPDKGRI (SEQ ID NO: 64) |
| | HCDR3 | SSVIDVAMDY (SEQ ID NO: 58) | ARSSVIDVAMDY (SEQ ID NO: 59) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 2-1 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDDAMDY (SEQ ID NO: 69) | ARSSVVDDAMDY (SEQ ID NO: 70) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 2-2 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDTAMDY (SEQ ID NO: 71) | ARSSVVDTAMDY (SEQ ID NO: 72) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 2-3 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDEAMDY (SEQ ID NO: 73) | ARSSVVDEAMDY (SEQ ID NO: 74) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 2-4 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDVALDY (SEQ ID NO: 75) | ARSSVVDVALDY (SEQ ID NO: 76) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 2-5 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDVAIDY (SEQ ID NO: 77) | ARSSVVDVAIDY (SEQ ID NO: 78) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 2-6 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDVAEDY (SEQ ID NO: 79) | ARSSVVDVAEDY (SEQ ID NO: 80) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 2-7 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDVAVDY (SEQ ID NO: 81) | ARSSVVDVAVDY (SEQ ID NO: 82) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 2-8 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVVDVADDY (SEQ ID NO: 83) | ARSSVVDVADDY (SEQ ID NO: 84) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 2-9 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVFDVAMDY (SEQ ID NO: 85) | ARSSVFDVAMDY (SEQ ID NO: 86) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 2-10 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVYDVAMDY (SEQ ID NO: 87) | ARSSVYDVAMDY (SEQ ID NO: 88) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |
| Antibody 2-11 | HCDR1 | SYWMH (SEQ ID NO: 46) | GYTFTSYW (SEQ ID NO: 52) |
| | HCDR2 | EIKPDQGRINYNEKFKT (SEQ ID NO: 47) | IKPDQGRI (SEQ ID NO: 53) |
| | HCDR3 | SSVLDVAMDY (SEQ ID NO: 89) | ARSSVLDVAMDY (SEQ ID NO: 90) |
| | LCDR1 | SASSSVSSSYLY (SEQ ID NO: 49) | SSVSSSY (SEQ ID NO: 55) |
| | LCDR2 | GTSILAS (SEQ ID NO: 50) | GTS |
| | LCDR3 | HQWSHYPFT (SEQ ID NO: 51) | HQWSHYPFT (SEQ ID NO: 51) |

**Table 3. Amino Acid Sequences of Humanized Antibody Variable Regions**

| | VH Amino acid sequence | VL Amino acid sequence |
|---|---|---|
| AB8D | EVQLVQSGAELKKPGASVKLSCKASG YTFTSYWMHWVRQTPGQGLEWIGEI KPDQGRINYNEKFKTRATLTADKSTST AYMELSSLRSEDTAVYYCARSSVVDV AMDYWGQGTTVTVSS SEQ ID NO: 2 | EIVLTQSPATLSLSPGERATLSCSASSS VSSSYLYWYQQKPGQAPRLWISGTSI LASGVPARFSGSGSGTKYTLTISSLEPE DFAVYFCHQWSHYPFTFGQGTKLEIK SEQ ID NO: 3 |
| Antibody 1-1 | EVQLVQSGAELKKPGASVKLSCKASG YTFTSYWMHWVRQAPGQGLEWIGEI KPDQGRINYNEKFKTRATLTADKSTST AYMELSSLRSEDTAVYYCARSSHVDV AMDYWGQGTTVTVSS SEQ ID NO: 4 | EIVLTQSPATLSLSPGERATLSCSASSS VSSSYLYWYQQKPGQAPRLWISGTSI LASGVPARFSGSGSGTKYTLTISSLEPE DFAVYFCHQWSHYPFTFGQGTKLEIK SEQ ID NO: 5 |
| Antibody 1-2 | EVQLVQSGAELKKPGASVKLSCKASG YTFTSYWMHWVRQAPGQGLEWIGEI KPDQGRINYNEKFKTRATLTADKSTST AYMELSSLRSEDTAVYYCARSSVIDVA MDYWGQGTTVTVSS SEQ ID NO: 6 | EIVLTQSPATLSLSPGERATLSCSASSS VSSSYLYWYQQKPGQAPRLWISGTSI LASGVPARFSGSGSGTKYTLTISSLEPE DFAVYFCHQWSHYPFTFGQGTKLEIK SEQ ID NO: 7 |
| Antibody 1-3 | EVQLVQSGAELKKPGASVKLSCKASG YTFTSYWMHWVRQAPGQGLEWIGEI KPDQGRIHYNEKFKTRATLTADKSTST AYMELSSLRSEDTAVYYCARSSVVDV AMDYWGQGTTVTVSS SEQ ID NO: 8 | EIVLTQSPATLSLSPGERATLSCSASSS VSSSYLYWYQQKPGQAPRLWISGTSI LASGVPARFSGSGSGTKYTLTISSLEPE DFAVYFCHQWSHYPFTFGQGTKLEIK SEQ ID NO: 9 |
| Antibody 1-4 | EVQLVQSGAELKKPGASVKLSCKASG YTFTSYWMHWVRQAPGQGLEWIGEI KPDHGRINYNEKFKTRATLTADKSTST AYMELSSLRSEDTAVYYCARSSVVDV AMDYWGQGTTVTVSS SEQ ID NO: 10 | EIVLTQSPATLSLSPGERATLSCSASSS VSSSYLYWYQQKPGQAPRLWISGTSI LASGVPARFSGSGSGTKYTLTISSLEPE DFAVYFCHQWSHYPFTFGQGTKLEIK SEQ ID NO: 11 |
| Antibody 1-5 | EVQLVQSGAELKKPGASVKLSCKASG YTFTSYWMHWVRQAPGQGLEWIGEI KPDKGRINYNEKFKTRATLTADKSTST AYMELSSLRSEDTAVYYCARSSVVDV AMDYWGQGTTVTVSS SEQ ID NO: 12 | EIVLTQSPATLSLSPGERATLSCSASSS VSSSYLYWYQQKPGQAPRLWISGTSI LASGVPARFSGSGSGTKYTLTISSLEPE DFAVYFCHQWSHYPFTFGQGTKLEIK SEQ ID NO: 13 |
| Antibody 1-6 | EVQLVQSGAELKKPGASVKLSCKASG YTFTSYWMHWVRQAPGQGLEWIGEI KPDQGRIDYNEKFKTRATLTADKSTST AYMELSSLRSEDTAVYYCARSSVVDV AMDYWGQGTTVTVSS SEQ ID NO: 14 | EIVLTQSPATLSLSPGERATLSCSASSS VSSSYLYWYQQKPGQAPRLWISGTSI LASGVPARFSGSGSGTKYTLTISSLEPE DFAVYFCHQWSHYPFTFGQGTKLEIK SEQ ID NO: 15 |
| Antibody 1-7 | EVQLVQSGAELKKPGASVKLSCKASG YTFTSYWMHWVRQAPGQGLEWIGEI KPDQGRINYNEKFKTRATLTADKSTST AYMELSSLRSEDTAVYYCARSSVVDV AMDYWGQGTTVTVSS SEQ ID NO: 16 | EIVLTQSPATLSLSPGERATLSCSASSS VSSSYLYWYQQKPGQAPRLWISGTSI LASGVPARFSGSGSGTKYTLTISSLEPE DFAVYFCHQWSYYPFTFGQGTKLEIK SEQ ID NO: 17 |
| Antibody 1-8 | EVQLVQSGAELKKPGASVKLSCKASG YTFTSYWMHWVRQAPGQGLEWIGEI KPDQGRINYNEKFKTRATLTADKSTST AYMELSSLRSEDTAVYYCARSSVVDV AMDYWGQGTTVTVSS SEQ ID NO: 18 | EIVLTQSPATLSLSPGERATLSCSASSS VSSSYLYWYQQKPGQAPRLWISGTSI LASGVPARFSGSGSGTKYTLTISSLEPE DFAVYFCHQWSFYPFTFGQGTKLEIK SEQ ID NO: 19 |
| Antibody 1-9 | EVQLVQSGAELKKPGASVKLSCKASG YTFTSYWMHWVRQAPGQGLEWIGEI KPDQGRINYNEKFKTRATLTADKSTST AYMELSSLRSEDTAVYYCARSSVVDV AMDYWGQGTTVTVSS SEQ ID NO: 20 | EIVLTQSPATLSLSPGERATLSCSASSS VSSSYLYWYQQKPGQAPRLWISGTSI LASGVPARFSGSGSGTKYTLTISSLEPE DFAVYFCHQWTHYPFTFGQGTKLEIK SEQ ID NO: 21 |
| Antibody 1-10 | EVQLVQSGAELKKPGASVKLSCKASG YTFTSYWMHWVRQAPGQGLEWIGEI KPDKGRINYNEKFKTRATLTADKSTST AYMELSSLRSEDTAVYYCARSSVIDVA MDYWGQGTTVTVSS SEQ ID NO: 22 | EIVLTQSPATLSLSPGERATLSCSASSS VSSSYLYWYQQKPGQAPRLWISGTSI LASGVPARFSGSGSGTKYTLTISSLEPE DFAVYFCHQWSHYPFTFGQGTKLEIK SEQ ID NO: 23 |
| Antibody 2-1 | EVQLVQSGAELKKPGASVKLSCKASG YTFTSYWMHWVRQAPGQGLEWIGEI KPDQGRINYNEKFKTRATLTADKSTST AYMELSSLRSEDTAVYYCARSSVVDD AMDYWGQGTTVTVSS SEQ ID NO: 24 | EIVLTQSPATLSLSPGERATLSCSASSS VSSSYLYWYQQKPGQAPRLWISGTSI LASGVPARFSGSGSGTKYTLTISSLEPE DFAVYFCHQWSHYPFTFGQGTKLEIK SEQ ID NO: 25 |
| Antibody 2-2 | EVQLVQSGAELKKPGASVKLSCKASG YTFTSYWMHWVRQAPGQGLEWIGEI KPDQGRINYNEKFKTRATLTADKSTST AYMELSSLRSEDTAVYYCARSSVVDT AMDYWGQGTTVTVSS SEQ ID NO: 26 | EIVLTQSPATLSLSPGERATLSCSASSS VSSSYLYWYQQKPGQAPRLWISGTSI LASGVPARFSGSGSGTKYTLTISSLEPE DFAVYFCHQWSHYPFTFGQGTKLEIK SEQ ID NO: 27 |
| Antibody 2-3 | EVQLVQSGAELKKPGASVKLSCKASG YTFTSYWMHWVRQAPGQGLEWIGEI KPDQGRINYNEKFKTRATLTADKSTST AYMELSSLRSEDTAVYYCARSSVVDE AMDYWGQGTTVTVSS SEQ ID NO: 28 | EIVLTQSPATLSLSPGERATLSCSASSS VSSSYLYWYQQKPGQAPRLWISGTSI LASGVPARFSGSGSGTKYTLTISSLEPE DFAVYFCHQWSHYPFTFGQGTKLEIK SEQ ID NO: 29 |
| Antibody 2-4 | EVQLVQSGAELKKPGASVKLSCKASG YTFTSYWMHWVRQAPGQGLEWIGEI | EIVLTQSPATLSLSPGERATLSCSASSS VSSSYLYWYQQKPGQAPRLWISGTSI |
| | KPDQGRINYNEKFKTRATLTADKSTST AYMELSSLRSEDTAVYYCARSSVVDV ALDYWGQGTTVTVSS SEQ ID NO: 30 | LASGVPARFSGSGSGTKYTLTISSLEPE DFΛVYFCHQWSHYPFTFGQGTKLEIK SEQ ID NO: 31 |
| Antibody 2-5 | EVQLVQSGAELKKPGASVKLSCKASG YTFTSYWMHWVRQAPGQGLEWIGEI KPDQGRINYNEKFKTRATLTADKSTST AYMELSSLRSEDTAVYYCARSSVVDV AIDYWGQGTTVTVSS SEQ ID NO: 32 | EIVLTQSPATLSLSPGERATLSCSASSS VSSSYLYWYQQKPGQAPRLWISGTSI LASGVPARFSGSGSGTKYTLTISSLEPE DFAVYFCHQWSHYPFTFGQGTKLEIK SEQ ID NO: 33 |
| Antibody 2-6 | EVQLVQSGAELKKPGASVKLSCKASG YTFTSYWMHWVRQAPGQGLEWIGEI KPDQGRINYNEKFKTRATLTADKSTST AYMELSSLRSEDTAVYYCARSSVVDV AEDYWGQGTTVTVSS SEQ ID NO: 34 | EIVLTQSPATLSLSPGERATLSCSASSS VSSSYLYWYQQKPGQAPRLWISGTSI LASGVPARFSGSGSGTKYTLTISSLEPE DFAVYFCHQWSHYPFTFGQGTKLEIK SEQ ID NO: 35 |
| Antibody 2-7 | EVQLVQSGAELKKPGASVKLSCKASG YTFTSYWMHWVRQAPGQGLEWIGEI KPDQGRINYNEKFKTRATLTADKSTST AYMELSSLRSEDTAVYYCARSSVVDV AVDYWGQGTTVTVSS SEQ ID NO: 36 | EIVLTQSPATLSLSPGERATLSCSASSS VSSSYLYWYQQKPGQAPRLWISGTSI LASGVPARFSGSGSGTKYTLTISSLEPE DFAVYFCHQWSHYPFTFGQGTKLEIK SEQ ID NO: 37 |
| Antibody 2-8 | EVQLVQSGAELKKPGASVKLSCKASG YTFTSYWMHWVRQAPGQGLEWIGEI KPDQGRINYNEKFKTRATLTADKSTST AYMELSSLRSEDTAVYYCARSSVVDV ADDYWGQGTTVTVSS SEQ ID NO: 38 | EIVLTQSPATLSLSPGERATLSCSASSS VSSSYLYWYQQKPGQAPRLWISGTSI LASGVPARFSGSGSGTKYTLTISSLEPE DFAVYFCHQWSHYPFTFGQGTKLEIK SEQ ID NO: 39 |
| Antibody 2-9 | EVQLVQSGAELKKPGASVKLSCKASG YTFTSYWMHWVRQAPGQGLEWIGEI KPDQGRINYNEKFKTRATLTADKSTST AYMELSSLRSEDTAVYYCARSSVFDV AMDYWGQGTTVTVSS SEQ ID NO: 40 | EIVLTQSPATLSLSPGERATLSCSASSS VSSSYLYWYQQKPGQAPRLWISGTSI LASGVPARFSGSGSGTKYTLTISSLEPE DFAVYFCHQWSHYPFTFGQGTKLEIK SEQ ID NO: 41 |
| Antibody 2-10 | EVQLVQSGAELKKPGASVKLSCKASG YTFTSYWMHWVRQAPGQGLEWIGEI KPDQGRINYNEKFKTRATLTADKSTST AYMELSSLRSEDTAVYYCARSSVYDV AMDYWGQGTTVTVSS SEQ ID NO: 42 | EIVLTQSPATLSLSPGERATLSCSASSS VSSSYLYWYQQKPGQAPRLWISGTSI LASGVPARFSGSGSGTKYTLTISSLEPE DFAVYFCHQWSHYPFTFGQGTKLEIK SEQ ID NO: 43 |
| Antibody 2-11 | EVQLVQSGAELKKPGASVKLSCKASG YTFTSYWMHWVRQAPGQGLEWIGEI KPDQGRINYNEKFKTRATLTADKSTST AYMELSSLRSEDTAVYYCARSSVLDV AMDYWGQGTTVTVSS SEQ ID NO: 44 | EIVLTQSPATLSLSPGERATLSCSASSS VSSSYLYWYQQKPGQAPRLWISGTSI LASGVPARFSGSGSGTKYTLTISSLEPE DFAVYFCHQWSHYPFTFGQGTKLEIK SEQ ID NO: 45 |

To obtain the full-length antibody sequence composed of two heavy chains and two light chains, the VH and VL sequences shown in Table 3 can be spliced or assembled with antibody heavy chain constant region sequences (preferably selected from human IgG1, IgG2, or IgG4) and light chain constant region sequences (preferably selected from human kappa light chain) using conventional techniques. Preferably, the heavy chain constant region is a human wild-type heavy chain constant region or a mutant thereof. More preferably, the heavy chain constant region is the heavy chain constant region of human IgG4, and the heavy chain constant region contains the amino acid mutation S228P.

### Example 4: Construction, Expression, and Preparation of Anti-Human TFPI Antibody Expression Vectors

Based on the heavy and light chain sequences obtained in the above examples, encoding cDNA was designed and inserted into the pcDNA3.1 eukaryotic expression vector to construct humanized expression vectors. This expression vector plasmid contains the cytomegalovirus early gene promoter-enhancer required for high-level expression in mammalian cells. The vector plasmid also contains selectable marker genes conferring kanamycin resistance in bacteria and G418 resistance in mammalian cells. Additionally, the vector plasmid contains a dihydrofolate reductase (DHFR) gene, allowing for co-amplification of the antibody gene and the DHFR gene with methotrexate (MTX) in suitable host cells.

The constructed recombinant expression vector plasmids were transfected into mammalian host cell lines to express the humanized antibodies. For stable high-level expression, the preferred host cell line is a DHFR-deficient Chinese Hamster Ovary (CHO) cell line (see U.S. Patent No. 4,818,679). The preferred transfection method is electroporation, although other methods can also be used, including calcium phosphate co-precipitation, lipofection, and protoplast fusion. For electroporation, 2×10⁷ cells suspended in 0.8 mL PBS containing 20 µg of expression vector plasmid were added to a cuvette and subjected to an electric field of 300 V and 1050 µF capacitance using a GenePulser (Bio-Rad Laboratories). Two days after transfection, selection was initiated by adding medium containing 0.2 mg/mL G418 and 200 nM MTX (Sigma). To achieve higher expression levels, the transfected antibody gene was co-amplified with the DHFR gene using MTX selection. Transfectants were subcloned by limiting dilution, and the secretion rates of individual cell lines were determined by ELISA to select cell clones with high-level antibody expression. Conditioned medium containing the antibodies was collected for assessing in vitro and in vivo biological activities.

### Example 5: Purification and Characterization of Anti-Human TFPI Antibodies

This example describes the purification and characterization methods for the humanized antibody AB8D. The cell culture supernatant was clarified by high-speed low-temperature centrifugation or depth filtration, followed by sterile filtration through a 0.22 µm filter. Subsequently, a three-step chromatography purification process involving Protein A affinity, anion exchange, and cation exchange chromatography was performed as follows: The first capture step used Protein A affinity chromatography with PBS as the equilibration buffer. Elution was performed using an elution buffer (50 mM NaAc-HAc, pH 3.7) in a linear gradient. The pH of the Protein A eluate was adjusted to 3.6-3.8 by adding 1M acetic acid and incubated at room temperature for 60 minutes for virus inactivation. The pH was then neutralized to 5.5 by adding 2M Tris. For intermediate purification, the anion exchange resin Q Bestarose FF was selected to remove residual DNA, endotoxins, and impurity proteins, using 50 mM NaAc-HAc, pH 5.5 as the equilibration buffer. The flow-through protein from the anion exchange step was directly loaded onto the cation exchange resin Monomix HC45-SP. Cation exchange chromatography was equilibrated with 50 mM NaAc-HAc, pH 5.5, and elution was performed using an elution buffer (50 mM NaAc-HAc, 0.2 M NaCl, pH 5.5) in a linear gradient. The eluted protein solution from the cation exchange step was processed through nanofiltration, ultrafiltration/diafiltration for concentration and buffer exchange, and final sterile filtration to obtain the drug substance.

The drug substance was analyzed for purity by SEC-HPLC and SDS-PAGE. SEC-HPLC results showed that the main peak purity of the purified antibody was above 99%. The theoretical molecular weight of the AB8D antibody is approximately 145 kDa. Under reducing conditions, SDS-PAGE showed a light chain of approximately 25 kDa and a heavy chain of approximately 50 kDa, consistent with the theoretical values.

### Example 6: Indirect ELISA Binding Assay for Anti-Human TFPI Antibodies

To compare the specific binding activities of antibody AB8D, the Novo Nordisk antibody Concizumab, and the Pfizer antibody Marstacimab to human TFPI, all three antibodies were quantified using a unified concentration standard (i.e., concentration determined using the IgG setting of the Onedrop OD280 instrument). The EC₅₀ values of antibody AB8D, Concizumab, and Marstacimab for binding to human TFPI were also compared.

Human TFPI/His (purchased from ACRO Biosystem) was diluted to 0.1 µg/ml in 1× PBS buffer and added to 96-well microplates at 100 µl/well, then incubated at 4°C for 16-20 hours. The PBS buffer was removed from the plates, and the wells were blocked by adding 200 µl/well of PBST/1.5% BSA and incubating at room temperature for 1 hour. After washing the plates three times with PBST (pH 7.4, PBS containing 0.05% Tween-20), the test TFPI antibodies were added at 100 µl/well and incubated at room temperature for 1.0 hour. The plates were washed three times with PBST, then 100 µl/well of a 1:5000 dilution of HRP-conjugated goat anti-human IgG secondary antibody (purchased from The Jackson Laboratory) was added and incubated at room temperature for 1 hour. After washing three times with PBST, 100 µl/well of TMB substrate was added and incubated at room temperature for 5-10 minutes. The reaction was stopped by adding 50 µl/well of 0.2 M sulfuric acid. Absorbance was read at dual wavelengths of 450/620 nm using a microplate reader.

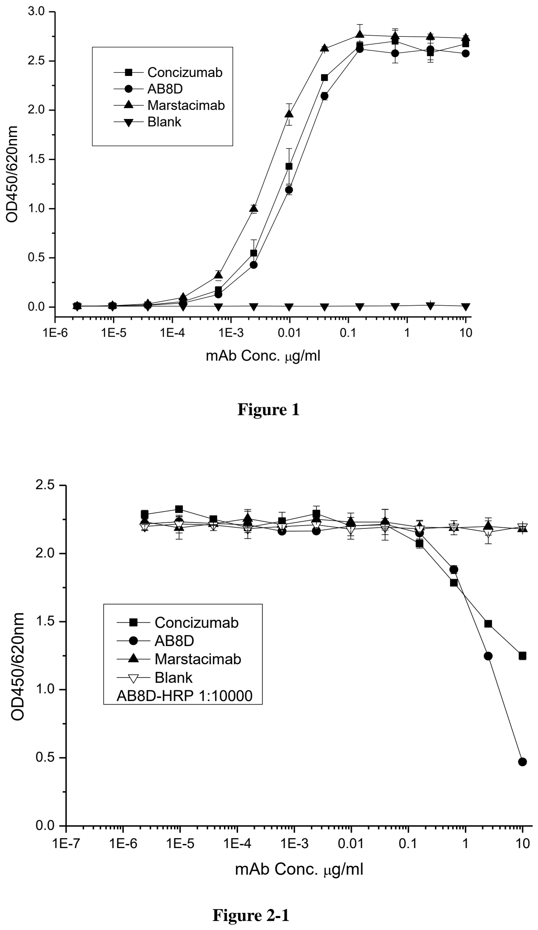

Figure 1 shows that antibody AB8D, the Novo Nordisk antibody Concizumab, and the Pfizer antibody Marstacimab all specifically bind to human TFPI. The binding strength was higher for the Pfizer antibody Marstacimab, while antibody AB8D and the Novo Nordisk antibody Concizumab showed similar binding. The EC₅₀ values for binding to human TFPI were 9.28 ng/ml for AB8D, 6.67 ng/ml for Concizumab, and 3.74 ng/ml for Marstacimab.

### Example 7: Competition Binding Assay for Anti-Human TFPI Antibodies

To compare the differences in the epitopes on human TFPI bound by antibody AB8D, the Novo Nordisk antibody Concizumab, and the Pfizer antibody Marstacimab, AB8D and Concizumab were labeled with HRP, respectively. Competitive ELISAs were performed using antibody AB8D, Concizumab, and Marstacimab against AB8D-HRP and Concizumab-HRP to determine the differences in their epitopes on human TFPI.

Human TFPI/His (purchased from ACROBiosystem) was diluted to 0.1 µg/ml in 1× PBS buffer and added to 96-well microplates at 100 µl/well, then incubated at 4°C for 16-20 hours. The PBS buffer was removed from the plates, and the wells were blocked by adding 200 µl/well of PBST/1.5% BSA and incubating at room temperature for 1 hour. After washing the plates three times with PBST (pH 7.4, PBS containing 0.05% Tween-20), the test TFPI antibodies (starting at 10 µg/ml and serially diluted in a U-bottom plate) were added along with 50 µl/well of a 1:10000 dilution of AB8D-HRP or Concizumab-HRP, and incubated at room temperature for 1.0 hour. The plates were washed three times with PBST, then 100 µl/well of TMB substrate was added and incubated at room temperature for 5-10 minutes. The reaction was stopped by adding 50 µl/well of 0.2 M sulfuric acid. Absorbance was read at dual wavelengths of 450/620 nm using a microplate reader.

Figure 2-1 shows that the Pfizer antibody Marstacimab does not compete with AB8D-HRP, indicating that they bind to different epitopes on human TFPI. The Novo Nordisk antibody Concizumab partially competes with AB8D-HRP, indicating that their epitopes are similar and partially overlap but are not identical.

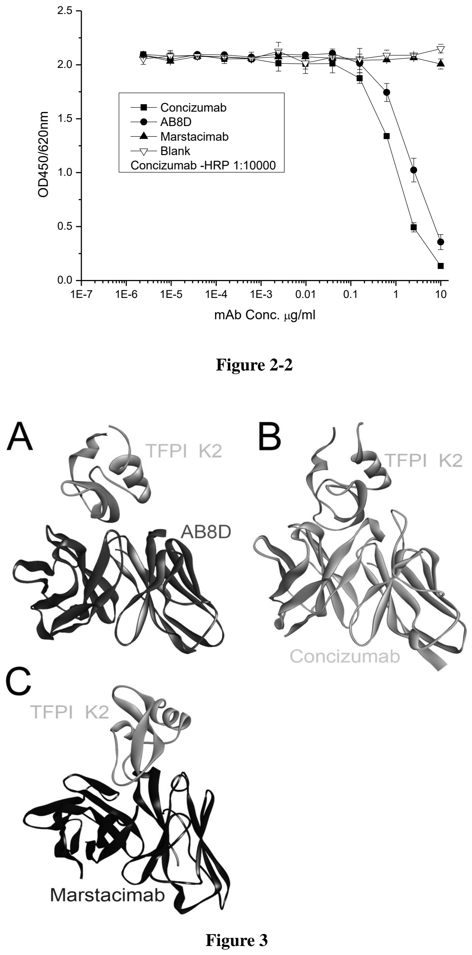

Figure 2-2 shows that the Pfizer antibody Marstacimab does not compete with Concizumab-HRP, indicating that they bind to different epitopes on human TFPI. Antibody AB8D partially competes with Concizumab-HRP, indicating that their epitopes are similar and partially overlap but are not identical.

### Example 8: pH-Dependent Binding Assay for Anti-Human TFPI Antibodies

### (1) Indirect ELISA Binding Assay

To compare the binding activities of antibody AB8D, the Novo Nordisk antibody Concizumab, and the Pfizer antibody Marstacimab to human TFPI under different pH conditions, all three antibodies were quantified using a unified concentration standard (i.e., concentration determined using the IgG setting of the Onedrop OD280 instrument). The EC₅₀ values of antibody AB8D, Concizumab, and Marstacimab for binding to human TFPI under different pH conditions were also compared.

Human TFPI/His (purchased from ACROBiosystem) was diluted to 0.1 µg/ml in 1× PBS buffer and added to 96-well microplates at 100 µl/well, then incubated at 4°C for 16-20 hours. The PBS buffer was removed from the plates, and the wells were blocked by adding 200 µl/well of PBST/1.5% BSA and incubating at room temperature for 1 hour. After washing the plates three times with PBST (pH 7.4, PBS containing 0.05% Tween-20), the test TFPI antibodies (diluted in PBST/1.5% BSA buffer with pH values of 4.0, 4.5, 5.0, 5.5, 6.0, 6.5, and 7.4) were added at 100 µl/well and incubated at room temperature for 1.0 hour. The plates were washed three times with PBST, then 100 µl/well of a 1:5000 dilution of HRP-conjugated goat anti-human IgG secondary antibody (purchased from The Jackson Laboratory) was added and incubated at room temperature for 1 hour. After washing three times with PBST, 100 µl/well of TMB substrate was added and incubated at room temperature for 5-10 minutes. The reaction was stopped by adding 50 µl/well of 0.2 M sulfuric acid. Absorbance was read at dual wavelengths of 450/620 nm using a microplate reader.

Table 4-1 shows the EC₅₀ values for binding of AB8D, the Novo Nordisk antibody Concizumab, and the Pfizer antibody Marstacimab to human TFPI under different pH conditions.

**Table 4-1. EC₅₀ Values for TFPI Antibodies Binding to Human TFPI under Different pH Conditions**

| | **Concizumab** | **AB8D** | **Marstacimab** |
|---|---|---|---|
| **pH** | **EC₅₀(ng/ml)** | **EC₅₀(ng/ml)** | **EC₅₀(ng/ml)** |
| 4.0 | 16.54 | 121.25 | 5.87 |
| 4.5 | 4.94 | 45.40 | 3.15 |
| 5.0 | 4.83 | 23.24 | 3.97 |
| 5.5 | 5.00 | 12.03 | 2.18 |
| 6.0 | 6.10 | 5.58 | 3.19 |
| 6.5 | 4.90 | 7.94 | 3.87 |
| 7.4 | 4.25 | 8.53 | 4.01 |

### (2) Affinity Determination Assay Using Bio-Layer Interferometry (BLI)

The affinity of the murine monoclonal antibody mAb3-71, AB8D, and the Novo Nordisk antibody Concizumab for human TFPI under pH 7.4 and pH 6.0 conditions was detected using a Sartorius Octet^{®} R8 biomolecular interaction instrument via Bio-Layer Interferometry (BLI). This analysis aimed to assess the differences in the binding kinetics of these antibodies to human TFPI under physiological (pH 7.4) and acidic (pH 6.0) conditions.

Antibodies were captured as ligands using AMC2 probes (Sartorius, Cat. Nos. 18-5163). Recombinant human TFPI protein solutions at concentrations ranging from 100 nM to 6.25 nM in pH 7.4 PBST solution (1× PBS, 0.05% Tween20) were used as analytes. Real-time binding of the antibodies to human TFPI and dissociation under pH 7.4 or pH 6.0 conditions were observed, and affinity analyses were performed separately. All measurements were conducted at 30°C. BLI signals were acquired and saved using Octet BLI Discovery 12.2 software, and data processing was performed using the Octet Analysis Studio 12.2 analysis software. The signal values from the detection channels were subtracted from the zero-concentration reference channel signal values to obtain corrected signal curves. Affinity kinetic curves were fitted according to the Langmuir 1:1 binding model, and the association rate constant (kon), dissociation rate constant (koff), and affinity constant (K_{D}) were calculated. The differences in dissociation rates under pH 7.4 and pH 6.0 conditions were compared to evaluate the pH dependency of the antibodies.

The kon, koff, and K_{D} values are shown in Table 4-2. The results indicate that the murine monoclonal antibody mAb3-71 showed a significantly lower dissociation rate at pH 6.0 compared to pH 7.4, demonstrating clear pH dependency. The dissociation rate ratios (pH 6.0/pH 7.4) for AB8D and Concizumab were 19.6 and 0.9, respectively, indicating that AB8D exhibits significant pH dependency.

**Table 4-2. Comparison of the Affinity of Anti-TFPI Antibodies for Human TFPI at pH 7.4 and pH 6.0**

| **Sample** | **K_{D} (M)** | **kon (1/Ms)** | **koff (1/s)** | **Koff Ratio (pH6.0/pH7.4)** | **K_{D} Ratio (pH6.0/pH7.4)** |
|---|---|---|---|---|---|
| mAb3-71 pH7.4 | <1.0E-12 | 3.056E05 | <1.0E-07 | / | / |
| mAb3-71 pH6.0 | 2.422E-10 | 3.727E05 | 9.024E-05 | | |
| AB8D pH7.4 | 1.447E-12 | 4.472E05 | 6.473E-07 | 19.6 | 17.7 |
| AB8D pH6.0 | 2.565E-11 | 4.948E05 | 1.269E-05 | | |
| Concizumab pH7.4 | <1.0E-12 | 4.956E05 | 4.013E-07 | 0.9 | / |
| Concizumab pH6.0 | <1.0E-12 | 5.667E05 | 3.600E-07 | | |

Based on real-time BLI measurements of the binding of other humanized antibodies obtained in Example 3 (Table 3) to human TFPI and their dissociation under pH 7.4 or pH 6.0 conditions, the results show that the other TFPI humanized antibodies listed in Table 3 also exhibit pH-dependent antigen-binding capability. The ratio of their K_{D} and/or koff for binding to TFPI at pH 6.0 relative to that at pH 7.4 is 2 or greater, preferably greater than 10-fold.

### Example 9: Determination of Antigenic Epitopes for Anti-Human TFPI Antibodies

The present invention used AlphaFold version 2.3.0 to predict the structure or interaction modes for a series of TFPI antigen/antibody complexes. The amino acid sequences used for AlphaFold modeling were the antibody light and heavy chain Fv region sequences and the human TFPI protein K2 domain sequence (K93-L152). The specific Linux command was as follows: python3 docker/run_docker.py --fasta_paths=multimer.fasta --max_template_date=2023-11-07 --model_preset=multimer --data_dir=$DOWNLOAD_DIR --output_dir=/home/ user/absolute_path_to_the_output_dir. In this command, run_docker.py is the Python application runner for AlphaFold version 2.3.0. multimer.fasta is the FASTA format amino acid sequence file for the antigen/antibody complex. 2023-11-07 is the date, meaning all structures in the Protein Data Bank before this date could serve as templates for modeling. multimer indicates that the molecule for this structure prediction is a multimer or complex. $DOWNLOAD_DIR is the directory name on the disk containing the search databases packaged with AlphaFold. absolute_path_to_the_output_dir should be filled with the output directory for the prediction results.

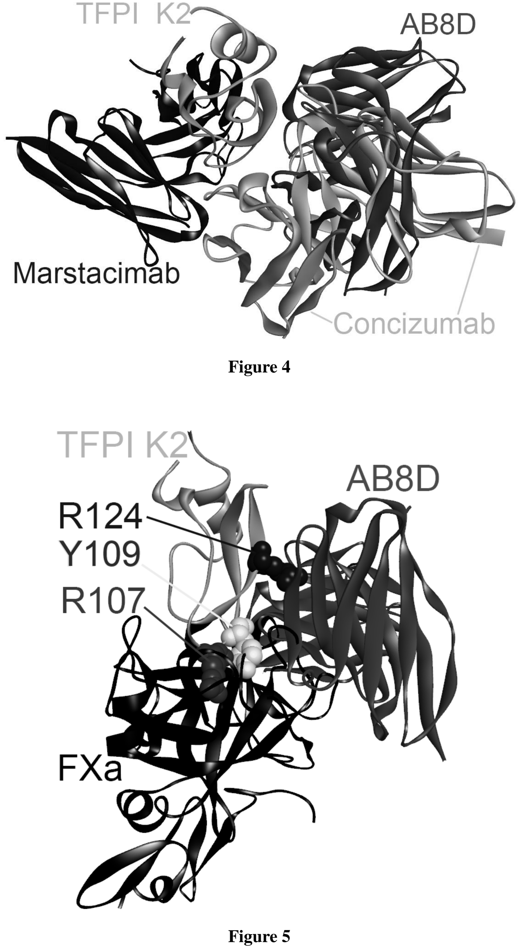

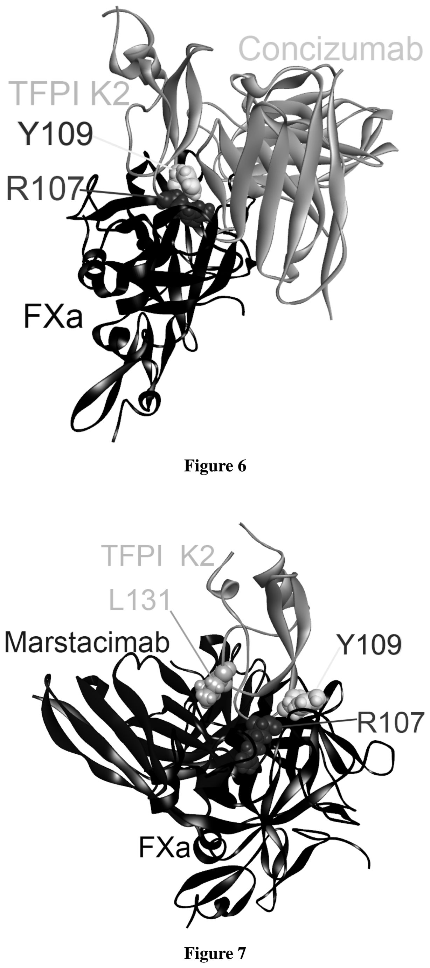

By default, AlphaFold uses five sets of modeling parameters, each with five prediction seeds, generating a total of 25 predicted structures. The predicted structures are scored for predicted accuracy on a scale of 0-100, with higher scores indicating higher confidence. The AlphaFold modeling results for the AB8D/TFPI K2 complex showed a confidence score of 86.00. The modeling confidence score for the Marstacimab antibody/TFPI K2 domain complex was 86.69. For the TFPI K2/coagulation factor FXa complex, AlphaFold modeling yielded a confidence score of 81.24. Schematic diagrams of the modeled structures for AB8D/TFPI K2, Concizumab/TFPI K2, and Marstacimab/TFPI K2 are shown in Figure 3. The superimposed structures of the AB8D/TFPI K2, Concizumab/TFPI K2, and Marstacimab/TFPI K2 complexes are shown in Figure 4. The superimposed structures of the AB8D/TFPI K2, Concizumab/TFPI K2, and Marstacimab/TFPI K2 complexes with the FXa/TFPI K2 complex are shown in Figures 5-7, respectively.

Based on the AlphaFold modeling results, antibody AB8D binds to the following amino acids on the TFPI K2 domain within 5 Å: T139, T119, E101, Y109, I110, T111, Y113, F114, N116, Q118, Q121, C122, E123, R124, F125, K126, E138, and L140 (Table 5). The Pfizer antibody Marstacimab binds to the following amino acids on the TFPI K2 domain within 5 Å: D102, G104-G108, I110, R112, Y127-M134, N136, E138. The crystal structure of the Novo Nordisk Concizumab antibody/TFPI K2 domain complex was obtained from the Protein Data Bank. Antibody Concizumab binds to the following amino acids on the TFPI K2 domain within 5 Å: E100-P103, R107, Y109, T111, Y113-F114, N116, Q118, Q121-K126, N133, E138, L140. By comparing the antigenic epitope amino acids bound by the three antibodies (AB8D, Marstacimab, and Concizumab), it was found that two antigenic epitope amino acids bound and/or occluded by the AB8D antibody within 5 Å, T139 and T119, are not bound and/or occluded by the other two antibodies, Concizumab and Marstacimab, within 5 Å. On the other hand, antibodies Concizumab and Marstacimab bind to R107 of the TFPI K2 domain within 5 Å. This amino acid is directly involved in inhibiting coagulation factor FXa and is at the center of the active region. The antibody AB8D of the present invention does not directly bind R107 within 5 Å, but binds to the Y109 amino acid near the spatial location of R107 within 5 Å. The Y109 amino acid of the TFPI K2 domain also has an important interaction with coagulation factor FXa and is a significant amino acid for TFPI/FXa binding, secondary to R107. Due to the large binding head or CDR exterior surface of the AB8D antibody, by binding Y109 and shielding the nearby R107 region, AB8D can block the binding between TFPI and coagulation factor FXa. Superposition of the AB8D/TFPI K2 and FXa/TFPI K2 complex structures shows that after alignment based on the common TFPI K2 domain, the AB8D antibody has spatial contact or steric hindrance with coagulation factor FXa, indicating that AB8D competes with FXa for binding to TFPI K2. Due to the high affinity of the AB8D antibody, it blocks the binding between TFPI K2 and coagulation factor FXa.

**Table 5. Amino Acids Involved in AB8D/TFPI K2 Antibody/Antigen Interaction (within 5 Å)**

| **TFPI K2 amino acid** | **AB8D heavy chain amino acid** | **AB8D light chain amino acid** | **Interaction type** |
|---|---|---|---|
| E101 | R57 | / | Electrostatic attraction |
| Y109 | / | Y95 | H-bond |
| Y109 | / | H94 | Van der Waals force |
| Y109 | / | P96 | Van der Waals force |
| Y109 | E62 | / | Van der Waals force |
| I110 | / | H94 | Van der Waals force |
| T111 | / | W92 | H-bond |
| T111 | / | S93 | H-bond |
| Y113 | V102 | / | H-bond |
| Y113 | / | Y33 | Van der Waals force |
| Y113 | / | W92 | Van der Waals force |
| F114 | R57 | / | Van der Waals force |
| N116 | Q55 | / | H-bond |
| Q118 | Q55 | / | H-bond |
| T119 | D54 | / | Van der Waals force |
| Q121 | V101 | / | Van der Waals force |
| C122 | V101 | / | Van der Waals force |
| C122 | V102 | / | Van der Waals force |
| E123 | K52 | / | Electrostatic attraction |
| E123 | R57 | / | Electrostatic attraction |
| R124 | W33 | / | Cation-pi bond |
| R124 | E50 | / | Electrostatic attraction |
| R124 | S99 | / | H-bond |
| R124 | H35 | / | Van der Waals force |
| R124 | / | Y95 | H-bond |
| F125 | R57 | / | Van der Waals force |
| K126 | E62 | / | Electrostatic attraction |
| E138 | / | Y33 | H-bond |
| T139 | / | Y33 | Van der Waals force |
| L140 | V102 | / | Hydrophobic interaction |
| According to the AlphaFold modeling results, antibody AB8D binds to the following amino acids within 5 Å on the TFPI K2 domain: T139, T119, E101, Y109, I110, T111, Y113, F114, N116, Q118, Q121, C122, E123, R124, F125, K126, E138, and L140. | | | |

### Example 10: Antibody/Antigen Dynamics Simulation of Anti-Human TFPI Antibodies

Gromacs software version 2022.4 was used to perform a 500 ns molecular dynamics simulation of the AB8D antibody/TFPI K2 complex. The simulation system was constructed using the pdb2gmx tool within the Gromacs software package, employing the Charmm36 force field and the TIP3P water model. The initial antigen/antibody complex model was centered in a dodecahedral box with a minimum distance of 10 Å between the model and the box edges. The initial antigen/antibody complex model was solvated by adding water molecules to the remaining space in the dodecahedral box, dissolving it in the TIP3P water solvent. Subsequently, 0.15 M NaCl ions were added using the Gromacs genion tool. After adding water and NaCl, the system was energy-minimized by performing up to 50,000 steps of the steepest descent algorithm. Following energy minimization, two position-restrained equilibrium simulations (NVT and NPT) were conducted for 2 ns each to bring the system to a state suitable for production dynamics simulation. Finally, a 500 ns production dynamics simulation was performed at 300 K temperature (V-rescale thermostat) and 1 atm pressure (Parrinello-Rahman barostat). Non-bonded interactions were handled using the Verlet cutoff scheme. Long-range electrostatic interactions were calculated using the Particle Mesh Ewald (PME) summation scheme. All bond lengths were constrained using the LINCS algorithm. The simulation time step was 2 fs, and low-precision trajectory coordinates were saved every 0.5 ns. The complete 500 ns simulation trajectory file contained 1000 frames of system conformations.

Using gmx_MMPBSA version 1.6.0-1.6.1, binding free energy calculations and per-residue binding free energy decomposition calculations were performed for a series of TFPI antigen/antibody complexes. The specific Linux command for the gmx_MMPBSA binding free energy calculation is as follows: mpirun -np 40 gmx_MMPBSA -O -i mmpbsa.in -cs com.tpr -ci index.ndx -cg 1 13 -ct com_traj.xtc -cp topol.top -o FINAL RESULTS MMPBSA. dat -eo FINAL_RESULTS MMPBSA.csv. In the above command line, mpirun -np 40 executes 40 gmx_MMPBSA processes simultaneously. mmpbsa.in is the program runtime parameter file. com.tpr is the topology structure parameter file for the antigen/antibody complex. index.ndx is the index file for groups of amino acid residue ranges, NaCl ions, or water molecules in the simulation system. -cg 1 13 indicates that the binding free energy calculation is performed between group 1 and group 13 amino acids defined in index.ndx. com_traj.xtc is the simulation trajectory file with the periodic boundary conditions removed from the dynamics simulation. topol.top is the topology file for the dynamics simulation. FINAL_RESULTS_MMPBSA.dat and

FINAL_RESULTS_MMPBSA.csv are output data files in different formats.The Linux command for the gmx_MMPBSA per-residue binding free energy decomposition calculation is similar to the binding free energy calculation command above. The specific command is as follows: mpirun -np 40 gmx_MMPBSA -O -i mmpbsa.in -cs com.tpr -ct com_traj.xtc -ci index.ndx -cg 1 13 -cp topol.top -o FINAL_RESULTS_MMPBSA.dat -eo FINAL_RESULTS_MMPBSA.csv -do FINAL_DECOMP_MMPBSA.dat -deo FINAL_DECOMP_MMPBSA. csv. Here, FINAL_DECOMP_MMPBSA.dat and FINAL_DECOMP _MMPBSA.csv are the binding free energy data decomposed per amino acid, presented in different formats. In the binding free energy calculation process, the polar solvation energy of the solute is calculated using the Poisson-Boltzmann (PB) equation, whereas in the per-amino acid decomposition calculation process, the solute polar solvation energy is calculated using the Generalized Born (GB) theory. Consequently, the total antigen/antibody binding free energy may slightly differ from the sum of the individual amino acid binding free energies calculated via decomposition.

The calculated binding free energies for the AB8D/TFPI K2, Concizumab/TFPI K2, and Marstacimab/TFPI K2 antibody/antigen complexes are shown in Table 6. Data on the contributions of key antigenic amino acids to the antigen/antibody binding free energy, calculated via per-residue decomposition (only amino acids with an absolute free energy contribution greater than 0.5 kcal/mol are listed), are shown in Table 7. In the AB8D/TFPI interaction, the TFPI R124 amino acid contributes significantly to the antigen/antibody binding free energy. The R124 amino acid exhibits strong electrostatic attraction with the AB8D antibody heavy chain E50 (see Table 5), cation-π interaction with the indole ring of the AB8D antibody heavy chain W33, hydrogen bonding with the AB8D antibody heavy chain S99, and hydrogen bonding with the AB8D antibody light chain Y95. It can be observed that the AB8D antibody has binding interactions with three amino acids of the TFPI K2 domain: Y113, L140, and Q121. These three amino acids do not interact with the Concizumab or Marstacimab antibodies, or in other words, they do not make significant contributions to the antigen/antibody binding free energy for Concizumab and Marstacimab. On the other hand, the AB8D antibody does not have significant interactions with the R107 amino acid of the TFPI K2 domain, whereas the Concizumab and Marstacimab antibodies have strong interactions with R107 (Table 7).

**Table 6. Antibody/Antigen Binding Free Energy**

| **Antibody/Antigen** | **Binding Free Energy (kcal/mol)** |
|---|---|
| AB8D/TFPI K2 | -54.86±15.2 |
| Concizumab//TFPI K2 | -60.98±15.67 |
| Marstacimab/TFPI K2 | -40.06±10.55 |

**Table 7. Contribution of Key TFPI Antigenic Amino Acids to Antigen/Antibody Binding Free Energy from Per-Residue Decomposition Calculation**

| **Serial Number** | **Concizuma b/ TFPI K2 Amino Acid** | **Contribution to Binding Free Energy (kcal/mol)** | **Marstacima b/ TFPI K2 Amino Acid** | **Contribution to Binding Free Energy (kcal/mol)** | **AB8D/TF PI K2 Amino Acid** | **Contribution to Binding Free Energy (kcal/mol)** |
|---|---|---|---|---|---|---|
| 1 | Y109 | -3.96±1.63 | L131 | -6.65±0.67 | R124 | -19.3±1.78 |
| 2 | R107 | -3.5±2.52 | I105 | -4.28±0.57 | Y109 | -3.18±1.03 |
| 3 | E100 | -2.33±2.88 | C130 | -4.00±0.44 | I110 | -3.11±0.82 |
| 4 | N116 | -2.05±1.3 | R107 | -3.52±1.85 | T111 | -2.58±2.04 |
| 5 | R124 | -1.86±1.32 | C106 | -2.74±0.38 | Y113 | -1.26±0.95 |
| 6 | Q118 | -1.59±1.09 | Y127 | -2.01±0.72 | L140 | -0.88±0.27 |
| 7 | T111 | -1.47±2.09 | M134 | -0.92±0.72 | Q121 | -0.6±0.28 |
| 8 | K126 | -0.9±2.98 | G108 | -0.62±0.2 | K126 | 0.51±1.52 |
| 9 | L99 | -0.64±0.49 | E138 | 1.48±0.59 | E101 | 0.57±1.17 |
| 10 | P103 | -0.6±0.29 | D102 | 1.76±0.42 | E123 | 0.96±1.07 |
| 11 | T119 | -0.54±0.28 | / | / | / | / |
| 12 | E101 | 0.66±1.14 | / | / | / | / |
| 13 | D102 | 0.91±0.5 | / | / | / | / |
| 14 | E123 | 1.01±0.49 | / | / | / | / |

### Example 11: Molecular Structural Basis for pH-Dependent Changes in Affinity of Anti-Human TFPI Antibodies for the TFPI K2 Antigen

Interacting salt bridges or ion pairs between amino acids exhibit strong interaction forces. When the ion-paired amino acids become uncharged, strong hydrogen bond interactions may still exist, but they are significantly weaker than those of salt bridges or ion pairs. The pH-dependent changes in antibody/antigen affinity primarily arise from the presence of weakly ionizable amino acids at the antibody/antigen binding interface that participate in the binding via salt bridges or ion pairs. When the pH changes near the pKa of these weakly ionizable amino acids, their protonation states are altered, thereby changing the antibody/antigen affinity. Under pH conditions below 7, the protonation states of weakly ionizable amino acids, primarily histidine, glutamic acid, and aspartic acid, undergo significant changes. The following predicts and describes the structural aspects of the pH-dependent affinity changes for antibodies AB8D, Concizumab, and Marstacimab to the antigen TFPI K2 under pH<7 conditions. Tables 5, 8, and 9 list the interacting amino acids between the three antibodies (AB8D, Concizumab, and Marstacimab) and the antigen TFPI K2, respectively. From Table 9, it can be seen that there are no significant electrostatic interactions in the form of salt bridges or ion pairs between antibody Marstacimab and antigen TFPI K2. Therefore, the affinity between Marstacimab and TFPI K2 should not be significantly affected by pH changes under pH<7 conditions. In Tables 5 and 8, AB8D and Concizumab each have approximately five pairs of electrostatic interactions, and pH changes may influence the strength of these attractive forces.

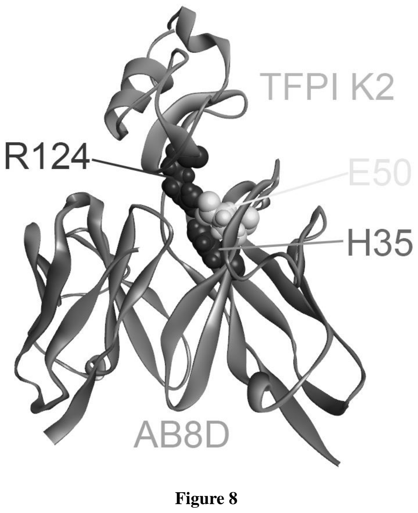

Tables 10 and 11 present the pKa values (calculated using the propka3 program) for weakly ionizable Glu/Asp amino acids at the binding interfaces of the AB8D/TFPI K2 and Concizumab/TFPI K2 complexes, respectively. In Table 10, the pKa of E50 in the AB8D heavy chain is significantly higher than the normal pKa of glutamic acid (4.50). Heavy chain amino acid E50 participates in the interaction with TFPI K2 amino acid R124 (Figure 8) and contributes substantially to the AB8D/TFPI K2 affinity (Tables 7, 12, and 13). Therefore, as the pH decreases from 7 to 6, given the pKa of E50 is 6.75, the pH change alters the protonation state of E50, reducing its degree of ionization and consequently decreasing the AB8D/TFPI K2 affinity, with a significant reduction (Table 15). Another notable feature of the AB8D antibody/antigen structure is that the AB8D heavy chain histidine H35 is spatially adjacent to heavy chain E50 and participates in the interaction with TFPI K2 R124 (Figure 8). The side chains of H35 and TFPI K2 R124 are separated by 3.51 Å. At pH 7.4, the interaction between H35 and TFPI K2 R124 is of the van der Waals type. When the pH decreases, for instance to pH 7.4-4.0, histidine H35 may become protonated, leading to electrostatic repulsion with TFPI K2 R124. In summary, on one hand, the high pKa (6.75) of heavy chain E50 results in reduced electrostatic attraction between E50 and TFPI K2 R124 as the pH drops; on the other hand, decreased pH may cause protonation of the heavy chain H35 side chain and the ensuing electrostatic repulsion between H35 and TFPI K2 R124. Thus, the combined effects of E50 and H35 likely contribute to the observed pH-dependent antigen binding phenomenon for the AB8D antibody. Furthermore, since the TFPI K2 R124 amino acid contributes significantly to the binding free energy between AB8D and the antigen, the AB8D heavy chain amino acids E50 and/or H35 interacting with TFPI K2 R124 are likely the primary residues responsible for the pH dependency of antibody AB8D within the pH range of 7.4-4.5.

In Table 10, TFPI K2 E101 participates in the interaction with heavy chain R57 (see Tables 7 and 13). Both amino acids contribute substantially to the binding free energy. Therefore, when the pH changes from 5 to 4, crossing the pKa of E101 (4.50), the affinity between AB8D and TFPI K2 will decrease significantly. Heavy chain E62 (pKa=3.71) participates in the electrostatic attraction with K126. The contribution of these two amino acids to the binding free energy is relatively small, being less than 0.5 kcal/mol or 0.51 kcal/mol, respectively (refer to Table 7 and Table 13; the absolute contribution of E62 to binding free energy is less than 0.5 kcal/mol and is not listed in Table 13). In Table 10, the TFPI K2 E123 amino acid (pKa=3.36) participates in the electrostatic interaction with K52. Their contributions to the binding free energy are 0.96 kcal/mol or -0.57 kcal/mol, respectively, which are not substantial. Therefore, when the pH decreases below 3.71-3.36, the reduction in antibody/antigen affinity due to heavy chain E62 or TFPI K2 E123 will not be significant.

Analyzing antibody Concizumab next, in Table 11, Concizumab heavy chain D106 (pKa=4.98) participates in an electrostatic interaction with TFPI K2 R124. These two amino acids have a certain degree of interaction force (refer to Tables 7, 8, and 14). Consequently, when the pH decreases to around 4.98, the affinity between Concizumab and TFPI K2 will decrease. TFPI K2 E101 (pKa=4.96) participates in an electrostatic interaction with heavy chain R53. E101's contribution to binding free energy is not large (0.66 kcal/mol), but R53 contributes significantly (-8.8 kcal/mol). Therefore, when the pH decreases to around 4.98, the affinity of this ion pair (E101/R53) will decrease substantially. E100 (pKa=4.68) also participates in an electrostatic interaction with heavy chain R53. E100 contributes relatively more to the binding free energy (-2.33 kcal/mol, Table 7). Thus, when the antibody's pH decreases to around 4.68, its affinity will decrease significantly. In Table 11, light chain E31 (pKa=3.98) participates in an electrostatic interaction with TFPI K2 R124. Both amino acids contribute substantially to the binding free energy (Tables 7 and 14). When the pH drops to around 3.98, it will significantly reduce the binding force between Concizumab and TFPI K2. Heavy chain D62 (pKa=3.93) participates in an electrostatic interaction with TFPI K2 R107. The contribution of heavy chain D62 to binding free energy is less than 0.5 kcal/mol (not listed in Table 14), but TFPI K2 R107 contributes significantly (-3.5 kcal/mol, Table 7). Hence, when the antibody's pH decreases to around 3.93, its affinity will still decrease considerably. In Table 11, light chain D33 (pKa=1.79) is relatively distant from the guanidinium group of TFPI K2 R124 (5.18 Å) and contributes minimally to binding free energy (0.56 kcal/mol, Table 14). Therefore, when the pH decreases to around 1.79, the change in antibody/antigen affinity due to light chain D33 before and after protonation will not be substantial.

According to the amino acid ionization formula, at pH = pKa, the ratio of protonated to deprotonated side chains is 1:1. When the pH is one unit below the pKa, the ratio becomes 10:1, at which point the amino acid side chain can be considered essentially uncharged. Therefore, summarizing the above, for antibody AB8D, when the pH decreases to one unit below the relevant pKa, i.e., pH 5.75 or 3.5, the antibody/antigen affinity will decrease significantly at each respective point. For antibody Concizumab, when the pH decreases to approximately 4.0 or 3.0, the antibody/antigen affinity will decrease significantly at each respective point.

Furthermore, a more detailed description and prediction of the pH-dependent characteristics of antibody/antigen affinity could be achieved by separately calculating the antibody/antigen binding free energy for the protonated and deprotonated states of each weakly ionizable amino acid listed in Tables 10 and 11, comparing the changes (Table 15), and then discussing these results in conjunction with the contributions of the charged amino acids to the binding free energy listed in Tables 7, 13, and 14.

**Table 8. Amino Acids Involved in Concizumab/TFPI K2 Antibody/Antigen Interaction (within 5 Å)**

| **TFPI K2 Amino Acid** | **Concizumab heavy chain amino acid** | **Concizumab light chain amino acid** | **interaction type** |
|---|---|---|---|
| E100 | R53 | / | Electrostatic attraction, H-bond |
| E100 | S54 | / | H-bond |
| E100 | S52 | / | H-bond |
| E101 | R53 | / | Electrostatic attraction, H-bond |
| D102 | Y57 | / | Van der Waals force |
| P103 | Y57 | / | Hydrophobic interaction |
| P103 | Y59 | / | Hydrophobic interaction |
| R107 | D62 | / | Electrostatic attraction |
| R107 | F60 | / | H-bond |
| Y109 | / | F99 | Hydrophobic interaction |
| T111 | / | E31 | H-bond |
| Y113 | / | S32 | H-bond |
| Y113 | / | D33 | H-bond |
| F114 | R53 | / | Van der Waals force |
| N116 | R53 | / | H-bond |
| Q118 | R53 | / | H-bond |
| Q118 | N31 | / | Van der Waals force |
| Q121 | D103 | / | H-bond |
| C122 | Y102 | / | Van der Waals force |
| E123 | Y102 | / | Van der Waals force |
| R124 | D106 | / | Electrostatic attraction |
| R124 | / | E31 | Electrostatic attraction |
| R124 | / | Y37 | Cation-pi bond |
| R124 | / | A96 | H-bond |
| F125 | / | Y59 | Van der Waals force |
| K126 | / | E31 | Electrostatic attraction |
| K126 | / | T97 | H-bond |
| N133 | Y57 | / | Van der Waals force |
| E138 | / | S32 | Van der Waals force |
| L140 | / | D33 | Van der Waals force |
| L140 | Y102 | / | Van der Waals force |
| Antibody Concizumab binds to the following amino acids within 5 Å on the TFPI K2 domain: E100-P103, R107, Y109, T111, Y113-F114, N116, Q118, Q121-K126, N133, E138, L140. | | | |

**Table 9. Amino Acids Involved in Marstacimab/TFPI K2 Antibody/Antigen Interaction (within 5 Å)**

| **TFPI K2 amino acid** | **Marstacimab heavy chain amino acid** | **Marstacimab light chain amino acid** | **interaction type** |
|---|---|---|---|
| D102 | Y59 | / | H-bond |
| G104 | Y59 | / | Van der Waals force |
| I105 | Y59 | / | Hydrophobic interaction |
| I105 | A33 | / | Hydrophobic interaction |
| I105 | A50 | / | Hydrophobic interaction |
| C106 | L99 | / | Hydrophobic interaction |
| R107 | Y32 | / | Cation-pi bond |
| R107 | S31 | / | H-bond |
| R107 | G100 | / | H-bond |
| R107 | L104 | / | Van der Waals force |
| G108 | L104 | / | Van der Waals force |
| I110 | / | G32 | Van der Waals force |
| R112 | / | Y33 | Cation-pi bond |
| R112 | / | A31 | H-bond |
| Y127 | / | Y33 | Van der Waals force |
| G128 | L104 | | Van der Waals force |
| G129 | / | Y33 | Van der Waals force |
| C130 | / | Y93 | Hydrophobic interaction |
| C130 | / | Y33 | Van der Waals force |
| L131 | W47 | / | Hydrophobic interaction |
| L131 | Y59 | / | Hydrophobic interaction |
| L131 | A50 | / | Hydrophobic interaction |
| G132 | Y59 | / | Van der Waals force |
| G132 | / | S98 | Van der Waals force |
| N133 | / | S98 | Van der Waals force |
| M134 | / | S98 | Van der Waals force |
| M134 | / | S96 | Van der Waals force |
| N136 | / | Y33 | Van der Waals force |
| E138 | / | A31 | Van der Waals force |
| Pfizer's antibody Marstacimab binds to the following amino acids within 5 Å on the TFPI K2 domain: D102, G104-G108, I110, R112, Y127-M134, N136, E138. | | | |

**Table 10. pKa of Glu/Asp Amino Acids Participating in Interaction at the AB8D/TFPI K2 Antibody/Antigen Binding Interface**

| **Serial Number** | **AB8D heavy chain amino acid** | **AB8D light chain amino acid** | **TFPI K2 amino acid** | **pKa of antibody or TFPI monomer in the free/unbound state** |
|---|---|---|---|---|
| 1 | E50 | / | / | 6.75 |
| 2 | / | / | E101 | 4.50 |
| 3 | E62 | / | / | 3.71 |
| 4 | / | / | E123 | 3.36 |

**Table 11. pKa of Glu/Asp Amino Acids Involved in the Interaction at the Concizumab/TFPI K2 Antibody/Antigen Binding Interface**

| **Serial Number** | **Concizumab heavy chain amino acid** | **Concizumab light chain amino acid** | **TFPI K2 amino acid** | **pKa of antibody or TFPI monomer in the free/unbound state** |
|---|---|---|---|---|
| 1 | D106 | / | / | 4.98 |
| 2 | / | / | E101 | 4.96 |
| 3 | / | / | E100 | 4.68 |
| 4 | / | E31 | / | 3.98 |
| 5 | D62 | / | / | 3.93 |
| 6 | / | D33 | / | 1.79 |

**Table 12. Pairwise Decomposition of Binding Free Energy for Amino Acid Pairs Interacting with TFPI K2 Amino Acid R124 in the AB8D/TFPI K2 Interaction (pairwise energy decomposition)**

| **Serial Number (sorted by importance of antigen-antibody binding in descending order)** | **AB8D heavy chain amino acid paired with TFPI K2 R124** | **AB8D light chain amino acid paired with TFPI K2 R124** | **Contribution to binding free energy from interaction with R124 (kcal/mol)** |
|---|---|---|---|
| 1 | E50 | / | -22.51 |
| 2 | S100 | / | -5.38 |
| 3 | W33 | / | -3.93 |
| 4 | S99 | / | -3.43 |
| 5 | D103 | / | -3.08 |
| 6 | V101 | / | -3.00 |
| 7 | V104 | / | -2.82 |
| 8 | V102 | / | -1.23 |
| Intra-residue interaction energy of R124 | / | / | 26.33 |

**Table 13. Contribution of AB8D Antibody Amino Acids to the AB8D/TFPI K2 Interaction Free Energy**

| **Serial Number(sorted by importance of antigen-antibody binding in descending order)** | **AB8D heavy chain amino acid** | **AB8D light chain amino acid** | **Contribution to binding free energy (kcal/mol)** |
|---|---|---|---|
| 1 | E50 | / | -5.16 |
| 2 | R57 | / | -4.71 |
| 3 | / | Y95 | -4.48 |
| 4 | V102 | / | -3.49 |
| 5 | V101 | / | -3.29 |
| 6 | S100 | / | -2.56 |
| 7 | / | H94 | -1.95 |
| 8 | S99 | / | -1.69 |
| 9 | W33 | / | -1.62 |
| 10 | V104 | / | -1.44 |
| 11 | / | W92 | -1.44 |
| 12 | / | S93 | -0.89 |
| | / | W33 | -0.84 |
| 13 | N59 | / | -0.83 |
| 14 | D103 | / | -0.83 |
| 15 | K52 | / | -0.57 |
| 16 | K65 | / | 0.65 |
| 17 | H35 | / | 0.7 |
| 18 | D54 | / | 1.11 |

**Table 14. Contribution of Concizumab Antibody Amino Acids to the Concizumab/TFPI K2 Interaction Free Energy**

| **Serial Number (sorted by importance of antigen-antibody binding in descending order)** | **Concizumab heavy chain amino acid** | **Concizumab light chain amino acid** | **Contribution to binding free energy (kcal/mol)** |
|---|---|---|---|
| 1 | R53 | / | -8.8 |
| 2 | S54 | / | -4.38 |
| 3 | S52 | / | -4.1 |
| 4 | S56 | / | -3.59 |
| 5 | Y102 | / | -3.37 |
| 6 | Y57 | / | -3.03 |
| 7 | / | F99 | -2.52 |
| 8 | / | E31 | -1.61 |
| 9 | G55 | / | -1.33 |
| 10 | / | T97 | -0.79 |
| 11 | / | P100 | -0.66 |
| 12 | / | D33 | 0.56 |
| 13 | / | D1 | 0.92 |
| 14 | D106 | / | 1.13 |

**Table 15. AB8D/TFPI K2 Antibody/Antigen Binding Free Energy**

| **Antibody/Antigen** | **Binding Free Energy (kcal/mol)** |
|---|---|
| **AB8D/TFPI K2** (heavy chain E50 side chain negatively charged, heavy chain H35 side chain uncharged, under normal pH 7.4 conditions) | -54.86±15.2 |
| **AB8D/TFPI K2** (heavy chain E50 side chain uncharged, heavy chain H35 side chain uncharged) | -23.57 ±16.4 |
| **AB8D/TFPI K2** (heavy chain E50 side chain negatively charged, heavy chain H35 side chain positively charged) | -23.41±20.07 |

### Example 12: Calculation of pKa for Amino Acids at the Antigen/Antibody Binding Interface of Anti-Human TFPI Antibodies

Based on the content of the preceding examples, the pKa values of Glu and Asp amino acids at the antigen/antibody binding interface, particularly heavy chain E50, for the other humanized antibodies obtained in Example 3 were calculated. The results are shown in Tables 16-25. The results indicate that the humanized antibodies obtained in the present invention all exhibit similar pH-dependent binding characteristics.

**Table 16. pKa of Glu/Asp Amino Acids Involved in the Interaction at the Humanized Antibody 1-1/TFPI K2 Antibody/Antigen Binding Interface**

| **Serial Number** | **Heavy chain amino acid** | **Light chain amino acid** | **TFPI K2 amino acid** | **pKa of antibody or TFPI monomer in the free state** |
|---|---|---|---|---|
| 1 | E50 | / | / | 6.83 |
| 2 | / | / | E101 | 4.50 |
| 3 | E62 | / | / | 3.68 |
| 4 | / | / | E123 | 3.36 |

**Table 17. pKa of Glu/Asp Amino Acids Involved in the Interaction at the Humanized Antibody 1-2/TFPI K2 Antibody/Antigen Binding Interface**

| **Serial Number** | **Heavy chain amino acid** | **Light chain amino acid** | **TFPI K2 amino acid** | **pKa of antibody or TFPI monomer in the free state** |
|---|---|---|---|---|
| 1 | E50 | / | / | 6.75 |
| 2 | / | / | E101 | 4.50 |
| 3 | E62 | / | / | 3.71 |
| 4 | / | / | E123 | 3.36 |

**Table 18. pKa of Glu/Asp Amino Acids Involved in the Interaction at the Humanized Antibody 1-4/TFPI K2 Antibody/Antigen Binding Interface**

| **Serial Number** | **Heavy chain amino acid** | **Light chain amino acid** | **TFPI K2 amino acid** | **pKa of antibody or TFPI monomer in the free state** |
|---|---|---|---|---|
| 1 | E50 | / | / | 6.69 |
| 2 | / | / | E101 | 4.50 |
| 3 | E62 | / | / | 3.72 |
| 4 | / | / | E123 | 3.36 |

**Table 19. pKa of Glu/Asp Amino Acids Involved in the Interaction at the Humanized Antibody 1-5/TFPI K2 Antibody/Antigen Binding Interface**

| **Serial Number** | **Heavy chain amino acid** | **Light chain amino acid** | **TFPI K2 amino acid** | **pKa of antibody or TFPI monomer in the free state** |
|---|---|---|---|---|
| 1 | E50 | / | / | 6.72 |
| 2 | / | / | E101 | 4.50 |
| 3 | E62 | / | / | 3.70 |
| 4 | / | / | E123 | 3.36 |

**Table 20. pKa of Glu/Asp Amino Acids Involved in the Interaction at the Humanized Antibody 1-6/TFPI K2 Antibody/Antigen Binding Interface**

| **Serial Number** | **Heavy chain amino acid** | **Light chain amino acid** | **TFPI K2 amino acid** | **pKa of antibody or TFPI monomer in the free state** |
|---|---|---|---|---|
| 1 | E50 | / | / | 7.31 |
| 2 | / | / | E101 | 4.50 |
| 3 | E62 | / | / | 3.69 |
| 4 | / | / | E123 | 3.36 |

**Table 21. pKa of Glu/Asp Amino Acids Involved in the Interaction at the Humanized Antibody 1-7/TFPI K2 Antibody/Antigen Binding Interface**

| **Serial Number** | **Heavy chain amino acid** | **Light chain amino acid** | **TFPI K2 amino acid** | **pKa of antibody or TFPI monomer in the free state** |
|---|---|---|---|---|
| 1 | E50 | / | / | 6.40 |
| 2 | / | / | E101 | 4.50 |
| 3 | E62 | / | / | 3.74 |
| 4 | / | / | E123 | 3.36 |

**Table 22. pKa of Glu/Asp Amino Acids Involved in the Interaction at the Humanized Antibody 2-1/TFPI K2 Antibody/Antigen Binding Interface**

| **Serial Number** | **Heavy chain amino acid** | **Light chain amino acid** | **TFPI K2 amino acid** | **pKa of antibody or TFPI monomer in the free state** |
|---|---|---|---|---|
| 1 | E50 | / | / | 7.15 |
| 2 | / | / | E101 | 4.50 |
| 3 | E62 | / | / | 3.68 |
| 4 | / | / | E123 | 3.36 |

**Table 23. pKa of Glu/Asp Amino Acids Involved in the Interaction at the Humanized Antibody 2-4/TFPI K2 Antibody/Antigen Binding Interface**

| **Serial Number** | **Heavy chain amino acid** | **Light chain amino acid** | **TFPI K2 amino acid** | **pKa of antibody or TFPI monomer in the free state** |
|---|---|---|---|---|
| 1 | E50 | / | / | 6.25 |
| 2 | / | / | E101 | 4.50 |
| 3 | E62 | / | / | 3.69 |
| 4 | / | / | E123 | 3.36 |

**Table 24. pKa of Glu/Asp Amino Acids Involved in the Interaction at the Humanized Antibody 2-6/TFPI K2 Antibody/Antigen Binding Interface**

| **Serial Number** | **Heavy chain amino acid** | **Light chain amino acid** | **TFPI K2 amino acid** | **pKa of antibody or TFPI monomer in the free state** |
|---|---|---|---|---|
| 1 | E50 | / | / | 5.89 |
| 2 | / | / | E101 | 4.50 |
| 3 | E62 | / | / | 3.68 |
| 4 | / | / | E123 | 3.36 |

**Table 25. pKa of Glu/Asp Amino Acids Involved in the Interaction at the Humanized Antibody 2-9/TFPI K2 Antibody/Antigen Binding Interface**

| **Serial Number** | **Heavy chain amino acid** | **Light chain amino acid** | **TFPI K2 amino acid** | **pKa of antibody or TFPI monomer in the free state** |
|---|---|---|---|---|
| 1 | E50 | / | / | 6.40 |
| 2 | / | / | E101 | 4.50 |
| 3 | E62 | / | / | 3.71 |
| 4 | / | / | E123 | 3.36 |

### Example 13: Affinity Determination of Anti-Human TFPI Antibodies

The binding affinity constants of the anti-human TFPI antibodies to human TFPI were determined according to the method described in Example 2. The affinity results are shown in Table 26. The results demonstrate that the anti-human TFPI monoclonal antibodies provided by the present invention exhibit extremely high binding affinity to human TFPI, with K_{D} values reaching the order of 10⁻¹² M.

**Table 26. Affinity Determination Results of Anti-Human TFPI Antibodies**

| **Antibody** | **K_{D} (M)** |
|---|---|
| AB8D | 1.447E-12 |
| Antibody 1-1 | 1.124E-12 |
| Antibody 1-2 | 1.070E-12 |
| Antibody 1-3 | 2.457E-12 |
| Antibody 1-4 | 1.567E-12 |
| Antibody 1-5 | 2.231E-12 |
| Antibody 1-6 | <1.0E-12 |
| Antibody 1-7 | 1.326E-12 |
| Antibody 1-8 | 1.154E-12 |
| Antibody 1-9 | 1.578E-12 |
| Antibody 1-10 | 1.123E-12 |
| Antibody 2-1 | <1.0E-12 |
| Antibody 2-2 | 1.534E-12 |
| Antibody 2-3 | 1.321E-12 |
| Antibody 2-4 | 1.654E-12 |
| Antibody 2-5 | 1.263E-12 |
| Antibody 2-6 | 1.543E-12 |
| Antibody 2-7 | 1.492E-12 |
| Antibody 2-8 | 1.650E-12 |
| Antibody 2-9 | 1.325E-12 |
| Antibody 2-10 | 1.076E-12 |
| Antibody 2-11 | 1.651E-12 |

### Example 14: In Vitro Activity Study of Anti-Human TFPI Antibodies - TFPI Neutralization Assay: FVlla/TF/FX Inhibition

Tissue factor pathway inhibitor (TFPI) is a key negative regulator of the extrinsic coagulation pathway. The mechanism of TFPI's anticoagulant action involves initiation through direct binding of its K2 domain to FXa, thereby inhibiting FXa activity and forming a TFPI/FXa complex. Within the TFPI/FXa complex, the K1 domain of TFPI binds to FVIIa in the tissue factor complex FVIIa/TF, forming a quaternary complex that inhibits the activity of the FVIIa/TF complex and prevents further activation of FX to FXa. The TFPI monoclonal antibodies of the present invention are designed to bind to the K2 domain of TFPI, blocking its interaction with FXa and the formation of the complex, thereby restoring the ability of the tissue factor complex FVIIa/TF to activate FX and promoting FXa generation. In this assay, FVIIa, TF, TFPI, FX, and the test article are co-incubated. The neutralizing effect of the test article on TFPI activity is evaluated by measuring the absorbance from the FXa-catalyzed substrate S-2765 reaction.

The research results demonstrate that all 22 humanized antibodies of the present invention possess the biological function of restoring the TFPI-mediated inhibition of the FVIIa/TF/FX coagulation pathway and exhibit neutralizing activity against TFPI. In this test system, the EC₅₀ values of the humanized antibodies of the invention range from 8.64 to 22.12 µg/mL.

### Example 15: In Vivo Pharmacodynamic Evaluation of Anti-Human TFPI Antibodies

Forty conventional male New Zealand White rabbits were randomly divided into 5 groups based on body weight. The rabbits in each group received a single subcutaneous injection in the dorsal neck region with the corresponding test article or vehicle on Day 1. All rabbits were then induced with A-type Hemophilia (Hemophilia A, HA) via intravenous injection of an anti-FVIII antibody via the marginal ear vein on Day 8. During the experiment, bleeding was induced in all rabbits 45 minutes after anti-FVIII antibody administration on Day 8 by nail clipping to cause stratum corneum bleeding. The initial bleeding time and bleeding volume were measured. Blood samples were collected at three time points: before HA modeling on Day 8 (prior to anti-FVIII antibody injection that day), before bleeding induction (45 minutes after anti-FVIII antibody injection that day), and 60 minutes after bleeding induction. The samples were analyzed for plasma activated partial thromboplastin time (APTT), diluted prothrombin time (dPT), and Factor-VIII (FVIII) activity.

The results showed that a single subcutaneous dose of 10 mg/kg of the positive control Marstacimab (a biosimilar of PF-06741086) significantly reduced bleeding volume and, to a certain extent, shortened initial bleeding time when observed 7 days post-administration. Single subcutaneous doses of 5 mg/kg, 10 mg/kg, and 20 mg/kg of AB8D resulted in a dose-dependent reduction in bleeding volume and shortening of initial bleeding time when observed 7 days post-administration (with the 20 mg/kg dose showing statistically significant differences in both bleeding volume reduction and initial bleeding time shortening). A single subcutaneous dose of 10 mg/kg and 20 mg/kg of AB8D significantly shortened dPT in HA model rabbits at 105 minutes post-modeling (i.e., after bleeding induction) compared to pre-modeling levels when observed 7 days post-administration. The results indicate that, under the conditions of this study, AB8D shortened initial bleeding time and dPT, and reduced bleeding volume, demonstrating a therapeutic effect.

**Table 27. Blood Volume and Initial Hemostasis Time (n=8)**

| **Group** | | **Bleeding Volume (g)** | **Initial Hemostasis Time (s)** |
|---|---|---|---|
| G1: Anti-FVIII antibody + Vehicle | Mean | 50.28 | 1817.63 |
| | SEM | 5.68 | 536.82 |
| G2: Anti-FVIII antibody + Marstacimab - 10 mg/kg | Mean | 10.75*** | 750.13 |
| | SEM | 1.31 | 416.81 |
| G3: Anti-FVIII antibody + AB8D - 5 mg/kg | Mean | 45.75 | 897.25 |
| | SEM | 3.42 | 397.43 |
| G4: Anti-FVIII antibody + AB8D - 10 mg/kg | Mean | 42.19 | 594.00 |
| | SEM | 4.03 | 115.75 |
| G5: Anti-FVIII antibody + AB8D - 20 mg/kg | Mean | 10.44*** | 415.25* |
| | SEM | 1.11 | 91.46 |

| | | | |
|---|---|---|---|
| Note: Compared with Group G1 at the same time point, *P<0.05; **P<0.01; ***P<0.001. The same applies below. | | | |

**Table 28. Diluted Prothrombin Time (n=7-8)**

| **Group** | | **dPT(s)** | | |
|---|---|---|---|---|
| | | **Day 8 0min** | **Day 8 45min** | **Day 8 105min** |
| G1: Anti-FVIII antibody + Vehicle | Mean | 130.26 | 125.58 | 128.36 |
| | SEM | 6.05 | 9.41 | 5.35 |
| G2: Anti-FVIII antibody + Marstacimab - 10 mg/kg | Mean | 113.53 | 112.91 | 121.16 |
| | SEM | 4.08 | 4.62 | 5.63 |
| G3: Anti-FVIII antibody + AB8D - 5 mg/kg | Mean | 122.91 | 124.32 | 124.80 |
| | SEM | 5.24 | 5.14 | 11.07 |
| G4: Anti-FVIII antibody + AB8D - 10 mg/kg | Mean | 102.13** | 104.19 | 102.92** |
| | SEM | 4.77 | 1.73 | 2.46 |
| G5: Anti-FVIII antibody + AB8D - 20 mg/kg | Mean | 104.68* | 104.07 | 100.51** |
| | SEM | 5.04 | 4.87 | 3.22 |

### Example 16: Safety Evaluation of Anti-Human TFPI Antibodies in Rhesus Monkeys

In a four-week repeated-dose toxicity study with a four-week recovery period in rhesus monkeys, which included concomitant safety pharmacology assessments of the central nervous system, cardiovascular system, and respiratory system following subcutaneous injection of the AB8D monoclonal antibody injection, the AB8D monoclonal antibody injection showed no significant effects on the central nervous system, cardiovascular system, or respiratory system of monkeys, and no local irritancy at the injection site was observed.

In a dose range finding and maximum tolerated dose study involving subcutaneous injection of AB8D monoclonal antibody bulk solution in rhesus monkeys, all animals survived until the scheduled necropsy. In the test article administration groups, female animals exhibited intermittent watery/liquid stools, decreased body weight, and at the end of the observation period, prolonged APTT, elevated serum BUN and CREA, and decreased Na and Cl levels were observed. Histopathological examination revealed mild myocardial cell degeneration in the heart and minimal macrophage aggregation in the lungs. Due to the limited number of animals, a definitive relationship of these abnormal changes to the test article could not be established.

In the four-week repeated-dose toxicity study with a four-week recovery period involving subcutaneous injection of the AB8D monoclonal antibody injection in rhesus monkeys, the observed changes included: compared to the vehicle control group, a decrease in plasma fibrinogen was observed in both male and female animals from 168 hours after the first dose until the end of the recovery period (Day 57) at doses ≥10 mg/kg; an increase or trend of increase in D-dimer was observed in both male and female animals from 24 hours after the first dose during the dosing period until the end of the dosing period (Day 29) at doses ≥10 mg/kg, while a trend of increase in D-dimer was observed at the end of the recovery period (Day 57) in both male and female animals at doses ≥30 mg/kg; histopathological examination revealed no thrombosis observed during or after the study period and no test article-related toxicological changes at doses of 10 and 30 mg/kg.

In the in vitro hemolysis test of the AB8D monoclonal antibody injection, the AB8D monoclonal antibody injection did not cause hemolysis or agglutination of rabbit red blood cells in vitro, indicating suitability for subcutaneous injection.

In the four-week repeated-dose toxicity study with a four-week recovery period in rhesus monkeys, which included an immunotoxicity evaluation following subcutaneous injection of the AB8D monoclonal antibody injection, no test article-related abnormalities were observed in immunotoxicity evaluation parameters. This specifically included hematological white blood cell indices, serum biochemical albumin and globulin levels, lymphocyte immunophenotyping (CD3⁺CD4⁺, CD3⁺CD8⁺, CD3⁺CD4⁺/CD3⁺CD8⁺, CD20⁺, CD16⁺), immunoglobulins and complement (IgA, IgG, IgM, C3, C4), immune organ weights (spleen and thymus), and gross anatomical observations and histopathological examinations of the spleen, thymus, lymph nodes (draining lymph nodes: inguinal and mesenteric), and bone marrow.

In a study of cytokine release from human peripheral blood mononuclear cells (PBMCs) incubated with the AB8D monoclonal antibody injection, the AB8D monoclonal antibody injection did not stimulate PBMCs to release IL-2, IL-4, IL-6, IL-10, TNF-α, or IFN-γ.

### Example 17: Pharmacokinetic Study of Anti-Human TFPI Antibodies in Rhesus Monkeys

Rhesus monkeys were randomly divided into 2 groups (6 monkeys per group, half male and half female). They received a single subcutaneous injection of the AB8D humanized antibody at doses of 15 mg/kg or 50 mg/kg, respectively. The pharmacokinetic parameters obtained from the analysis are shown in Table 29. After subcutaneous injection of the 50 mg/kg dose, the half-life of the AB8D humanized antibody reached 136 hours.

**Table 29. Pharmacokinetic Parameters in Rhesus Monkeys**

| **Administration method** | **Subcutaneous injection** | **Subcutaneous injection** |
|---|---|---|
| **Dose (mg/kg)** | 15 | 50 |
| **Sample** | Plasma | Plasma |

| **PK Parameters:** | | |
|---|---|---|
| AUC_{INF_obs}(h*µg/mL) | 27100±5280 | 123000±13900 |
| AUCₗₐₛₜ(h*µg/mL) | 26900±5410 | 123000±13900 |
| C₀(µg/mL) | - | - |
| Cₘₐₓ(µg/mL) | 137±16.7 | 523±26.9 |
| T_{1/2_z}(h) | 37.9±7.58 | 136±42.5 |
| Tₘₐₓ(h) | 36.0±36.0 | 36.0±24.0 |
| MRT_{INF_obs}(h) | 129±9.84 | 179±16.5 |
| MRTₗₐₛₜ(h) | 127±12.0 | 177±16.8 |
| Cl_{_F_obs}(mL/h/kg) | 0.571±0.104 | 0.410±0.0484 |
| Cl _{_obs}(mL/h/kg) | - | - |
| V_{z_F_obs}(mL/kg) | 31.7±10.7 | 79.3±22.4 |
| V_{z_obs}(mL/kg) | - | - |
| F(%) | 104 | - |

### Example 18: Safety Evaluation of Anti-Human TFPI Antibodies in Healthy Chinese Adult Subjects

Healthy subjects receiving single doses of the AB8D monoclonal antibody ranging from 30 to 200 mg exhibited a certain dose correlation in the incidence of adverse events. The vast majority of adverse events were Grade 1 in severity (according to CTCAE 5.0 grading), with only one Grade II adverse event reported, which was judged by the investigator to be unrelated to the investigational drug. All adverse events were transient, with outcomes of complete recovery. No serious adverse events, adverse events leading to withdrawal, or death events were reported. No clinically significant trends were observed in important safety laboratory parameters for any subject. No other potentially important safety issues related to the AB8D monoclonal antibody were reported. Healthy subjects tolerated single subcutaneous injections of the AB8D monoclonal antibody at doses from 10 to 200 mg, with overall good safety and tolerability.

Globally, several drugs targeting the same pathway (anti-TFPI monoclonal antibodies) are under development for the treatment of Hemophilia A and B. Only limited data have been disclosed for Concizumab and Marstacimab. Concizumab has been approved in Canada, Japan, Switzerland, and other regions. Marstacimab is at the highest global development stage of Phase III clinical trials. Adverse events reported for Concizumab in healthy subjects were mild or moderate in severity, with the most common adverse reactions being headache, common cold, fatigue, or influenza. Adverse events reported for Marstacimab in healthy subjects were mild or moderate in severity, with the most common adverse events including headache, fatigue, and local or systemic pain/discomfort. In the Phase I clinical trial of the AB8D monoclonal antibody, all reported adverse events were mild in severity, and headache, fatigue, or systemic pain/discomfort were not reported, indicating a superior safety profile compared to similar products.

### Example 19: Clinical Pharmacokinetic Study of Anti-Human TFPI Antibody Following a Single Dose in Healthy Chinese Adults

An ELISA method was used to quantitatively determine the concentration of active AB8D in human plasma. Human TFPI/His (purchased from ACRO Biosystem, Cat. No. TFI-H5226) was diluted to 0.1 µg/ml in 1× PBS buffer and added to a 96-well microplate at 100 µl/well, then incubated at 4°C for 16-20 hours. The PBS buffer in the plate was removed, and the wells were blocked by adding 200 µl/well of PBST/1.5% BSA and incubating at room temperature for 1 hour. After washing the plate three times with PBST (pH 7.4, PBS containing 0.05% Tween-20), the test plasma samples were added at 100 µl/well and incubated at room temperature for 1.0 hour. The plate was washed three times with PBST, then 100 µl/well of a 10 µg/ml biotin-labeled mouse anti-AB8D idiotype antibody (produced by Ampsource Biopharma, AP651651) was added and incubated at room temperature for 1 hour. After washing three times with PBST, 100 µl/well of a 1:5000 dilution of SA-Avidin-HRP was added and incubated at room temperature for 1 hour. The plate was washed three times with PBST, then 100 µl/well of TMB substrate was added and incubated at room temperature for 5-10 minutes. The reaction was stopped by adding 50 µl/well of 0.2 M sulfuric acid. Absorbance was read at dual wavelengths of 450/620 nm using a microplate reader.

Pharmacokinetically, the AB8D humanized antibody exhibited non-linear pharmacokinetic characteristics within the tested dose range, with a target-mediated drug disposition (TMDD) effect. The pharmacokinetic parameters in healthy subjects are shown in Table 30. Following subcutaneous administration in healthy subjects, the time to peak concentration (Tmax) ranged from 31 to 36 hours; the mean t1/2 ranged from 104 to 145 hours, with the half-life increasing as the dose increased. Compared to drugs targeting the same pathway, the time to peak concentration was faster and the half-life was longer, both of which are PK characteristics associated with TMDD.

**Table 30. Pharmacokinetic Parameters in Healthy Subjects**

| **Dose Group** | **S1 Group (30mg)** | **S2 Group (100mg)** |
|---|---|---|
| **Sample Size (n)** | N=4 | N=8 |
| Tmax, h | 31.00(10~96) | 36.00(4~48) |
| Cmax, ng/mL(CV) | 74.40±9.52 (12.80) | 1,935.63±2179.05 (112.58) |
| AUC0-t, h*ng/mL(CV) | 8,691.65±1293.08 (14.88) | 108,490.75±92644.93 (85.39) |
| AUC0-∞, h*ng/mL(CV) | 13,356.91±1896.87 (14.20) | 116,282.68±92218.51 (79.31) |
| t1/2, h | 116,282.68±92218.51 (79.31) | 145.85±35.03 (24.02) |
| MRT0-t,h | 80.8 | 80.67 |

### Example 20: Clinical Pharmacodynamic Study of Anti-Human TFPI Antibody Following a Single Dose in Healthy Chinese Adults

A sandwich ELISA method was used to quantitatively determine the concentration of TFPI in human plasma. AB8D was diluted to 5 µg/ml in 1× PBS buffer and added to a 96-well microplate at 100 µl/well, then incubated at 4°C for 16-20 hours. The PBS buffer in the plate was removed, and the wells were blocked by adding 200 µl/well of PBST/1.5% BSA and incubating at room temperature for 1 hour. After washing the plate three times with PBST (pH 7.4, PBS containing 0.05% Tween-20), the test plasma samples were added at 100 µl/well and incubated at room temperature for 1.0 hour. The plate was washed three times with PBST, then 100 µl/well of a 0.1 µg/ml rabbit anti-human TFPI antibody (purchased from Abcam, ab274436) was added and incubated at room temperature for 1 hour. After washing three times with PBST, 100 µl/well of a 1:80000 dilution of Peroxidase-conjugated AffiniPure Donkey Anti-Rabbit IgG(H+L) (purchased from Jackson Immuno Research) was added and incubated at room temperature for 1 hour. The plate was washed three times with PBST, then 100 µl/well of TMB substrate was added and incubated at room temperature for 5-10 minutes. The reaction was stopped by adding 50 µl/well of 0.2 M sulfuric acid. Absorbance was read at dual wavelengths of 450/620 nm using a microplate reader.

A dose-dependent reduction in free TFPI levels was observed following administration of the AB8D monoclonal antibody at doses ranging from 30 mg to 200 mg. The AB8D monoclonal antibody rapidly reduced TFPI levels within 8 hours after the first dose. Free TFPI levels returned to baseline around day 14 in the low-dose group (30 mg) and around day 21 in the high-dose group (200 mg).

While preferred examples of the present invention have been illustrated and described, it should be understood that those skilled in the art may make various modifications in light of the teachings herein, provided such modifications do not depart from the scope of the present invention.

All documents mentioned in the present invention are incorporated herein by reference, as if each document were individually incorporated by reference. Furthermore, it should be understood that after reading the above teachings of the present invention, those skilled in the art may make various modifications or alterations to the present invention, and such equivalent forms are likewise intended to fall within the scope defined by the appended claims of this application.

## Claims

1. An antibody or an antigen-binding fragment thereof that binds to Tissue Factor Pathway Inhibitor (TFPI), **characterized in that** binding affinity of the antibody or antigen-binding fragment thereof to TFPI is pH-dependent over a pH range of 4.5 to 7.4.

2. The antibody or antigen-binding fragment thereof according to claim 1, **characterized in that** the half-life of 100 mg of the antibody or antigen-binding fragment thereof in the human body exceeds 100 hours, preferably exceeds 120 hours, and more preferably exceeds 140 hours.

3. The antibody or antigen-binding fragment thereof according to claim 1 or 2, **characterized in that** the binding between the antibody or antigen-binding fragment thereof and TFPI occurs through amino acid residues of the tissue factor pathway inhibitor, wherein the amino acid residues comprise basic amino acid residues of the tissue factor pathway inhibitor.

4. The antibody or antigen-binding fragment thereof according to claim 3, **characterized in that** the basic amino acid is at least one amino acid residue selected from the group consisting of R124, R112, K93, K120, K126, and K144, and preferably the basic amino acids are R124 and K126.

5. The antibody or antigen-binding fragment thereof according to claim 1 or 2, **characterized in that** the amino acid residues further comprise T139 and/or T119, and wherein the amino acid residues do not include R107.

6. The antibody or antigen-binding fragment thereof according to claim 5, **characterized in that** the amino acid residues further comprise Y109, preferably the amino acid residues further comprise at least one amino acid residue selected from the group consisting of I110, T111, F125, and E138, and/or preferably the amino acid residues further comprise at least one amino acid residue selected from the group consisting of E101, Y113, F114, N116, Q118, Q121, C122, E123, and L140.

7. The antibody or antigen-binding fragment thereof according to claim 1, **characterized in that** the amino acid sequence of TFPI is as shown in SEQ ID NO: 1.

8. The antibody or antigen-binding fragment thereof according to claim 1, **characterized in that** the antibody or antigen-binding fragment comprises a heavy chain variable region (VH) and a light chain variable region (VL), wherein:
(a) the heavy chain variable region as defined according to the Kabat numbering system comprises at least one, two, or three complementarity determining regions (CDRs) selected from the group consisting of:
(1) HCDR1 having the amino acid sequence SYWMH;
(2) HCDR2 having the amino acid sequence EIKPDX₁GRIX₂YNEKFKT, wherein X₁ is selected from Q, H, or K, and X₂ is selected from N, D, or H; and
(3) HCDR3 having the amino acid sequence SSX₃X₄DX₅AX₆DY, wherein X₃ is selected from V or H, X₄ is selected from V, I, F, Y, or L, X₅ is selected from V, D, T, or E, and X₆ is selected from M, L, I, E, V, or D;
and/or, the light chain variable region comprises at least one, two, or three CDRs selected from the group consisting of:
(4) LCDR1 having the amino acid sequence SASSSVSSSYLY;
(5) LCDR2 having the amino acid sequence GTSILAS; and
(6) LCDR3 having the amino acid sequence HQWX₇X₈YPFT, wherein X₇ is selected from S or T, and X₈ is selected from H, Y, or F;
or,
(b) the heavy chain variable region as defined according to the IMGT numbering system comprises at least one, two, or three CDRs selected from the group consisting of:
(1) HCDR1 having the amino acid sequence GYTFTSYW;
(2) HCDR2 having the amino acid sequence IKPDX₁GRI, wherein X1 is selected from Q, H, or K; and
(3) HCDR3 having the amino acid sequence ARSSX₃X₄DX₅AX₆DY, wherein X₃ is selected from V or H, X₄ is selected from V, I, F, Y, or L, X₅ is selected from V, D, T, or E, and X₆ is selected from M, L, I, E, V, or D;
and/or, the light chain variable region comprises at least one, two, or three CDRs selected from the group consisting of:
(4) LCDR1 having the amino acid sequence SSVSSSY;
(5) LCDR2 having the amino acid sequence GTS; and
(6) LCDR3 having the amino acid sequence HQWX₇X₈YPFT, wherein X₇ is selected from S or T, and X₈ is selected from H, Y, or F.

9. The antibody or antigen-binding fragment thereof according to claim 8, **characterized in that** the antibody or antigen-binding fragment comprises three VH CDRs and three VL CDRs, selected from the group consisting of:
(1) its HCDR1, HCDR2, HCDR3, LCDR1, LCDR2, and LCDR3 respectively having sequences as shown in SEQ ID NO: 46, 47, 48, 49, 50, or 51, or sequences having one or several amino acid substitutions, deletions, or additions (e.g., 1, 2, or 3 substitutions, deletions, or additions) compared to any of the aforementioned sequences;
(2) its HCDR1, HCDR2, HCDR3, LCDR1, LCDR2, and LCDR3 respectively having sequences as shown in SEQ ID NO: 46, 47, 56, 49, 50, or 51, or sequences having one or several amino acid substitutions, deletions, or additions (e.g., 1, 2, or 3 substitutions, deletions, or additions) compared to any of the aforementioned sequences;
(3) its HCDR1, HCDR2, HCDR3, LCDR1, LCDR2, and LCDR3 respectively having sequences as shown in SEQ ID NO: 46, 47, 58, 49, 50, or 51, or sequences having one or several amino acid substitutions, deletions, or additions (e.g., 1, 2, or 3 substitutions, deletions, or additions) compared to any of the aforementioned sequences;
(4) its HCDR1, HCDR2, HCDR3, LCDR1, LCDR2, and LCDR3 respectively having sequences as shown in SEQ ID NO: 46, 60, 48, 49, 50, or 51, or sequences having one or several amino acid substitutions, deletions, or additions (e.g., 1, 2, or 3 substitutions, deletions, or additions) compared to any of the aforementioned sequences;
(5) its HCDR1, HCDR2, HCDR3, LCDR1, LCDR2, and LCDR3 respectively having sequences as shown in SEQ ID NO: 46, 61, 48, 49, 50, or 51, or sequences having one or several amino acid substitutions, deletions, or additions (e.g., 1, 2, or 3 substitutions, deletions, or additions) compared to any of the aforementioned sequences;
(6) its HCDR1, HCDR2, HCDR3, LCDR1, LCDR2, and LCDR3 respectively having sequences as shown in SEQ ID NO: 46, 63, 48, 49, 50, or 51, or sequences having one or several amino acid substitutions, deletions, or additions (e.g., 1, 2, or 3 substitutions, deletions, or additions) compared to any of the aforementioned sequences;
(7) its HCDR1, HCDR2, HCDR3, LCDR1, LCDR2, and LCDR3 respectively having sequences as shown in SEQ ID NO: 46, 65, 48, 49, 50, or 51, or sequences having one or several amino acid substitutions, deletions, or additions (e.g., 1, 2, or 3 substitutions, deletions, or additions) compared to any of the aforementioned sequences;
(8) its HCDR1, HCDR2, HCDR3, LCDR1, LCDR2, and LCDR3 respectively having sequences as shown in SEQ ID NO: 46, 47, 48, 49, 50, or 66, or sequences having one or several amino acid substitutions, deletions, or additions (e.g., 1, 2, or 3 substitutions, deletions, or additions) compared to any of the aforementioned sequences;
(9) its HCDR1, HCDR2, HCDR3, LCDR1, LCDR2, and LCDR3 respectively having sequences as shown in SEQ ID NO: 46, 47, 48, 49, 50, or 67, or sequences having one or several amino acid substitutions, deletions, or additions (e.g., 1, 2, or 3 substitutions, deletions, or additions) compared to any of the aforementioned sequences;
(10) its HCDR1, HCDR2, HCDR3, LCDR1, LCDR2, and LCDR3 respectively having sequences as shown in SEQ ID NO: 46, 47, 48, 49, 50, or 68, or sequences having one or several amino acid substitutions, deletions, or additions (e.g., 1, 2, or 3 substitutions, deletions, or additions) compared to any of the aforementioned sequences;
(11) its HCDR1, HCDR2, HCDR3, LCDR1, LCDR2, and LCDR3 respectively having sequences as shown in SEQ ID NO: 46, 63, 58, 49, 50, or 51, or sequences having one or several amino acid substitutions, deletions, or additions (e.g., 1, 2, or 3 substitutions, deletions, or additions) compared to any of the aforementioned sequences;
(12) its HCDR1, HCDR2, HCDR3, LCDR1, LCDR2, and LCDR3 respectively having sequences as shown in SEQ ID NO: 46, 47, 69, 49, 50, or 51, or sequences having one or several amino acid substitutions, deletions, or additions (e.g., 1, 2, or 3 substitutions, deletions, or additions) compared to any of the aforementioned sequences;
(13) its HCDR1, HCDR2, HCDR3, LCDR1, LCDR2, and LCDR3 respectively having sequences as shown in SEQ ID NO: 46, 47, 71, 49, 50, or 51, or sequences having one or several amino acid substitutions, deletions, or additions (e.g., 1, 2, or 3 substitutions, deletions, or additions) compared to any of the aforementioned sequences;
(14) its HCDR1, HCDR2, HCDR3, LCDR1, LCDR2, and LCDR3 respectively having sequences as shown in SEQ ID NO: 46, 47, 73, 49, 50, or 51, or sequences having one or several amino acid substitutions, deletions, or additions (e.g., 1, 2, or 3 substitutions, deletions, or additions) compared to any of the aforementioned sequences;
(15) its HCDR1, HCDR2, HCDR3, LCDR1, LCDR2, and LCDR3 respectively having sequences as shown in SEQ ID NO: 46, 47, 75, 49, 50, or 51, or sequences having one or several amino acid substitutions, deletions, or additions (e.g., 1, 2, or 3 substitutions, deletions, or additions) compared to any of the aforementioned sequences;
(16) its HCDR1, HCDR2, HCDR3, LCDR1, LCDR2, and LCDR3 respectively having sequences as shown in SEQ ID NO: 46, 47, 77, 49, 50, or 51, or sequences having one or several amino acid substitutions, deletions, or additions (e.g., 1, 2, or 3 substitutions, deletions, or additions) compared to any of the aforementioned sequences;
(17) its HCDR1, HCDR2, HCDR3, LCDR1, LCDR2, and LCDR3 respectively having sequences as shown in SEQ ID NO: 46, 47, 79, 49, 50, or 51, or sequences having one or several amino acid substitutions, deletions, or additions (e.g., 1, 2, or 3 substitutions, deletions, or additions) compared to any of the aforementioned sequences;
(18) its HCDR1, HCDR2, HCDR3, LCDR1, LCDR2, and LCDR3 respectively having sequences as shown in SEQ ID NO: 46, 47, 81, 49, 50, or 51, or sequences having one or several amino acid substitutions, deletions, or additions (e.g., 1, 2, or 3 substitutions, deletions, or additions) compared to any of the aforementioned sequences;
(19) its HCDR1, HCDR2, HCDR3, LCDR1, LCDR2, and LCDR3 respectively having sequences as shown in SEQ ID NO: 46, 47, 83, 49, 50, or 51, or sequences having one or several amino acid substitutions, deletions, or additions (e.g., 1, 2, or 3 substitutions, deletions, or additions) compared to any of the aforementioned sequences;
(20) its HCDR1, HCDR2, HCDR3, LCDR1, LCDR2, and LCDR3 respectively having sequences as shown in SEQ ID NO: 46, 47, 85, 49, 50, or 51, or sequences having one or several amino acid substitutions, deletions, or additions (e.g., 1, 2, or 3 substitutions, deletions, or additions) compared to any of the aforementioned sequences;
(21) its HCDR1, HCDR2, HCDR3, LCDR1, LCDR2, and LCDR3 respectively having sequences as shown in SEQ ID NO: 46, 47, 87, 49, 50, or 51, or sequences having one or several amino acid substitutions, deletions, or additions (e.g., 1, 2, or 3 substitutions, deletions, or additions) compared to any of the aforementioned sequences;
(22) its HCDR1, HCDR2, HCDR3, LCDR1, LCDR2, and LCDR3 respectively having sequences as shown in SEQ ID NO: 46, 47, 89, 49, 50, or 51, or sequences having one or several amino acid substitutions, deletions, or additions (e.g., 1, 2, or 3 substitutions, deletions, or additions) compared to any of the aforementioned sequences;
(23) its HCDR1, HCDR2, HCDR3, LCDR1, LCDR2, and LCDR3 respectively having sequences as shown in SEQ ID NO: 52, 53, 54, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(24) its HCDR1, HCDR2, HCDR3, LCDR1, LCDR2, and LCDR3 respectively having sequences as shown in SEQ ID NO: 52, 53, 57, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(25) its HCDR1, HCDR2, HCDR3, LCDR1, LCDR2, and LCDR3 respectively having sequences as shown in SEQ ID NO: 52, 53, 59, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(26) its HCDR1, HCDR2, HCDR3, LCDR1, LCDR2, and LCDR3 respectively having sequences as shown in SEQ ID NO: 52, 62, 54, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(27) its HCDR1, HCDR2, HCDR3, LCDR1, LCDR2, and LCDR3 respectively having sequences as shown in SEQ ID NO: 52, 65, 54, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(28) its HCDR1, HCDR2, HCDR3, LCDR1, LCDR2, and LCDR3 respectively having sequences as shown in SEQ ID NO: 52, 53, 54, 55, Gly-Thr-Ser, or SEQ ID NO: 66, or sequences having one or several amino acid substitutions, deletions, or additions (e.g., 1, 2, or 3 substitutions, deletions, or additions) compared to any of the aforementioned sequences;
(29) its HCDR1, HCDR2, HCDR3, LCDR1, LCDR2, and LCDR3 respectively having sequences as shown in SEQ ID NO: 52, 53, 54, 55, Gly-Thr-Ser, or SEQ ID NO: 67, or sequences having one or several amino acid substitutions, deletions, or additions (e.g., 1, 2, or 3 substitutions, deletions, or additions) compared to any of the aforementioned sequences;
(30) its HCDR1, HCDR2, HCDR3, LCDR1, LCDR2, and LCDR3 respectively having sequences as shown in SEQ ID NO: 52, 53, 54, 55, Gly-Thr-Ser, or SEQ ID NO: 68, or sequences having one or several amino acid substitutions, deletions, or additions (e.g., 1, 2, or 3 substitutions, deletions, or additions) compared to any of the aforementioned sequences;
(31) its HCDR1, HCDR2, HCDR3, LCDR1, LCDR2, and LCDR3 respectively having sequences as shown in SEQ ID NO: 52, 64, 59, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(32) its HCDR1, HCDR2, HCDR3, LCDR1, LCDR2, and LCDR3 respectively having sequences as shown in SEQ ID NO: 52, 53, 70, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(33) its HCDR1, HCDR2, HCDR3, LCDR1, LCDR2, and LCDR3 respectively having sequences as shown in SEQ ID NO: 52, 53, 72, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(34) its HCDR1, HCDR2, HCDR3, LCDR1, LCDR2, and LCDR3 respectively having sequences as shown in SEQ ID NO: 52, 53, 74, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(35) its HCDR1, HCDR2, HCDR3, LCDR1, LCDR2, and LCDR3 respectively having sequences as shown in SEQ ID NO: 52, 53, 76, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(36) its HCDR1, HCDR2, HCDR3, LCDR1, LCDR2, and LCDR3 respectively having sequences as shown in SEQ ID NO: 52, 53, 78, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(37) its HCDR1, HCDR2, HCDR3, LCDR1, LCDR2, and LCDR3 respectively having sequences as shown in SEQ ID NO: 52, 53, 80, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(38) its HCDR1, HCDR2, HCDR3, LCDR1, LCDR2, and LCDR3 respectively having sequences as shown in SEQ ID NO: 52, 53, 82, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(39) its HCDR1, HCDR2, HCDR3, LCDR1, LCDR2, and LCDR3 respectively having sequences as shown in SEQ ID NO: 52, 53, 84, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(40) its HCDR1, HCDR2, HCDR3, LCDR1, LCDR2, and LCDR3 respectively having sequences as shown in SEQ ID NO: 52, 53, 86, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(41) its HCDR1, HCDR2, HCDR3, LCDR1, LCDR2, and LCDR3 respectively having sequences as shown in SEQ ID NO: 52, 53, 88, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences;
(42) its HCDR1, HCDR2, HCDR3, LCDR1, LCDR2, and LCDR3 respectively having sequences as shown in SEQ ID NO: 52, 53, 90, 55, Gly-Thr-Ser, or SEQ ID NO: 51, or sequences having one or several amino acid substitutions, deletions, or additions (e.g., 1, 2, or 3 substitutions, deletions, or additions) compared to any of the aforementioned sequences.

10. The antibody or antigen-binding fragment thereof according to claim 8, **characterized in that** the antibody or antigen-binding fragment is humanized.

11. The antibody or antigen-binding fragment thereof according to claim 10, **characterized in that** the antibody or antigen-binding fragment is selected from the following VH and VL sequence combinations:
(1) a VH domain comprising the amino acid sequence as shown in SEQ ID NO: 2, or a sequence substantially identical thereto (e.g., at least 80%, 85%, 90%, 92%, 95%, 97%, 98%, 99%, or higher identity, or having one or more amino acid substitutions, e.g., conservative substitutions); and a VL domain comprising the amino acid sequence as shown in SEQ ID NO: 3, or a sequence substantially identical thereto (e.g., at least 80%, 85%, 90%, 92%, 95%, 97%, 98%, 99%, or higher identity, or having one or more amino acid substitutions, e.g., conservative substitutions);
(2) a VH domain comprising the amino acid sequence as shown in SEQ ID NO: 4, or a sequence substantially identical thereto (e.g., at least 80%, 85%, 90%, 92%, 95%, 97%, 98%, 99%, or higher identity, or having one or more amino acid substitutions, e.g., conservative substitutions); and a VL domain comprising the amino acid sequence as shown in SEQ ID NO: 5, or a sequence substantially identical thereto (e.g., at least 80%, 85%, 90%, 92%, 95%, 97%, 98%, 99%, or higher identity, or having one or more amino acid substitutions, e.g., conservative substitutions);
(3) a VH domain comprising the amino acid sequence as shown in SEQ ID NO: 6, or a sequence substantially identical thereto (e.g., at least 80%, 85%, 90%, 92%, 95%, 97%, 98%, 99%, or higher identity, or having one or more amino acid substitutions, e.g., conservative substitutions); and a VL domain comprising the amino acid sequence as shown in SEQ ID NO: 7, or a sequence substantially identical thereto (e.g., at least 80%, 85%, 90%, 92%, 95%, 97%, 98%, 99%, or higher identity, or having one or more amino acid substitutions, e.g., conservative substitutions);
(4) a VH domain comprising the amino acid sequence as shown in SEQ ID NO: 8, or a sequence substantially identical thereto (e.g., at least 80%, 85%, 90%, 92%, 95%, 97%, 98%, 99%, or higher identity, or having one or more amino acid substitutions, e.g., conservative substitutions); and a VL domain comprising the amino acid sequence as shown in SEQ ID NO: 9, or a sequence substantially identical thereto (e.g., at least 80%, 85%, 90%, 92%, 95%, 97%, 98%, 99%, or higher identity, or having one or more amino acid substitutions, e.g., conservative substitutions);
(5) a VH domain comprising the amino acid sequence as shown in SEQ ID NO: 10, or a sequence substantially identical thereto (e.g., at least 80%, 85%, 90%, 92%, 95%, 97%, 98%, 99%, or higher identity, or having one or more amino acid substitutions, e.g., conservative substitutions); and a VL domain comprising the amino acid sequence as shown in SEQ ID NO: 11, or a sequence substantially identical thereto (e.g., at least 80%, 85%, 90%, 92%, 95%, 97%, 98%, 99%, or higher identity, or having one or more amino acid substitutions, e.g., conservative substitutions);
(6) a VH domain comprising the amino acid sequence as shown in SEQ ID NO: 12, or a sequence substantially identical thereto (e.g., at least 80%, 85%, 90%, 92%, 95%, 97%, 98%, 99%, or higher identity, or having one or more amino acid substitutions, e.g., conservative substitutions); and a VL domain comprising the amino acid sequence as shown in SEQ ID NO: 13, or a sequence substantially identical thereto (e.g., at least 80%, 85%, 90%, 92%, 95%, 97%, 98%, 99%, or higher identity, or having one or more amino acid substitutions, e.g., conservative substitutions);
(7) a VH domain comprising the amino acid sequence as shown in SEQ ID NO: 14, or a sequence substantially identical thereto (e.g., at least 80%, 85%, 90%, 92%, 95%, 97%, 98%, 99%, or higher identity, or having one or more amino acid substitutions, e.g., conservative substitutions); and a VL domain comprising the amino acid sequence as shown in SEQ ID NO: 15, or a sequence substantially identical thereto (e.g., at least 80%, 85%, 90%, 92%, 95%, 97%, 98%, 99%, or higher identity, or having one or more amino acid substitutions, e.g., conservative substitutions);
(8) a VH domain comprising the amino acid sequence as shown in SEQ ID NO: 16, or a sequence substantially identical thereto (e.g., at least 80%, 85%, 90%, 92%, 95%, 97%, 98%, 99%, or higher identity, or having one or more amino acid substitutions, e.g., conservative substitutions); and a VL domain comprising the amino acid sequence as shown in SEQ ID NO: 17, or a sequence substantially identical thereto (e.g., at least 80%, 85%, 90%, 92%, 95%, 97%, 98%, 99%, or higher identity, or having one or more amino acid substitutions, e.g., conservative substitutions);
(9) a VH domain comprising the amino acid sequence as shown in SEQ ID NO: 18, or a sequence substantially identical thereto (e.g., at least 80%, 85%, 90%, 92%, 95%, 97%, 98%, 99%, or higher identity, or having one or more amino acid substitutions, e.g., conservative substitutions); and a VL domain comprising the amino acid sequence as shown in SEQ ID NO: 19, or a sequence substantially identical thereto (e.g., at least 80%, 85%, 90%, 92%, 95%, 97%, 98%, 99%, or higher identity, or having one or more amino acid substitutions, e.g., conservative substitutions);
(10) a VH domain comprising the amino acid sequence as shown in SEQ ID NO: 20, or a sequence substantially identical thereto (e.g., at least 80%, 85%, 90%, 92%, 95%, 97%, 98%, 99%, or higher identity, or having one or more amino acid substitutions, e.g., conservative substitutions); and a VL domain comprising the amino acid sequence as shown in SEQ ID NO: 21, or a sequence substantially identical thereto (e.g., at least 80%, 85%, 90%, 92%, 95%, 97%, 98%, 99%, or higher identity, or having one or more amino acid substitutions, e.g., conservative substitutions);
(11) a VH domain comprising the amino acid sequence as shown in SEQ ID NO: 22, or a sequence substantially identical thereto (e.g., at least 80%, 85%, 90%, 92%, 95%, 97%, 98%, 99%, or higher identity, or having one or more amino acid substitutions, e.g., conservative substitutions); and a VL domain comprising the amino acid sequence as shown in SEQ ID NO: 23, or a sequence substantially identical thereto (e.g., at least 80%, 85%, 90%, 92%, 95%, 97%, 98%, 99%, or higher identity, or having one or more amino acid substitutions, e.g., conservative substitutions);
(12) a VH domain comprising the amino acid sequence as shown in SEQ ID NO: 24, or a sequence substantially identical thereto (e.g., at least 80%, 85%, 90%, 92%, 95%, 97%, 98%, 99%, or higher identity, or having one or more amino acid substitutions, e.g., conservative substitutions); and a VL domain comprising the amino acid sequence as shown in SEQ ID NO: 25, or a sequence substantially identical thereto (e.g., at least 80%, 85%, 90%, 92%, 95%, 97%, 98%, 99%, or higher identity, or having one or more amino acid substitutions, e.g., conservative substitutions);
(13) a VH domain comprising the amino acid sequence as shown in SEQ ID NO: 26, or a sequence substantially identical thereto (e.g., at least 80%, 85%, 90%, 92%, 95%, 97%, 98%, 99%, or higher identity, or having one or more amino acid substitutions, e.g., conservative substitutions); and a VL domain comprising the amino acid sequence as shown in SEQ ID NO: 27, or a sequence substantially identical thereto (e.g., at least 80%, 85%, 90%, 92%, 95%, 97%, 98%, 99%, or higher identity, or having one or more amino acid substitutions, e.g., conservative substitutions);
(14) a VH domain comprising the amino acid sequence as shown in SEQ ID NO: 28, or a sequence substantially identical thereto (e.g., at least 80%, 85%, 90%, 92%, 95%, 97%, 98%, 99%, or higher identity, or having one or more amino acid substitutions, e.g., conservative substitutions); and a VL domain comprising the amino acid sequence as shown in SEQ ID NO: 29, or a sequence substantially identical thereto (e.g., at least 80%, 85%, 90%, 92%, 95%, 97%, 98%, 99%, or higher identity, or having one or more amino acid substitutions, e.g., conservative substitutions);
(15) a VH domain comprising the amino acid sequence as shown in SEQ ID NO: 30, or a sequence substantially identical thereto (e.g., at least 80%, 85%, 90%, 92%, 95%, 97%, 98%, 99%, or higher identity, or having one or more amino acid substitutions, e.g., conservative substitutions); and a VL domain comprising the amino acid sequence as shown in SEQ ID NO: 31, or a sequence substantially identical thereto (e.g., at least 80%, 85%, 90%, 92%, 95%, 97%, 98%, 99%, or higher identity, or having one or more amino acid substitutions, e.g., conservative substitutions);
(16) a VH domain comprising the amino acid sequence as shown in SEQ ID NO: 32, or a sequence substantially identical thereto (e.g., at least 80%, 85%, 90%, 92%, 95%, 97%, 98%, 99%, or higher identity, or having one or more amino acid substitutions, e.g., conservative substitutions); and a VL domain comprising the amino acid sequence as shown in SEQ ID NO: 33, or a sequence substantially identical thereto (e.g., at least 80%, 85%, 90%, 92%, 95%, 97%, 98%, 99%, or higher identity, or having one or more amino acid substitutions, e.g., conservative substitutions);
(17) a VH domain comprising the amino acid sequence as shown in SEQ ID NO: 34, or a sequence substantially identical thereto (e.g., at least 80%, 85%, 90%, 92%, 95%, 97%, 98%, 99%, or higher identity, or having one or more amino acid substitutions, e.g., conservative substitutions); and a VL domain comprising the amino acid sequence as shown in SEQ ID NO: 35, or a sequence substantially identical thereto (e.g., at least 80%, 85%, 90%, 92%, 95%, 97%, 98%, 99%, or higher identity, or having one or more amino acid substitutions, e.g., conservative substitutions);
(18) a VH domain comprising the amino acid sequence as shown in SEQ ID NO: 36, or a sequence substantially identical thereto (e.g., at least 80%, 85%, 90%, 92%, 95%, 97%, 98%, 99%, or higher identity, or having one or more amino acid substitutions, e.g., conservative substitutions); and a VL domain comprising the amino acid sequence as shown in SEQ ID NO: 37, or a sequence substantially identical thereto (e.g., at least 80%, 85%, 90%, 92%, 95%, 97%, 98%, 99%, or higher identity, or having one or more amino acid substitutions, e.g., conservative substitutions);
(19) a VH domain comprising the amino acid sequence as shown in SEQ ID NO: 38, or a sequence substantially identical thereto (e.g., at least 80%, 85%, 90%, 92%, 95%, 97%, 98%, 99%, or higher identity, or having one or more amino acid substitutions, e.g., conservative substitutions); and a VL domain comprising the amino acid sequence as shown in SEQ ID NO: 39, or a sequence substantially identical thereto (e.g., at least 80%, 85%, 90%, 92%, 95%, 97%, 98%, 99%, or higher identity, or having one or more amino acid substitutions, e.g., conservative substitutions);
(20) a VH domain comprising the amino acid sequence as shown in SEQ ID NO: 40, or a sequence substantially identical thereto (e.g., at least 80%, 85%, 90%, 92%, 95%, 97%, 98%, 99%, or higher identity, or having one or more amino acid substitutions, e.g., conservative substitutions); and a VL domain comprising the amino acid sequence as shown in SEQ ID NO: 41, or a sequence substantially identical thereto (e.g., at least 80%, 85%, 90%, 92%, 95%, 97%, 98%, 99%, or higher identity, or having one or more amino acid substitutions, e.g., conservative substitutions);
(21) a VH domain comprising the amino acid sequence as shown in SEQ ID NO: 42, or a sequence substantially identical thereto (e.g., at least 80%, 85%, 90%, 92%, 95%, 97%, 98%, 99%, or higher identity, or having one or more amino acid substitutions, e.g., conservative substitutions); and a VL domain comprising the amino acid sequence as shown in SEQ ID NO: 43, or a sequence substantially identical thereto (e.g., at least 80%, 85%, 90%, 92%, 95%, 97%, 98%, 99%, or higher identity, or having one or more amino acid substitutions, e.g., conservative substitutions);
(22) a VH domain comprising the amino acid sequence as shown in SEQ ID NO: 44, or a sequence substantially identical thereto (e.g., at least 80%, 85%, 90%, 92%, 95%, 97%, 98%, 99%, or higher identity, or having one or more amino acid substitutions, e.g., conservative substitutions); and a VL domain comprising the amino acid sequence as shown in SEQ ID NO: 45, or a sequence substantially identical thereto (e.g., at least 80%, 85%, 90%, 92%, 95%, 97%, 98%, 99%, or higher identity, or having one or more amino acid substitutions, e.g., conservative substitutions).

12. The antibody or antigen-binding fragment thereof according to claim 1, **characterized in that** it is selected from the group consisting of a full-length antibody, a Fab, a Fab', a (Fab')₂, an Fd, an Fv, a dAb, an scFv, and an scFv-scFv.

13. The antibody or antigen-binding fragment thereof according to claim 1, **characterized in that** the antibody comprises a heavy chain constant region and a light chain constant region derived from a human immunoglobulin; preferably, the heavy chain constant region is selected from the heavy chain constant regions of human IgG1, IgG2, IgG3, and IgG4; and the heavy chain constant region has a native sequence or a sequence having one or more amino acid substitutions, deletions, or additions compared to the native sequence from which it is derived; and the light chain constant region is preferably the constant region of a human κ light chain.

14. The antibody or antigen-binding fragment thereof according to claim 13, **characterized in that** the antibody comprises a heavy chain constant region of human IgG4, and the heavy chain constant region comprises an amino acid mutation S228P.

15. A bispecific antibody or an antigen-binding portion thereof, comprising the antibody or antigen-binding portion thereof according to any one of claims 1-14 linked to a second antibody or antigen-binding portion thereof.

16. The bispecific antibody or antigen-binding portion thereof according to claim 15, **characterized in that** the second antibody or antigen-binding portion thereof binds to TFPI.

17. A DNA molecule encoding the antibody or antigen-binding fragment thereof according to any one of claims 1-16.

18. A vector comprising the DNA molecule according to claim 17.

19. A host cell comprising the vector according to claim 18, preferably the host cell comprises a prokaryotic cell, a yeast cell, or a mammalian cell, and more preferably is a CHO cell.

20. A pharmaceutical composition comprising the antibody or antigen-binding fragment thereof according to any one of claims 1-16, and a pharmaceutically acceptable excipient, carrier, or diluent.

21. Use of the antibody or antigen-binding fragment thereof according to any one of claims 1-16, or the pharmaceutical composition according to claim 20, in the manufacture of a medicament for preventing or treating a disease or condition of hereditary or acquired coagulation factor deficiency.

22. A method for preventing or treating a disease or condition of hereditary or acquired coagulation factor deficiency, comprising administering an effective dose of the antibody or antigen-binding fragment thereof according to any one of claims 1-16, or the pharmaceutical composition according to claim 20, to a patient in need thereof.

23. The use according to claim 21 or the method according to claim 22, **characterized in that** the disease is hemophilia.

24. Use of the antibody or antigen-binding fragment thereof according to any one of claims 1-16, or the pharmaceutical composition according to claim 20, in the manufacture of a medication for preventing or treating a hereditary coagulation disorder.

25. A method for preventing or treating a hereditary coagulation disorder, comprising administering an effective dose of the antibody or antigen-binding fragment thereof according to any one of claims 1-16, or the pharmaceutical composition according to claim 20, to a patient in need thereof.

26. The use according to claim 24 or the method according to claim 25, **characterized in that** the disease is Glanzmann Thrombasthenia or Bernard-Soulier Syndrome.
